(19) Europäisches Patentamt
European Patent Office
Office européen des brevets

(11) EP 3 147 298 A1

(12) EUROPEAN PATENT APPLICATION
published in accordance with Art. 153(4) EPC

(43) Date of publication:
**29.03.2017 Bulletin 2017/13**

(21) Application number: **15795999.0**

(22) Date of filing: **26.05.2015**

(51) Int Cl.:
***C07K 19/00*** $^{(2006.01)}$ ***C12N 15/62*** $^{(2006.01)}$
***A61K 38/17*** $^{(2006.01)}$ ***A61K 39/395*** $^{(2006.01)}$
***A61P 37/02*** $^{(2006.01)}$

(86) International application number:
**PCT/KR2015/005256**

(87) International publication number:
**WO 2015/178746 (26.11.2015 Gazette 2015/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **23.05.2014 KR 20140062399**
**22.05.2015 KR 20150071820**

(71) Applicants:
- **Genexine, Inc.**
**Bundang-gu, Seongnam-si**
**Gyeonggi-do 463-400 (KR)**
- **Postech Academy-Industry Foundation**
**Pohang-si, Gyeongsangbuk-do 790-784 (KR)**

(72) Inventors:
- **SUNG, Young Chul**
**Seoul 140-762 (KR)**
- **LEE, Ji Yeung**
**Yongin-si**
**Gyeonggi-do 448-529 (KR)**
- **SONG, Mi-Young**
**Ulsan 680-840 (KR)**
- **LIM, Hye Seong**
**Seongnam-si**
**Gyeonggi-do 463-904 (KR)**
- **LEE, Byung Ha**
**Gunpo-si**
**Gyeonggi-do 435-743 (KR)**

(74) Representative: **Rigby, Barbara Nicole**
**Dehns**
**St Bride's House**
**10 Salisbury Square**
**London EC4Y 8JD (GB)**

# (54) PD-L1 FUSION PROTEIN AND USE THEREOF

(57) The present invention relates to a fusion protein composed of the extracellular domain of PD-L1 and a modified immunoglobulin Fc region. The extracellular domain of PD-L1 and a fragment thereof have excellent immunomodulatory activity, and can be used as an immunomodulatory agent if a modified immunoglobulin Fc region is coupled thereto. Accordingly, the PD-L1 fusion protein according to the present invention demonstrated its excellent effect in disease models of inflammatory bowel disease, colitis, psoriasis, asthma and arthritis, and thus can be very effectively used for the treatment of such diseases.

Fig. 17

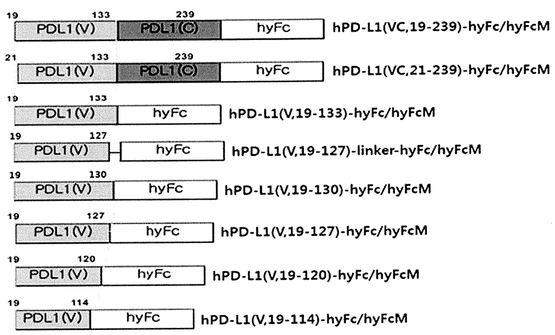


EP 3 147 298 A1

Printed by Jouve, 75001 PARIS (FR)

## Description

### TECHNICAL FIELD

The present invention relates to a PD-L1 fusion protein prepared by coupling PD-L1 to an immunoglobulin Fc region, which has increased stability and activity. Moreover, the present invention relates to a pharmaceutical composition comprising PD-L1 or a specific fragment thereof, and more particularly, to a pharmaceutical composition for treating an immune disease, which comprises PD-L1 or a specific fragment thereof.

### BACKGROUND ART

Human hPD-L1 (human Programmed Cell Death-Ligand 1), a ligand for PD-1 (programmed death-1), is a type 1 transmembrane protein expressed not only in hematopoietic cells such as T lymphocytes, B lymphocytes, dendritic cells, or macrophages, but also in non-hematopoietic cells such as keratinocytes, islet cells, or hepatocytes.

It is known that PD-1 binding to PD-L1 is expressed mainly in activated T cells and B cells, macrophages, or dendritic cells, and binds to PD-L1 to inhibit the cytokine production and cell proliferation of T cells. It was reported that PD-L1 binds not only to PD-1 but also B7-1 (Immunity. 2007 Jul; 27(1): 111-22), which leads not only to blocking of the B7-1:CD28 binding to suppress T cell activity, but transduction of an inhibitory signal into T cells through B7-1.

Meanwhile, T cell activation requires both an antigen-specific signal (signal 1) via the T cell receptor and a co-stimulatory signal (signal 2). Without any one of the two signals, T cells become anergic. Programmed cell death 1 (PD-1) is a co-stimulatory factor (immune check point or immune modulator) that regulates co-stimulatory activity on T cells. It may bind to programmed cell death ligand 1 (PD-L1), B7.1 (CD80), or the like that is expressed on the surface of cells such as activated T cells (CD8 and/or CD4) or dendritic cells, to inhibit the functions of T cells, such as inhibiting T cell proliferation and reducing cytokine expression in T cells, etc.

It is known that the binding between PD-1 and PD-L1 induces the activity of regulatory T cells (Immunol Rev. 2010 Jul; 236:219-42). It was observed that, when PD-L1-Ig protein obtained by fusing IgG1 Fc to PD-L1 was injected into a collagen-induced arthritis (CIA) mouse model in order to test the immune tolerance-inducing function of PD-L1, symptoms of arthritis in the mouse model were alleviated (Rheumatol Int. 2011 Apr; 31(4):513-9). Since PD-1 is expressed in activated T cells, it is expected that the PD-L1 protein can be effectively used as a therapeutic agent that specifically targets activated immune cells to induce immune tolerance not only in an autoimmune disease but also in organ transplantation.

To date, therapeutic agents regarding PD-1/PD-L1 signaling have been developed such that they break immune tolerance as antagonists, thereby increasing T cell activity. However, a T cell immune tolerance-based immunotherapeutic agent using an agonist has not yet been developed. This is because a PD-1/PD-L1 agonist particularly needs to be developed as a soluble-type protein, whereas a PD-1/PD-L1 signal antagonist can be easily developed using an antibody fusion technology.

Immunoglobulin (Ig) Fc fusion technologies can be used for increasing the half-life of protein therapeutic agents *in vivo.* However, IgG1 used in conventional Ig fusion technologies cause antibody dependent cell-mediated cytotoxicity (ADCC) and complement dependent cytotoxicity (CDC) *in vivo.* Thus, when Ig fusion proteins are used as an agent for treating an autoimmune disease or as an agent for inducing immune tolerance in organ transplantation, they cannot play the role of inhibiting inflammatory responses, and may rather aggravate inflammation.

Accordingly, there is a need to develop a technology that increases the therapeutic effect of PD-L1 as an immunosuppressive agent by preventing PD-L1 from causing ADCC and CDC, while maintaining the half-life of PD-L1 at a level similar to conventional Ig fusion protein therapeutic agents.

### DISCLOSURE

### TECHNICAL PROBLEM

An object of the present invention is to provide a fusion protein comprising the extracellular domain of PD-L1 protein or a fragment thereof and a modified immunoglobulin Fc region, in order to increase the therapeutic effect of PD-L1. Another object of the present invention is to provide an extracellular domain fragment of PD-L1 protein, which shows an excellent therapeutic effect and high productivity, a pharmaceutical composition comprising such fragment, and the use thereof.

### TECHNICAL SOLUTION

In accordance with an object of the present invention, there is provided a fusion protein comprising the extracellular domain of PD-L1 protein or a fragment thereof and a modified immunoglobulin Fc region.

In accordance with another object of the present invention, there is provided a nucleic acid encoding the fusion protein, a vector comprising the nucleic acid, and a host cell comprising the vector.

In accordance with another object of the present invention, there is provided a method for producing the fusion protein, comprising the steps of: introducing a DNA molecule encoding the fusion protein into a mammalian host cell; expressing the fusion protein in the host cell; and recovering the expressed protein.

In accordance with another object of the present invention, there is provided a pharmaceutical composition for preventing or treating an immune disease, and a composition for inducing immune tolerance, which comprise the fusion protein.

In accordance with another object of the present invention, there is provided a method for preventing or treating an immune disease, and a method for inducing immune tolerance, which comprise administering the fusion protein.

ADVANTAGEOUS EFFECTS

The fusion protein of the present invention, which comprises the extracellular domain of PD-L1 protein or a fragment thereof and a modified immunoglobulin Fc region, has characteristics in that it has higher stability than conventional Ig fusion proteins and can be produced in large amounts. In addition, it can inhibit cytokine production and cell proliferation of T cells, and has the effects of controlling regulatory T cells (Treg) that can inhibit the function of abnormally activated immune cells and control inflammatory responses.

Therefore, the fusion protein of the present invention can be effectively used as a pharmaceutical composition for preventing or treating an autoimmune disease caused by abnormal regulation of an immune response, an inflammatory disease, and an immune disease such as transplant rejection disease, or as an immune tolerance inducer. Specifically, the fusion protein of the present invention, which comprises the extracellular domain of PD-L1 protein or a fragment thereof and a modified immunoglobulin Fc region, showed its excellent effect in disease models of inflammatory bowel disease, colitis, psoriasis, asthma and arthritis, and thus can be very effectively used for the treatment of such diseases.

DESCRIPTION OF DRAWINGS

Fig. 1 depicts a gene construct and a method for producing a mPD-L1-mFc protein using the same.

Fig. 2 illustrates cell line screening and the productivity of cells lines. Fig. 2(a) shows the result of comparing the productivity of suspension cell lines for cell line screening, and Fig. 2(b) shows the results of SE-HPLC for examining the culture productivity of cell lines and identifying the target protein.

Fig. 3 shows the results of SDS-PAGE analysis (Fig. 3(a)) and SE-HPLC analysis (Fig. 3(b)) conducted to identify a purification product and examine purity after purification of a PD-L1-hyFc recombinant protein.

Fig. 4 depicts a T cell proliferation assay method among *in vitro* experimental methods for a mPD-L1-mFc recombinant protein.

Fig. 5 shows the results of T cell proliferation assay for mPD-L1-mFc and hPD-L1 (VC, 19-239)-hyFcM1 recombinant proteins. It was observed that treatment with the fusion protein of the present invention inhibited stimulation-induced proliferation of T cells.

Fig. 6 presents graphs showing the results of observing the inhibitory effects of a mPD-L1-mFc fusion protein on weight loss (Fig. 6(a)) and colon length reduction (Fig. 6(b)) in DSS-induced inflammatory bowel disease animal models.

Fig. 7 depicts the results of histological staining (Fig. 7(a)) and histological scoring analysis (Fig. 7(b)) conducted to analyze the therapeutic effect of a mPD-L1-mFc fusion protein in DSS-induced inflammatory bowel disease animal models.

Fig. 8 shows the results of examining the therapeutic effect of a mPD-L1-mFc fusion protein in T cell-induced inflammatory bowel disease animal models. Fig. 8(a) shows the results of measuring the change in body weight after administration of mPD-L1-mFc, and Fig. 8(b) compares the symptom scoring according to lesions.

Fig. 9 shows the results of histological staining (Fig. 9(a)) and histological scoring analysis (Fig. 9(b)) conducted to analyze the therapeutic effect of a mPD-L1-mFc fusion protein in T cell-induced inflammatory bowel disease animal models.

Fig. 10 shows the results of observing the inhibitory effect of a mPD-L1-mFc fusion protein on weight loss in T cell-induced inflammatory bowel disease animal models.

Fig. 11 shows the results of measuring the amount of inflammatory cytokines secreted by colonic LP cells after administration of a mPD-L1-mFc fusion protein in T cell-induced inflammatory bowel disease animal models.

Fig. 12 shows the results of conducting histological staining (Fig. 12(a)) and measuring epidermal thickness (Fig.

12(b)) to examine the effect of a mPD-L1-mFc fusion protein in acute psoriasis mouse models.

Fig. 13 shows the results of measuring ear thickness to examine the effect of mPD-L1-mFc in acute psoriasis mouse models.

Fig. 14 shows the results of histological staining for examining the effect of mPD-L1-mFc in acute psoriasis mouse models.

Fig. 15 shows the results of measuring the change in epidermal thickness after the treatment of mPD-L1-mFc in acute psoriasis mouse models.

Fig. 16 shows the results of examining the immune rejection inhibition ability (immune tolerance) of mPD-L1-mFc in pancreas transplantation models. Fig. 16(a) and Fig. 16(b) show the results of observing the changes in blood glucose and body weight in untreated transplantation models and in a group administered with a mPD-L1-mFc fusion protein, respectively.

Fig. 17 is a schematic figure illustrating the exemplary structures of hPD-L1 and hyFc fusion protein variants.

Fig. 18 shows the results of comparing the productivity of fusion protein variants comprising hPD-L1 and hyFc. Fig. 18(a) and Fig. 18(b) show the results of SE-HPLC of a N-terminal sequence having amino acid residues starting from position 19, and of a N-terminal sequence having amino acid residues starting from position 21, respectively.

Fig. 19 shows a gene construct of a hPD-L1-hyFc recombinant protein and a process for producing the same.

Fig. 20 is a graph showing the productivity of recombinant proteins (hPD-L1-hyFc) obtained by fusing the extracellular domain of hPD-L1 or a fragment thereof to hyFc (§: fusion proteins produced from a CHO cell line).

Fig. 21 shows the results of SDS-PAGE for identifying the hPD-L1-hyFc fusion proteins produced.

Fig. 22 shows the results of HPLC for identifying the hPD-L1-hyFc fusion proteins produced.

Fig. 23 shows the results of T cell proliferation assay for examining the inhibition ability of hPD-L1 (VC,19-239)-hyFcM1 and hPD-L1 (V,19-133)-hyFcM1 on $CD4^+$ T cell proliferation.

Fig. 24 shows the results of T cell proliferation assay for examining the inhibition ability of hPD-L1 (VC,19-239)-hyFcM1 and hPD-L1 (V,19-133)-hyFcM1 on $CD8^+$ T cell proliferation.

Fig. 25 shows the results of ELISA assay (Fig. 24(a)) and SPR assay (Fig. 24(b)) for analyzing the PD-1 binding affinity of hPD-L1-hyFc fusion proteins recovered from several cell lines.

Fig. 26 shows the results of measuring the pK values of hPD-L1 (VC,19-239), hPD-L1 (VC,19-239)-IgG1 (wild-type IgG1 Fc), hPD-L1 (VC,19-239)-hyFcM1 and hPD-L1 (V,19-133)-hyFcM1 fusion proteins in normal white rat models.

Fig. 27 shows the results of examining the IL-2 production inhibition by treatment with various concentrations of hPD-L1 (VC,19-239)-hyFcM1.

Fig. 28 shows the results of examining the inhibition ability of hPD-L1 (VC,19-239)-hyFcM1 on T cell proliferation.

Fig. 29 shows the results of examining the IL-6 expression inhibition by various concentrations of hPD-L1 (VC,19-239)-hyFcM1.

Fig. 30 shows the results of examining the inhibition ability of hPD-L1 (VC,19-239)-hyFcM1 on IL-2 production and IFN-gamma production in mouse cells.

Fig. 31 shows the results of examining the inhibitory effects of hPD-L1 (VC,19-239)-hyFcM1, hPD-L1 (V,19-133)-hyFcM1 and hPD-L1 (V,19-127)-hyFcM1 on the activity of CD4+ T cells.

Fig. 32 shows the results of examining the inhibition ability of hPD-L1 (VC,19-239)-hyFcM1 and hPD-L1 (V,19-133)-hyFcM1 on IFN-gamma secretion in T cells according to concentrations.

Fig. 33 shows the results of MTT assay for measuring the inhibition ability of hPD-L1 (VC,19-239)-hyFcM1 and hPD-L1 (V,19-133)-hyFcM1 on T cell proliferation according to concentrations.

Fig. 34 shows the results of examining the inhibition ability of hPD-L1 (VC,19-239)-hyFcM1 and hPD-L1 (V,19-133)-hyFcM1 on IL-2 secretion in Jurkat cells.

Fig. 35 shows the results of examining the therapeutic effect of hPD-L1-hyFc in T cell-induced inflammatory bowel disease animal models. Fig. 35(a) and Fig. 35(b) respectively show the results of symptom scoring and the change in body weight, after administration of hPD-L1 (V,19-133)-hyFcM1.

Fig. 36 shows the results of comparing the survival rate of mice administered with hPD-L1 (VC,19-239)-hyFcM1 and hPD-L1 (V,19-133)-hyFcM1 in T cell-induced inflammatory bowel disease animal models.

Fig. 37 shows the results of measuring ear thickness to examine the effect of hPD-L1 (VC,19-239)-hyFcM1 in acute psoriasis mouse animal models.

Fig. 38 shows the results of histological staining for examining the effect of hPD-L1 (VC,19-239)-hyFcM1 in acute psoriasis mouse animal models.

Fig. 39 shows the results of measuring the change in epidermal tissue thickness after the treatment of hPD-L1 (VC,19-239)-hyFcM1 in acute psoriasis mouse animal models.

Fig. 40 shows the results of verifying the effect of a mPD-L1-mFc fusion protein by scoring the results of phenotype observation in rheumatoid arthritis models.

BEST MODE

In accordance with the object of the present invention, in one aspect, there is provided a fusion protein comprising the extracellular domain of PD-L1 (Programmed Cell Death-Ligand 1) protein or a fragment thereof and a modified immunoglobulin Fc region ("PD-L1-Fc fusion protein," "PD-L1-Fc protein," "PD-L1 fusion protein" or "fusion protein").

The extracellular domain of PD-L1 may be a polypeptide comprising an immunoglobulin V (Ig V) like domain of PD-L1 and an immunoglobulin C (Ig C) like domain of PD-L1.

The extracellular domain of PD-L1 is a domain exposed outside a cell membrane, and may be a polypeptide comprising the amino acids at positions 19 to 238 of SEQ ID NO: 3 or a polypeptide comprising the amino acids at positions 19 to 239 of SEQ ID NO: 3.

The extracellular domain of PD-L1 comprises an Ig V like sequence (Ig V sequence) that is a conserved sequence similar to the amino acid sequence of an immunoglobulin (Ig), and the highly conserved Ig V like sequence consists of the amino acids at positions 68 to 114 of SEQ ID NO: 3. In addition, the extracellular domain of PD-L1 comprises an Ig C like sequence (Ig C sequence), and the highly conserved Ig C like sequence consists of the amino acids at positions 153 to 210 of SEQ ID NO: 3. Moreover, the extracellular domain fragment of PD-L1 may comprise at least a part of the Ig V like domain comprising the Ig V like sequence of PD-L1.

In addition, the extracellular domain of PD-L1 or a fragment thereof may be of human or mouse origin.

The extracellular domain of PD-L1 may comprise a polypeptide (SEQ ID NO: 41) consisting of the amino acids at positions 19 to 239 of SEQ ID NO: 3 or a polypeptide consisting of the amino acids at positions 19 to 239 of SEQ ID NO: 1. In addition, the extracellular domain of PD-L1 may have about 70%, 75%, 80%, 85%, 90%, 91%, 92%, 93%, 94%, 95%, 96%, 97%, 98%, 99% or more homology to the polypeptide sequence consisting of the amino acids at positions 19 to 239 of SEQ ID NO: 41 or 1.

In addition, the Ig V like domain of the extracellular domain of PD-L1 is a domain interacting with PD-1, and may be a polypeptide having the amino acids at positions 21 to 114 of SEQ ID NO: 3, a polypeptide having the amino acids at positions 19 to 114 of SEQ ID NO: 3, a polypeptide having the amino acids at positions 21 to 120 of SEQ ID NO: 3, a polypeptide having the amino acids at positions 19 to 120 of SEQ ID NO: 3, a polypeptide (SEQ ID NO: 48) having the amino acids at positions 21 to 127 of SEQ ID NO: 3, a polypeptide (SEQ ID NO: 39) having the amino acids at positions 19 to 127 of SEQ ID NO: 3, a polypeptide having the amino acids at positions 21 to 130 of SEQ ID NO: 3, a polypeptide having the amino acids at positions 19 to 130 of SEQ ID NO: 3, a polypeptide having the amino acids at positions 21 to 131 of SEQ ID NO: 3, a polypeptide having the amino acids at positions 19 to 131 of SEQ ID NO: 3, a polypeptide having the amino acids at positions 21 to 133 of SEQ ID NO: 3, a polypeptide (SEQ ID NO: 40) having the amino acids at positions 19 to 133 of SEQ ID NO: 3, a polypeptide having the amino acids at positions 21 to 239 of SEQ ID NO: 3, or a polypeptide (SEQ ID NO: 41) having the amino acids at positions 19 to 239 of SEQ ID NO: 3.

In addition, the Ig V like domain of the extracellular domain of PD-L1 may be a polypeptide having the amino acids at positions 21 to 114 of SEQ ID NO: 1, a polypeptide having the amino acids at positions 19 to 114 of SEQ ID NO: 1, a polypeptide having the amino acids at positions 21 to 120 of SEQ ID NO: 1, a polypeptide having the amino acids at positions 19 to 120 of SEQ ID NO: 1, a polypeptide having the amino acids at positions 21 to 127 of SEQ ID NO: 1, a polypeptide having the amino acids at positions 19 to 127 of SEQ ID NO: 1, a polypeptide having the amino acids at positions 21 to 130 of SEQ ID NO: 1, a polypeptide having the amino acids at positions 19 to 130 of SEQ ID NO: 1, a polypeptide having the amino acids at positions 21 to 131 of SEQ ID NO: 1, a polypeptide having the amino acids at positions 19 to 131 of SEQ ID NO: 1, a polypeptide having the amino acids at positions 21 to 133 of SEQ ID NO: 1, a polypeptide having the amino acids at positions 19 to 133 of SEQ ID NO: 1, a polypeptide having the amino acids at positions 21 to 239 of SEQ ID NO: 1, or a polypeptide having the amino acids at positions 19 to 239 of SEQ ID NO: 1.

When the extracellular domain fragment of PD-L1 comprises an Ig V like domain or a fragment thereof, it may further comprise an immunoglobulin C (Ig C) like domain of the extracellular domain of PD-L1. The Ig C like domain may be a polypeptide having the amino acids at positions 133 to 225 of SEQ ID NO: 3, or a polypeptide having the amino acids at positions 134 to 225 of SEQ ID NO: 3.

When the extracellular domain fragment of PD-L1 comprises an Ig V like domain or a fragment thereof, it may further comprise a polypeptide comprising an Ig C like domain of the extracellular domain of PD-L1 or a fragment thereof. The polypeptide comprising the Ig C like domain refers to the extracellular domain of PD-L1 excluding the Ig V domain, and may be a polypeptide (SEQ ID NO: 47) having the amino acids at positions 134 to 239 of SEQ ID NO: 3, or a polypeptide (SEQ ID NO: 49) having the amino acids at positions 134 to 238 of SEQ ID NO: 3.

Particularly, human PD-L1 protein has 290 amino acid residues, and has the amino acid sequence represented by SEQ ID NO: 3 (Accession Number: Q9NZQ7). The sequence consisting of the amino acid residues at positions 1 to 18 of the N-terminus in the amino acid sequence of SEQ ID No: 3 is a signal sequence. Mature human PD-L1 is a protein consisting of the amino acids at positions 19 to 290 of SEQ ID NO: 3. The extracellular domain of human PD-L1 comprises the amino acid residues at positions 19 to 238 of SEQ ID NO: 3 or the amino acid residues at positions 19 to 239 of SEQ ID NO: 3.

The human PD-L1 protein comprises the Ig V like domain consisting of the amino acids at positions 19 to 127 of SEQ ID NO: 3 and the Ig V like domain consisting of the amino acids at positions 134 to 226 of SEQ ID NO: 3.

It was reported that mouse PD-L1 comprises 290 amino acids and has the amino acid sequence represented by SEQ ID NO: 1 (Accession Number: Q9EP73). The sequence consisting of the amino acid residues at positions 1 to 18 of SEQ ID NO: 1 is a signal sequence, and mature mouse PD-L1 protein consists of the amino acids at positions 19 to 290 of SEQ ID NO: 1.

A sequence consisting of the amino acids at positions 19 to 239 of SEQ ID NO: 1 is an extracellular domain. Mouse PD-L1 protein consists of the Ig V like protein consisting of the amino acids at positions 19 to 127 of SEQ ID NO: 1 and the Ig C like domain consisting of the amino acids at positions 133 to 224 of SEQ ID NO: 1.

In an embodiment, the extracellular domain fragment of PD-L1 may comprise the Ig V like domain or a fragment thereof. In addition, the extracellular domain fragment of PD-L1 may further comprise an Ig C like domain or a polypeptide comprising the Ig C like domain (i.e., PD-L1 extracellular domain excluding the Ig V like domain).

The PD-L1 extracellular domain or a fragment thereof may comprise various modified proteins or peptides. The modification may be substitution, deletion or addition of at least one amino acid in wild-type PD-L1, as long as it does not change the function of PD-L1. Such various proteins or peptides may have a sequence homology of 70%, 75%, 80%, 85%, 90%, 91% 92%, 93%, 94%, 95%, 96%, 97%, 98% or 99% to the wild-type protein.

Conventionally, the amino acid residue of the wild-type protein may be substituted with alanine, or a conservative amino acid which imposes little or no effect on the net charge, polarity, or hydrophobicity of the entire protein.

Conservative amino acid substitutions are set forth in Table 1 below, for reference.

Table 1

| | |
|---|---|
| asic | arginine (Arg, R), lysine (lys, K), histidine (His, H) |
| Acidic | glutamic acid (Glu, E), aspartic acid (Asp, D) |
| Uncharged polar | glutamine (Gln, Q), asparagine (Asn, N), serine (Ser, S), threonine (Thr, T), tyrosine (Tyr, Y) |
| Non-polar | phenylalanine (Phe, F), tryptophan (Trp, W), cysteine (Cys, C), glycine (Gly, G), alanine (Ala, A), valine (Val, V), proline (Pro, P), methionine (Met, M), leucine (Leu, L), norleucine, isoleucine |

For each amino acid, an additional conservative substitution includes a homolog of the amino acid. As used herein, the term "homolog" refers to an amino acid having a methylene group ($CH_2$) inserted in the beta position of the side chain of the amino acid.

Examples of such "homolog" include, but are not limited to, homophenylalanine, homoarginine, homoserine, etc. As used herein, the term "extracellular domain of PD-L1" is intended to include the extracellular domain of PD-L1 and a fragment thereof. The terms "protein," "polypeptide" and "peptide" may be used interchangeably with one another unless otherwise specified.

As used herein, each of the terms "PD-L1 fusion protein" and "Fc region fusion protein of PD-L1-modified immunoglobulin" refers to a fusion protein wherein the PD-L1 protein, the extracellular domain of PD-L1, or a fragment thereof, is coupled to a modified immunoglobulin Fc region.

The extracellular domain of PD-L1 may have the sequence of SEQ ID NO: 41.

In addition, two PD-L1-Fc conjugates may form a dimer. Herein, each Fc domain may be linked to the extracellular domain of PD-L1 to form a dimer. PD-L1 may be linked to the Fc domain directly or via a linker.

The extracellular domain of PD-L1 may comprise a polypeptide having 96 to 115 consecutive amino acid residues counted from position 19 of the N-terminus in the direction to the C-terminus, among the amino acid residues at positions 19 to 133 of SEQ ID NO: 3.

Preferably, the extracellular domain of PD-L1 comprises a polypeptide selected from the group consisting of a polypeptide having the amino acids at positions 19 to 133 of SEQ ID NO: 3, a polypeptide having the amino acids at positions 19 to 131 of SEQ ID NO: 3, a polypeptide having the amino acids at positions 19 to 130 of SEQ ID NO: 3, a polypeptide having the amino acids at positions 19 to 127 of SEQ ID NO: 3, a polypeptide having the amino acids at positions 19 to 120 of SEQ ID NO: 3, and a polypeptide having the amino acids at positions 19 to 114 of SEQ ID NO: 3.

In addition, the extracellular domain of PD-L1 may comprise a polypeptide consisting of the amino acids at positions 19 to 239 of SEQ ID NO: 1. In addition, the extracellular domain of PD-L1 may comprise a polypeptide consisting of the amino acids at positions 19 to 133 of SEQ ID NO: 1.

The PD-L1 protein may have about 70%, 75%, 80%, 85%, 90%, 91%, 92%, 93%, 94%, 95%, 96%, 97%, 98%, 99% or more homology to the sequence of SEQ ID NO: 1 or 3.

In addition, an IgC like domain of PD-L1 or a polypeptide comprising the Ig C like domain may be linked to the extracellular domain of PD-L1 or a fragment thereof via a linker.

The linker may be a polypeptide consisting of 1-10 amino acids, preferably 3-6 amino acids. The amino acids of the linker may be selected from the group consisting of leucine (Leu, L), isoleucine (Ile, I), alanine (Ala, A), valine (Val,V), proline (Pro, P), lysine (Lys, K), arginine (Arg, R), asparagine (Asn, N), and glutamine (Gln, Q).

The linker may comprise an amino acid sequence of VKV, KVN, VNA and NAP. Preferably, the linker may have the amino acid sequence of SEQ ID NO: 9, 45 or 46.

In addition, the linker may be a polypeptide consisting of 3-15 amino acids which consist of glycine (Gly, G) and serine (Ser, S) residues, preferably 6-11 amino acids. In an embodiment of the present invention, the linker may comprise an amino acid sequence of GSGGGS or GSGGGGSGGGS.

The fusion protein of the present invention may further comprise a linker. Herein, the extracellular domain of PD-L1 and the modified immunoglobulin Fc may be linked to each other via the linker. The linker may be linked to the N-terminus, C-terminus or free radical of the Fc fragment, and may also be linked to the N-terminus, C-terminus or free radical of PD-L1. When the linker is a peptide linker, the linkage may take place at a certain linking site. When the linker and the Fc are coupled to each other after they are separately expressed, the coupling may be conducted using a certain cross-linking agent known in the art. Examples of the cross-linking agents include, but are not limited to, 1,1-bis(diazoacetyl)-2-phenylethane, glutaraldehyde, N-hydroxysuccinimide ester such as 4-azidosalicylic acid, imidoesters including disuccinimidyl esters such as 3,3'-dithiobis(succinimidylpropionate), and bifunctional maleimide such as bis-N-maleimido-1,8-octane.

In addition, the linker may also be an albumin linker or a peptide linker. The peptide linker may be a peptide consisting of 10-20 amino acid residues which consist of Gly and Ser residues. The peptide linker may also be a peptide consisting of 1-10 amino acids selected from the group consisting of leucine (Leu, L), isoleucine (Ile, I), alanine (Ala, A), valine (Val,V), proline (Pro, P), lysine (Lys, K), arginine (Arg, R), asparagine (Asn, N), serine (Ser, S) and glutamine (Gln, Q).

The linker may comprise the amino acid sequence of SEQ ID NO: 8, 9, 45 or 46.

In an embodiment, the fusion protein may be represented by the following formula (I) or (I'):

$$(FT)_{w1} - X1 - (L1)_{w2} - (X2)_{w3} - (L2)_{w4} - IgFc \qquad (I)$$

$$IgFc - (L2)_{w4} - (FT)_{w1} - X1 - (L1)_{w2} - (X2)_{w3} \qquad (I'),$$

wherein FT is a dipeptide consisting of phenylalanine and threonine;
w1, w2, w3 and w4 are each 0 or 1;
X1 is an Ig V like domain of PD-L1, which comprises a polypeptide having the amino acid sequence of SEQ ID NO: 48 or 50;
L1 and L2 are each a linker;
X2 is a polypeptide comprising an immunoglobulin C (Ig C) like domain of PD-L1, or a fragment thereof; and
IgFc is a modified immunoglobulin Fc region.

In addition, the polypeptide comprising the Ig C like domain of PD-L1 may have the amino acid sequence of SEQ ID NO: 47 or 49.

L1 in the formula may consist of 1 to 10 amino acids, preferably 3 to 6 amino acids. The amino acid may be selected from the group consisting of leucine (Leu, L), isoleucine (Ile, I), alanine (Ala, A), valine (Val, V), proline (Pro, P), lysine (Lys, K), arginine (Arg, R), asparagine (Asn, N), and glutamine (Gln, Q). L1 may have the amino acid sequence of SEQ ID NO: 9, 45 or 46.

L2 in the formula may be a polypeptide consisting of 10 to 20 amino acids, which consists of glycine (Gly, G) and serine (Ser, S) residues; or a polypeptide consisting of 1 to 10 amino acids selected from the group consisting of leucine (Leu, L), isoleucine (Ile, I), alanine (Ala, A), valine (Val,V), proline (Pro, P), lysine (Lys, K), arginine (Arg, R), asparagine (Asn, N), and glutamine (Gln, Q). L2 may have the amino acid sequence of SEQ ID NO: 8, 9, 45 or 46.

Herein, w1, w2, w3 or w4 may be 0 or 1. Specifically, when w2 is 0, the Ig V like domain of PD-L1 may be directly coupled to the peptide comprising an Ig C like domain, or a fragment thereof. When w2 and w3 are each 1, the fusion protein may have a structural formula represented by the following formula (I-a) or (I'-a):

$$(FT)_{w1}\text{-}X1\text{-}L1\text{-}X2\text{-}(L2)_{w4}\text{-}IgFc \qquad (I\text{-}a),$$

or

$$\text{IgFc-(L2)}_{w4}\text{-(FT)}_{w1}\text{-X1-L1-X2} \qquad \text{(I'-a).}$$

Herein, FT-X1-L1-X2 may have the amino acid sequence of SEQ ID NO: 41.
In addition, when w3 and w4 are each 0, the fusion protein may have a structural formula represented by the following formula (I-b) or (I'-b):

$$\text{(FT)}_{w1}\text{-X1-L1-IgFc} \qquad \text{(I-b),}$$

or

$$\text{IgFc-(FT)}_{w1}\text{-X1-L1} \qquad \text{(I'-b).}$$

Herein, FT-X1-L1 may have the amino acid sequence of SEQ ID NO: 40.
In addition, when w2, w3 and w4 are each 0, the fusion protein may have a structural formula represented by the following formula (I-c) or (I'-c):

$$\text{(FT)}_{w1}\text{-X1-IgFc} \qquad \text{(I-c),}$$

or

$$\text{IgFc-(FT)}_{w1}\text{-X1} \qquad \text{(I'-c).}$$

Herein, FT-X1 may have the amino acid sequence of SEQ ID NO: 39.
In addition, the modified immunoglobulin Fc region ("IgFc" in Formula (I), (I'), (I-a), (I'-a), (I-b) and (I'-b)) may be any one of the Fc regions of IgG1, IgG2, IgG3, IgD and IgG4, or a combination of thereof.
The Fc region is modified such that the Fc region does not bind to Fc receptor and/or complements. Particularly, the modified immunoglobulin Fc region comprises a hinge region, a CH2 domain and a CH3 domain, which are arranged from the N-terminus to the C-terminus. The hinge region may comprise a human IgD hinge region, the CH2 domain may comprise an amino acid residue portion of the CH2 domain of human IgD and human IgG4, and the CH3 domain may comprise an amino acid residue portion of the CH3 domain of human IgG4.
As used herein, the term "Fc region," "Fc fragment" or "Fc" refers to a protein that comprises the heavy-chain constant region 2 (CH2) and heavy-chain constant region 3 (CH3) of immunoglobulin but does not comprise the heavy-chain and light-chain variable regions and light-chain constant region 1 (CL1) of immunoglobulin. The protein may further comprise a hinge region of the heavy-chain constant region. "Hybrid Fc" or "hybrid Fc fragment" may also herein be referred to as "hFc" or "hyFc." As used herein, the term "Fc region variant" refers to a Fc region prepared by substituting a part of the amino acids of the Fc region or combining Fc regions of different types. The Fc region variant may be modified to prevent cleavage at the hinge region. Specifically, the amino acid at position 144 and/or 145 of SEQ ID NO: 4 may be mutated. Preferably, it may be a variant wherein the amino acid K at position 144 of SEQ ID NO: 4 is substituted with G or S, and the amino acid E at position 145 of SEQ ID NO: 4 is substituted with G or S.
The modified immunoglobulin Fc region or Fc region variant may be represented by the following formula:

$$\text{N'-(Z1)p-Y-Z2-Z3-Z4-C'}$$

wherein N' is the N-terminus of a polypeptide, and C' is the C-terminus of a polypeptide;
p is an integer of 0 or 1; and
Z1 is an amino acid sequence having 5 to 9 consecutive amino acid residues counted from position 98 in the direction to the N-terminus, among the amino acid residues at positions 90 to 98 of SEQ ID NO: 4,
Y is an amino acid sequence having 5 to 64 consecutive amino acid residues counted from position 162 in the direction to the N-terminus, among the amino acid residues at positions 99 to 162 of SEQ ID NO: 4,

Z2 is an amino acid sequence having 4 to 37 consecutive amino acid residues counted from position 163 in the direction to the C-terminus, among the amino acid residues at positions 163 to 199 of SEQ ID NO: 4,
Z3 is an amino acid sequence having 71 to 106 consecutive amino acid residues counted from position 220 in the direction to the N-terminus, among the amino acid residues at positions 115 to 220 of SEQ ID NO: 5, and
Z4 is an amino acid sequence having 80 to 107 consecutive amino acid residues counted from position 221 in the direction to the C-terminus, among the amino acid residues at positions 221 to 327 of SEQ ID NO: 5.

The modified Ig Fc domain may be one disclosed in US Patent No. 7,867,491, and production of the modified Ig Fc domain may be conducted with reference to the disclosure of US Patent No. 7,867,491.
In addition, the Fc fragment in the present invention may be in the form having native sugar chains, increased sugar chains compared to a native form or decreased sugar chains compared to the native form, or may be in a deglycosylated form. The increase, decrease or removal of the immunoglobulin Fc sugar chains may be achieved by conventional methods known in the art, such as a chemical method, an enzymatic method and a genetic engineering method using a microorganism, etc. The removal of a sugar chain from an Fc region results in a sharp decrease in binding affinity of C1, a first complement component, to C1q, and a decrease or loss in antibody-dependent cell-mediated cytotoxicity (ADCC) or complement-dependent cytotoxicity (CDC), thereby not inducing unnecessary immune responses *in vivo.* In this regard, an immunoglobulin Fc fragment in a deglycosylated or aglycosylated form may be more suitable for the object of the present invention as a drug carrier. As used herein, the term "deglycosylation" means enzymatic removal of a sugar moiety from a Fc fragment, and the term "aglycosylation" means that a Fc fragment is produced in an unglycosylated form by a prokaryote, preferably *E. coli.*
In addition, the modified immunoglobulin Fc region may comprise the amino acid sequence of SEQ ID NO: 6 (hyFc), 7 (hyFcM1), 42 (hyFcM2), 43 (hyFcM3), or 44 (hyFcM4). Furthermore, the modified immunoglobulin Fc region may comprise the amino acid sequence of SEQ ID NO: 2 (nonlytic mouse Fc).
The extracellular domain of PD-L1 may be coupled to the N-terminus or C-terminus of the modified immunoglobulin Fc region. The fusion protein comprising PD-L1 extracellular domain-modified immunoglobulin Fc region may have the amino acid sequences of SEQ ID NOS: 10 to 23. In an embodiment, the fusion protein comprising PD-L1 extracellular domain-modified immunoglobulin Fc region may have the amino acid sequence of SEQ ID NO: 12, 13, 18 or 19. In another embodiment, the fusion protein comprising PD-L1 extracellular domain-modified immunoglobulin Fc region may have the amino acid sequence of SEQ ID NO: 14, 15, 16, 17, 20, 21, 22 or 23.
In still another embodiment, the fusion protein comprising PD-L1 extracellular domain-modified immunoglobulin Fc region may have a sequence having a sequence homology of 70%, 75%, 80%, 85%, 90%, 91% 92%, 93%, 94%, 95%, 96%, 97%, 98%, 99% or more to the amino acid sequences of SEQ ID NOS: 10 to 23.
In another aspect of the present invention, there is provided an isolated nucleic acid molecule encoding the fusion protein.
The nucleic acid molecule may encode a polypeptide having an amino acid sequence selected from the group consisting of SEQ ID NOS: 10 to 23. The nucleic acid molecule may comprise a polynucleotide having a nucleotide sequence selected from the group consisting of SEQ ID NOS: 24 to 37.
The nucleic acid molecule may further comprise a signal sequence or a leader sequence.
As used herein, the term "signal sequence" refers to a fragment that directs the secretion of a biologically active molecular agent or the fusion protein, which is cleaved after its translation in a host cell. The signal sequence in the present invention is a polynucleotide encoding an amino acid sequence that initiates transport of a protein across the membrane of the endoplasmic reticulum (ER). Signal sequences useful in the present invention include antibody light-chain signal sequences, e.g., antibody 14.18 (Gillies et al., J. Immunol. Meth 1989. 125:191-202), antibody heavy-chain signal sequences, e.g., the MOPC141 antibody heavy-chain signal sequence (Sakano et al., Nature 1980. 286: 676-683), and other signal sequences known in the art (*see* Watson et al., Nucleic Acid Research 1984. 12:5145-5164, for example).
A signal peptide has been well characterized in the art and is generally known to contain 16 to 30 amino acid residues, but it may contain greater or smaller number of amino acid residues. A typical signal peptide consists of three regions: a basic N-terminal region, a hydrophobic central region, and a more polar C-terminal region.
The hydrophobic central region contains 4 to 12 hydrophobic residues that anchor the signal sequence across the membrane lipid bilayer during transport of the immature polypeptide. Following initiation, the signal sequence is usually cleaved within the lumen of the endoplasmic reticulum by a cellular enzyme known as signal peptidase. The signal sequence may be a secretory signal sequence of tPa (tissue Plasminogen Activator), HSV gDs, or a growth hormone. Preferably, the signal sequence may be a secretory signal sequence that is used in higher eukaryotic cells, including mammalian cells. More preferably, it may be tPa sequence or an amino acid sequence consisting of the amino acids at positions 1 to 18 of SEQ ID NO: 1 or 3. Most preferably, the signal sequence may comprise the DNA sequence of SEQ ID NO: 38. In addition, a codon of the secretory signal sequence used in the present invention may be substituted with a codon having a high expression frequency in a host cell.
In still another aspect of the present invention, there is provided an expression vector comprising an isolated nucleic acid molecule encoding the fusion protein composed of the extracellular domain of PD-L1 and the modified immunoglobulin Fc region.

As used herein, the term "vector" is understood to refer to a nuclear acid vehicle which can be introduced into a host cell to be recombined with and integrated into the host cell genome, or which comprises a nucleotide sequence capable of replicating autonomously as an episome. Such vectors include linear nucleic acids, plasmids, phagemids, cosmids, RNA vectors, viral vectors and the like. Examples of the viral vector include, but are not limited to, a retrovirus, an adenovirus and an adeno-associated virus.

As used herein, the term "host cell" refers to a prokaryotic or eukaryotic cell into which the recombinant expression vector can be introduced. As used herein, the terms "transformed" and "transfected" are intended to encompass the introduction of a nucleic acid (e.g. a vector) into a cell by a number of techniques known in the art.

As used herein, the term "gene expression" or "expression" of a target protein is understood to mean the transcription of a DNA sequence, the translation of the mRNA transcript, and the secretion of an Fc fusion protein product or an antibody or an antibody fragment.

A useful expression vector may be RcCMV (Invitrogen, Carlsbad) or a variant thereof. The useful expression vector may carry human cytomegalovirus (CMV) promoter for constitutive transcription of the target gene in mammalian cells, and a bovine growth hormone polyadenylation signal sequence to increase steady state level of RNA after transcription. In an embodiment of the present invention, the expression vector is pAD15, which is a modified vector of RcCMV.

In still another aspect of the present invention, there is provided a host cell comprising the expression vector. An appropriate host cell can be transformed or transfected with a DNA sequence of the present invention, and can be utilized for the expression and/or secretion of the target protein. Currently preferred host cells for use in the present invention include immortal hybridoma cells, NS/0 myeloma cells, 293 cells, Chinese hamster ovary cells (CHO cells), HeLa cells, CapT cells (Human amniotic fluid derived cells), and COS cells.

One expression system that is used for the high level expression of fusion proteins or an antibody or an antibody fragment in a mammalian cell is a DNA construct encoding, in the 5' to 3' direction, a secretion cassette including a signal sequence and an immunoglobulin Fc region.

In still another aspect of the present invention, there is provided a pharmaceutical composition for preventing or treating an immune disease, which comprises a fusion protein composed of the extracellular domain of PD-L1 or a fragment thereof and a modified immunoglobulin Fc region.

Herein, the immune disease may be selected from the group consisting of an autoimmune disease, an inflammatory disease, and a transplantation rejection disease of a cell, a tissue or an organ.

The autoimmune disease may be selected from the group consisting of arthritis [acute arthritis, chronic rheumatoid arthritis, gouty arthritis, acute gouty arthritis, chronic inflammatory arthritis, degenerative arthritis, infectious arthritis, Lyme arthritis, proliferative arthritis, psoriatic arthritis, vertebral arthritis, and rheumatoid arthritis such as juvenile-onset rhematoid arthritis, osteoarthritis, arthritis chronica progrediente, arthritis deformans, polyarthritis chronica primaria, reactive arthritis, and ankylosing spondylitis], psoriasis such as inflammatory hyperproliferative skin diseases, plaque psoriasis, gutatte psoriasis, pustular psoriasis, and psoriasis of the nails, dermatitis including contact dermatitis, chronic contact dermatitis, allergic dermatitis, allergic contact dermatitis, herpetiformis dermatitis, and atopic dermatitis, X-linked hyper-IgM syndrome, urticaria such as chronic allergic urticaria and chronic idiopathic urticaria, including chronic autoimmune urticaria, polymyositis/dermatomyositis, juvenile dermatomyositis, toxic epidermal necrolysis, scleroderma (including systemic scleroderma), systemic sclerosis, sclerosis including multiple sclerosis (MS) such as spino-optical MS, primary progressive MS (PPMS) and relapsing remitting MS (RRMS), progressive systemic sclerosis, atherosclerosis, arteriosclerosis, sclerosis disseminata, and ataxic sclerosis, inflammatory bowel disease (IBD) [e.g., Crohn's disease, autoimmune-mediated gastrointestinal disease, colitis such as ulcerative colitis, colitis ulcerosa, microscopic colitis, collagenous colitis, colitis polyposa, necrotizing enterocolitis, and transmural colitis, and autoimmune inflammatory bowel disease], pyoderma gangrenosum, erythema nodosum, primary sclerosing cholangitis (episcleritis), respiratory distress syndrome including adult or acute respiratory distress syndrome (ARDS), meningitis, inflammation of all or part of the uvea, iritis, choroiditis, autoimmune hematological disorder, rheumatoid spondylitis, acute hearing loss, IgE-mediated disease such as anaphylaxis and allergic and atopic rhinitis, encephalitis such as Rasmussen's encephalitis and limbic and/or brainstem encephalitis, uveitis such as anterior uveitis, acute anterior uveitis, granulomatous uveitis, nongranulomatous uveitis, phacoantigenic uveitis, posterior uveitis, or autoimmune uveitis, glomerulonephritis (GN) with or without nephrotic syndrome such as chronic or acute glomerulonephritis such as primary GN, immune-mediated GN, membranous GN (membranous nephropathy), idiopathic membranous nephropathy or idiopathic membranous GN, membrano- or membranous proliferative GN (MPGN) including Type I and Type II, and rapidly progressive GN, allergic diseases, allergic reaction, eczema including allergic or atopic eczema, asthma such as asthma bronchiale, bronchial asthma and autoimmune asthma, disease related with T cell infiltration and chronic inflammatory response, chronic pulmonary inflammatory disease, autoimmune myocarditis, leukocyte adhesion deficiency, systemic lupus erythematosus (SLE) or systemic lupus erythematosus such as cutaneous SLE, subacute cutaneous lupus erythematosus, neonatal lupus syndrome (NLE), lupus erythematosus disseminatus, lupus [including nephritis, cerebritis, pediatric, non-renal, extra-renal, discoid, and alopecia], juvenile-onset (Type I) diabetes mellitus including pediatric insulin-dependent diabetes mellitus (IDDM), adult-onset (Type II) diabetes mellitus, autoimmune diabetes mellitus, idiopathic diabetes insipidus, immune responses associated with acute and delayed hypersensitivity mediated by T-lymphocytes and cytokines, granulomatosis including tuberculosis, sarcoidosis, lymphomatoid granulomatosis, Wegener's granulomatosis, agranulocytosis, vasculitis [including giant vessel vasculitis (polymyalgia rheumatic and Takayasu's arteritis], Kawasaki disease, medium vessel vasculitis including polyarteritis nodosa, microscopic polyarteritis, CNS arthritis, necrotizing, cutaneous or hypersensitivity vasculitis, systemic necrotizing vasculitis, vasculitides including ANCA-related vasculitis such as Churg-Strauss vasculitis or syndrome (CSS), temporal arteritis, aplastic anemia, autoimmune aplastic anemia, coombs benign anemia, Diamond Blackfan anemia, immune-hemolytic anemia including hemolytic anemia or autoimmune hemolytic anemia (AIHA), pernicious anemia (anemia perniciosa), Addison's disease, pure red cell anemia aplasia (PRCA), factor VHI deficiency, hemophilia A, autoimmune neutropenia, pancytopenia, leukopenia, leukocyte diapedesis-related disease, CNS inflammatory disorder, multiple organ injury syndrome such as those secondary to septicemia, trauma or hemorrhage, antigen-antibody complex-mediated diseases, anti-glomerular basement membrane disease, anti-phospholipid antibody syndrome, allergic neuritis, Bechet's or Behcet's disease, Castleman's syndrome, Goodpasture's syndrom, Reynaud's syndrome, Sjogren's syndrome, Stevens-Johnson syndrome, pemphigoid such as bullous pemphigoid and skin pemphigoid, pemphigus (including pemphigus vulgaris, pemphigus foliaceus, pemphigus mucus-membrane pemphigoid, and pemphigus erythematosus), autoimmune polyendocrinopathies, Reiter's disease or syndrome, immune complex nephritis, antibody-mediated nephritis, neuromyelitis optica, polyneuropathies, chronic neuropathy such as IgM polyneuropathy or IgM-mediated neuropathy, thrombocytopenia (e.g., one which develops in myocardial infarction patient), including thrombotic thrombocytopenic purpura (TTP) and autoimmune or immune-mediated thrombocytopenia such as idiopathic thrombocytopenic purpura (ITP) including chronic or acute ITP, autoimmune disorder of the testis and ovary including autoimmune orchitis and oophoritis, primary hypothyroidism, hypothyroidism including thyroiditis such as autoimmune thyroiditis, autoimmune endocrine disease, Hashimoto's disease, chronic thyroiditis (Hashimoto's thyroiditis) or subacute thyroiditis, autoimmune thyroid disease, idiopathic hypothyroidism, Grave's disease, polyglandular syndrome such as autoimmune polyglandular syndrome (or polyglandular endocrinopathy syndrome), paraneoplastic syndromes including paraneoplastic neurological syndrome such as Lambert-Eaton myasthenic syndrome or Eaton-Lambert syndrome, stiff-man or stiff-person syndrome, encephalomyelitis such as allergic encephalomyelitis or encephalomyelitis allergica, and experimental allergic encephalomyelitis (EAE), myasthenia gravis such as thymoma-associated myasthenia gravis, cerebellar degeneration, neuromyotonia, opsoclonus or opsoclonus myoclonus syndrome (OMS), and sensory neuropathy, multifocal motor neuropathy, Sheehan's syndrome, autoimmune hepatitis, chronic hepatitis, lupoid hepatitis, giant cell hepatitis, chronic active hepatitis or autoimmune chronic active hepatitis, lymphoid interstitial pneumonitis, bronchiolitis obliterans (non-transplant) vs. NSIP, Guillain-Barre syndrome, Berger's disease (IgA nephropathy), idiopathic IgA nephropathy, linear IgA dermatosis, primary biliary cirrhosis, pneumonocirrhosis, autoimmune enteropathy syndrome, Celiac disease, Coeliac disease, celiac sprue (gluten enteropathy), refractory sprue, idiopathic sprue, cryoglobulinemia, amyotrophic lateral sclerosis (ALS, Lou Gehrig's disease), coronary artery disease, autoimmune ear disease such as autoimmune inner ear disease (AGED), autoimmune hearing loss, opsoclonus myoclonus syndrome (OMS), polychondritis such as refractory or relapsing polychondritis, pulmonary alveolar proteinosis, amyloidosis, scleritis, non-cancerous lymphocytosis, primary lymphocytosis including monoclonal B cell lymphocytosis (e.g., benign monoclonal gammopathy and monoclonal gammopathy of undetermined significance; MGUS), peripheral neuropathy, paraneoplastic syndrome, epilepsy, migraine, arrhythmia, muscular disorder, deafness, blindness, periodic paralysis, channelopathies such as CNS channelopathies, autism, inflammatory myopathy, focal segmental glomerulosclerosis (FSGS), endocrine opthalmopathy, uveoretinitis, chorioretinitis, autoimmune hepatological disorder, fibromyalgia, multiple endocrine failure, Schmidt's syndrome, adrenalitis, gastric atrophy, presenile dementia, demyelinating diseases such as autoimmune demyelinating diseases, diabetic nephropathy, Dressler's syndrome, alopecia areata, CREST syndrome (calcinosis), Raynaud's phenomenon, esophageal dysmotility, sclerodactyly, and telangiectasia, male and female infertility, mixed connective tissue disease, Chagas' disease, rheumatic fever, recurrent abortion, farmer's lung, erythema multiforme, post-cardiotomy syndrome, Cushing's syndrome, bird-fancier's lung, allergic granulomatous angiitis, benign lymphocytic angiitis, Alport's syndrome, alveolitis such as allergic alveolitis and fibrous periostitis, interstitial lung disease, transfusion diseases, leprosy, malaria, leishmaniasis, kypanosomiasis, schistosomiasis, ascariasis, aspergillosis, Sampter's syndrome, Caplan's syndrome, dengue, endocarditis, endomyocardial fibrosis, diffuse interstitial pulmonary fibrosis, interstitial lung fibrosis, idiopathic pulmonary fibrosis, cystic fibrosis, endophthalmitis, erythema elevatum et diutinum, erythroblastosis fetalis, eosinophilic fasciitis, Shulman's syndrome, Felty's syndrome, filariasis, cyclitis such as chronic cyclitis, heterochromia chronic cyclitis, iridocyclitis or Fuch's cyclitis, Henoch-Schonlein purpura, human immunodeficiency virus (HIV) infection, ECHO virus infection, cardiomyopathy, Alzheimer's disease, parvovirus infection, rubella virus infection, post-vaccination syndromes, congenital rubella infection, Epstein-Barr virus infection, mumps, Evan's syndrome, autoimmune gonadal failure, Sydenham's chorea, poststreptococcal nephritis, thromboangitis obliterans, thyrotoxicosis, tabes dorsalis, chorioiditis, giant cell polymyalgia, endocrine ophthamopathy, chronic hypersensitivity pneumonitis, keratoconjunctivitis sicca, epidemic keratoconjunctivitis, idiopathic nephritic syndrome, minimal change nephropathy, benign familial and ischemia-reperfusion injury, retinal autoimmunity, joint inflammation, bronchitis, chronic obstructive airway disease, silicosis, aphthae, aphthous stomatitis, arteriosclerotic disorders, aspermiogenese, autoimmune hemolysis, Boeck's disease, cryoglobulinemia, Dupuytren's contracture, endophthalmia phacoanaphylactica, enteritis allergica, erythema nodosum leprosum, idiopathic facial paralysis, chronic fatigue syndrome, febris rheumatica, Hamman-Rich's disease, sensorineural hearing loss, haemoglobinuria paroxysmatica, hypogonadism, ileitis regionalis, leucopenia, mononucleosis infectiosa, transverse myelitis, primary idiopathic myxedema, nephrosis, ophthalmia symphatica, orchitis granulomatosa, pancreatitis, polyradiculitis acuta, pyoderma gangrenosum, Quervain's thyroiditis, acquired spenic atrophy, infertility due to antispermatozoan antobodies, non-malignant thymoma, vitiligo, SCID and Epstein-Barr virus-associated diseases, acquired immune deficiency syndrome (AIDS), parasitic disease such as Lesihmaniasis, toxic-shock syndrome, food poisoning, disease associated with T cell infiltration, leukocyte adhesion deficiency, immune responses associated with acute and delayed hypersensitivity mediated by cytokines and T cells, leukocyte diapedesis-related disease, multiple organ injury syndrome, antigen-antibody complex mediated diseases, antiglomerular basement membrane disease, allergic neuritis, autoimmune polyendocrinopathies, oophoritis, primary myxedema, autoimmune atrophic gastritis, sympathetic ophthalmia, rheumatic diseases, mixed connective tissue disease, nephrotic syndrome, insulitis, polyendocrine failure, peripheral neuropathy, autoimmune polyglandular syndrome type I, adult-onset idiopathic hypoparathyroidism (AOIH), alopecia totalis, dilated cardiomyopathy, epidermolysis bullosa acquisita (EBA), hemochromatosis, myocarditis, nephrotic syndrome, primary sclerosing cholangitis, purulent or non-purulent sinusitis, acute or chronic sinusitis, ethmoid, frontal, maxillary, or sphenoid sinusitis, eosinophilic disorder such as eosinophilia, pulmonary infiltration eosinophilia, eosinophilia-myalgia syndrome, Loffler's syndrome, chronic eosinophilic pneumonia, topical pulmonary eosinophilia, bronchopneumonic aspergillosis, aspergilloma, or granulomas including eosinophils, anaphylaxis, seronegative spondyloarthritides, polyendocrine autoimmune disease, sclerosing cholangitis, sclera, episclera, chronic mucocutaneous candidiasis, Bruton's syndrome, transient hypogammaglobulinemia of infancy, Wiskott-Aldrich syndrome, ataxia telangiectasia, autoimmune disorders associated with collagen diseases, rheumatism, neurological diseases, ischemic reperfusion disorder, reduction in blood pressure response, blood vessel malfunction, angiectasis, tissue injury, cardiovascular ischemia, hyperalgesia, cerebral ischemia, and disease accompanying vascularization, allergic hypersensitivity disorder, glomerulonephritides, reperfusion injury, reperfusion injury of myocardium or other tissues, dermatoses having acute inflammatory component, acute purulent meningitis or other central nervous system inflammatory disorder, ocular and orbital inflammatory disorder, granulocyte transfusion-associated syndromes, cytokine-induced toxicity, acute serious inflammation, chronic intractable inflammation, pyelitis, pneumonocirrhosis, diabetic retinopathy, diabetic large-artery disorder, endarterial hyperplasia, peptic ulcer, valvulitis, and endometriosis.

The inflammatory disease may be selected from the group consisting of rheumatic diseases (including, but not limited to, rheumatoid arthritis, osteoarthritis, and psoriatic arthritis), spondyloarthropathies (including, but not limited to, ankylosing spondylitis, reactive arthritis, and Reiter's syndrome), crystal arthropathies (including, but not limited to, gout, pseudogout, and calcium pyrophosphate deposition disease), Lyme disease, polymyalgia rheumatic; connective tissue diseases (including, but not limited to, systemic lupus erythematosus, systemic sclerosis, polymyositis, dermatomyositis, Sjogren's syndrome); vasculitides (including, but not limited to, polyarteritis nodosa, Wegener's granulomatosis, Churg-Strauss syndrome); inflammatory disease including the result of trauma or ischaemia; sarcoidosis; atherosclerotic vascular disease, atherosclerosis, vascular occlusive disease (including, but not limited to, atherosclerosis and ischaemic heart disease, myocardial infarction, stroke, and peripheral vascular disease), and vascular diseases including vascular stent restenosis; and ocular diseases including uveitis, corneal disease, iritis, iridocyclitis, and cataracts.

Preferably, the immune disease may be selected from the group consisting of an autoimmune disease, an inflammatory disease, a transplantation rejection disease, colitis, psoriasis, asthma, autoimmune diabetes, inflammatory bowel disease, and arthritis.

In still another aspect of the present invention, there is provided a composition for inducing immune tolerance, which comprises a fusion protein composed of the extracellular domain of PD-L1 and a modified immunoglobulin Fc region. As used herein, the term "immune tolerance" refers to a state which is not a continuous immune suppression state and in which the immune system shows no tissue destruction response to a specific antigen. Particularly, the fusion protein may be used for immune tolerance in which regulatory T cells are involved. Preferably, the fusion protein may be used to inhibit immune responses that occur upon tissue transplantation.

The fusion protein comprising the extracellular domain of PD-L1 or a fragment thereof may comprise a pharmaceutically acceptable carrier. The pharmaceutically acceptable carrier may be any carrier, as long as it is a non-toxic substance suitable for delivering an antibody to a patient. Examples of the carrier include sterile water, alcohol, fats, waxes, and inert solids. A pharmaceutically acceptable adjuvant (a buffering agent, a dispersing agent) may also be contained in the pharmaceutical composition.

In addition, the fusion protein comprising the extracellular domain of PD-L1 or a fragment thereof may be administered to a subject in various ways. For example, the pharmaceutical composition may be administered parenterally, e.g., subcutaneously, intramuscularly or intravenously. Such composition may be sterilized using a conventional sterilization technique well known in the art. The composition may contain a pharmaceutically acceptable auxiliary substance, a buffering agent, a toxicity adjusting agent, and the like, as required to adjust a physiological condition, such as pH, for example sodium acetate, sodium chloride, potassium chloride, calcium chloride, sodium lactate, etc. The concentration of the fusion protein in the dosage form can vary widely, e.g., less than about 0.5%, usually or at least about 1% to as much as 15 or 20% by weight, and may be selected primarily based on the fluid volume, viscosity, etc., in accordance with the particular mode of administration selected.

In still another aspect of the present invention, there is provided a method for treating a disease by administering a composition comprising the PD-L1 fusion protein as a pharmacologically active ingredient.

Such method comprises administering an effective amount of the PD-L1 fusion protein to a mammal having the health condition which may or may not be directly related to the disease of interest. For example, a nucleic acid, such as DNA or RNA, encoding a desired PD-L1 fusion protein, can be administered to a subject, preferably a mammal, as a therapeutic agent. Additionally, a cell containing the nucleic acids encoding the PD-L1 fusion protein can be administered to a subject, preferably a mammal, as a therapeutic agent. Furthermore, the PD-L1 fusion protein can be administered to a subject, preferably a mammal including human in a therapeutically effective amount. The chimeric polypeptide may be administered via an intravenous, subcutaneous, peroral, oral, sublingual, nasal, parenteral, rectal, vaginal or pulmonary route.

Compositions of the present invention may be administered via any route. The compositions of the present invention may be provided to an animal by any suitable means, directly (e.g., locally, by injection, implantation or topical administration to a tissue locus) or systemically (e.g., parenterally or perorally). Where the composition of the present invention is to be provided parenterally, for example, by intravenous, subcutaneous, ophthalmic, intraperitoneal, intramuscular, oral, rectal, intraorbital, intracerebral, intracranial, intraspinal, intraventricular, intrathecal, intracisternal, intracapsular, intranasal or by aerosol administration, the composition preferably includes part of an aqueous or physiologically compatible fluid suspension or solution. Accordingly, the carrier or vehicle is physiologically acceptable, which can be delivered to a patient as an additive to the composition without imposing an adverse effect on the patient's electrolyte and/or volume balance. Thus, the fluid medium for the formulation may generally include a physiologic saline.

A DNA construct (or gene construct) comprising a nucleic acid encoding the PD-L1 fusion protein of the present invention can be used as a part of a gene therapy protocol to deliver the nucleic acid encoding the PD-L1 fusion protein.

In the present invention, an expression vector for transfection and expression *in vivo* of the PD-L1 fusion protein in a specific type of cell can be administered with a certain biologically effective carrier in order to reconstitute or supplement the function of the desired PD-L1. For example, a certain dosage form or composition capable of effectively delivering a PD-L1 fusion protein-encoding gene or fusion protein construct thereof to cells *in vivo* can be used.

For the gene therapy using a nucleic acid encoding the PD-L1 fusion protein, the target gene may be inserted into a viral vector including a recombinant retrovirus, adenovirus, adeno-associated virus, and herpes simplex virus-1, or recombinant bacterial or eukaryotic plasmids. A dosage for administration of a nucleic acid encoding the fusion protein of the present invention is in the range of 0.1 to 100 mg for human. In one example, a preferred dosage for administration of a nucleic acid encoding the fusion protein of the present invention is in the range of 1 to 10 mg for human. In another example, a preferred dosage for administration of a nucleic acid encoding the fusion protein of the present invention is in the range of 2 to 10 mg for human. The optimal dosage and mode of administration may be determined by routine experimentation within the level of skills in the art.

A preferred unit dosage for administration of the fusion protein of the present invention is in the range of 0.1 to 1,500 mg/kg for human. In one example, a preferred unit dosage for administration of the fusion protein is in the range of 1 to 100 mg/kg for human. In another example, a preferred unit dosage for administration of the fusion protein is in the range of 5 to 20 mg/kg for human. It is understood that the optimal dosage of administration may be determined by routine experimentation. Administration of the fusion protein may carried out by periodic bolus injections, or by continuous intravenous, subcutaneous, or intraperitoneal administration from an external (e.g., from an intravenous bag) or internal (e.g., from a bioerodable implant) reservoir.

A composition of the present invention may be administered in combination with one or more other drugs or physiologically active substances which have the effect of preventing or treating the disease to be prevented or treated by the composition, or may be formulated in the form of a combination formulation with such other drugs or substances.

A method for preventing or treating a disease of interest using the fusion protein or composition of the present invention may also comprise administering one or more other drugs or physiologically active substances which have the effect of preventing or treating the disease in combination with the fusion protein or the composition of the present invention. Herein, the route, timing, and the dosage of administration may be determined depending on the type of a disease, the disease status of a patient, the purpose of treatment or prevention, and other drugs or physiologically active substances used in combination.

MODE FOR INVENTION

Hereinafter, the present invention is explained in detail by Examples. The following Examples are intended to further illustrate the present invention without limiting its scope.

## I. Preparation of PD-L1-Fc Fusion Protein Using Mouse PD-L1 and Mouse Fc Region and Analysis of Activity Thereof

### Example 1: Preparation of mPD-L1-mFc (mouse PD-L1-mouse non-lytic IgG2a) Gene Construct

Mouse PD-L1 protein (mPD-L1) and human PD-L1 protein (hPD-L1) have a sequence homology of about 70%. Thus, when human PD-L1 protein is repeatedly administered to a mouse, an antibody (anti-drug antibody, ADA) against the human PD-L1 protein might be developed, which could make it difficult to predict the accurate efficacy of the human PD-L1 protein, in some cases.

Thus, before an experiment was conducted using a human PD-L1-Fc fusion protein, the effect of a mouse PD-L1-Fc fusion protein was analyzed by an *in vivo* experiment in mice. Non-lytic Fc (hereinafter referred to as "mFc") was constructed to provide a gene construct capable of producing mPD-L1-mFc, and a cell line expressing mPD-L1-mFc was constructed. The recombinant protein-expressing cell line was suspension-cultured, and the culture medium was collected, after which the recombinant protein was recovered by column purification. The *in vitro* and *in vivo* effects of the obtained mPD-L1-mFc protein were evaluated.

Particularly, in order to construct a vector comprising the mouse PD-L1 gene, an extracellular domain of a known amino acid sequence (Accession number: Q9EP73, SEQ ID NO: 1) was used as the mouse PD-L1 (Programmed Cell Death-Ligand 1, mPD-L1) gene. In addition, as the fusion partner Fc region, a variant sequence was used such that it would not cause ADCC (antibody dependent cell cytotoxicity) and CDC (Complement Dependent Cytotoxicity). The constructed variant Fc region (SEQ ID NO: 2) of mouse IgG2a was fused to mPD-L1, to obtain an expression vector for production of the recombinant protein.

The pAD15-mPD-L1-mFc plasmid comprising the mPD-L1-mFc gene (SEQ ID NO: 24) was constructed as shown in Fig. 1 such that the gene can be expressed in an animal cell line. The constructed expression vector was transformed into a CHO (Chinese Hamster Ovary Cell)-DG44 cell line by electroporation. Among the transformed cell line, cells showing a high expression level of the gene were selected by ELISA quantitative analysis. A "MTX amplification-monoclonal selection-productivity measurement" process was repeated while the amount of methotrexate (MTX) was increased stepwise, to select a high expression cell line (Fig. 2(a)). The selected final cell line was suspension-cultured, and the target protein was verified by SE-HPLC (Fig. 2(b)).

### Example 2: Production of mPD-L1-mFc Protein

To produce the mPD-L1-mFc (mouse PD-L1-mouse non-lytic IgG2a) protein (SEQ ID NO: 10) in large amounts, the target protein was separated and purified from the cell culture medium produced by the mPD-L1-mFc suspension cell line obtained in Example 1.

To purify the protein, the protein purification process was monitored with time at a UV wavelength of 280 nm. As a result, elution of the target protein was verified by the peak that appeared when elution buffer (0.1M glycine, pH 3.0) passed through the protein A resin column (Fig. 3). In addition, the purified mPD-L1-mFc protein was analyzed by SDS-PAGE, and as a result, the protein size was about 150 KDa in a non-reducing condition and about 75 KDa in a reducing condition, indicating that mPD-L1-mFc is in the form of a homodimer (Fig. 3a). In addition, the purification product was analyzed by SE-HPLC to determine its purity (Fig. 3b), and the level of the impurity endotoxin was measured, thereby obtaining the purified target protein for use in the evaluation of the effects.

### Example 3: Evaluation of *in vitro* Activity of mPD-L1-mFc Protein

To evaluate the activity of the mPD-L1-mFc protein (SEQ ID NO: 10) purified in Example 2, the immunosuppressive effect of the protein was analyzed *in vitro* using mouse splenocytes.

As schematically shown in Fig. 4, anti-CD3 and the mPD-L1-mFc protein were coated on microbeads at a ratio of 1:1 or 1:4. In addition, the beads and the mouse splenocytes were used at a ratio of 10:1 to stimulate the splenocytes ($5 \times 10^6$ beads: $5 \times 10^5$ splenocytes).

Using the microbeads coated with the anti-CD3 antibody and the mPD-L1-mFc protein, the mouse splenocytes were stimulated in a microwell plate. After 48 hrs, the expression level of the cell proliferation factor Ki-67 in the mouse splenocytes was analyzed.

As a result, inhibition of the proliferation of the mouse splenocytes by the mPD-L1-mFc protein (reduction in Ki-67 expression) was observed, which indicates that the mPD-L1-mFc fusion protein has immunosuppressive activity (Fig. 5).

**Example 4: Evaluation of Effect of mPD-L1-mFc Protein in IBD (inflammatory bowel disease) mouse models**

**Example 4-1: Evaluation of Effect of mPD-L1-mFc in DSS-Induced Enteritis Models**

To evaluate the *in vivo* effect of the mPD-L1-mFc protein, DSS (Dextran Sodium Sulfate)-induced mouse enteritis model, which is similar to human acute inflammatory bowel disease model, was used as an IBD (inflammatory bowel disease) model.

In DSS-induced enteritis, it is known that inflammatory cells infiltrate into the colon. In order to examine the effect of the mPD-L1-mFc protein on the migration of innate immune cells to the colon, evaluation was conducted in the DSS (dextran sodium sulfate)-induced mouse enteritis model using Rag-1 knockout (KO) mice lacking T cells and B cells (The Jackson Laboratory, US). On day 2 after the start of supply of drinking water containing DSS, 30 $\mu$g of the mPD-L1-mFc protein (obtained in Example 2) was administered to each mouse intraperitoneally once.

As a result, as shown in Fig. 6, weight loss (Fig. 6(a)) and colon length reduction (Fig. 6(b)) in the group administered with the mPD-L1-mFc protein were alleviated as compared to the control group fed with DSS-containing drinking water alone. Meanwhile, on day 9 after feeding of DSS, the colon was isolated and subjected to H&E (Hematoxylin and Eosin) staining (Fig. 7a). A histological analysis of the isolated tissue was conducted in a blind manner, and as a result, the mice administered with the mPD-L1-mFc protein showed lower degree of colon tissue damage compared to the mice not administered with the mPD-L1-mFc protein, and maintained the tissue structure (Fig. 7(b)).

It was verified that the PD-L1-Fc fusion protein has a therapeutic effect on acute inflammatory bowel disease and also plays an important role in the protection and treatment of non-lymphoid cells of the bowel.

**Example 4-2: Evaluation of Effect of mPD-L1-mFc in Enteritis Model Induced by T Cells**

The *in vivo* activity of the mPD-L1-mFc protein in chronic mouse enteritis model induced by T cells was examined.

Using fluorescent activating cell sorting (FACS), CD4+CD25-CD45RB$^{high}$ T cells were separated from the splenocytes of C57BL/6 mice. Next, 5 x $10^5$ separated CD4+CD25-CD45RB$^{high}$ T cells were injected to Rag-1 KO mice lacking T cells and B cells intraperitoneally. From week 3 after the T cell injection, 20 $\mu$g of the mPD-L1-mFc protein obtained in Example 2 was injected to each mouse intraperitoneally at one-week intervals for a total of four times. As a control, 200 $\mu$g of CTLA4-IgG1 fusion protein (Orencia) was injected to each mouse intraperitoneally three times a week (a total of 12 times). For the dosage and mode of administration of the control, reference was made to International Journal of inflammation, 2012:412178. Throughout the entire experimental period, changes in the body weights of the mice were observed, and clinical scores in the mice were recorded.

The clinical score was determined based on the following items:

hunched posture (0 or 1), stool (0 to 3), and colon thickness (0 to 3).

As a result, in the case of the experimental group administered with mPD-L1-mFc, the weight loss was significantly reduced (Fig. 8(a)), and the clinical score also showed significant difference from the control group (Fig. 8(b)). Furthermore, although the total dosage of the mPD-L1-mFc protein was about 1/30 of the control CTLA-4-IgG1 (Orencia), the mPD-L1-mFc protein showed an effect similar to that of the control.

In addition, histological analysis was conducted in a blind manner after H&E (Hematoxylin and Eosin) staining (Fig. 9(a)), and as a result, it was shown that the degree of damage to the tissue was lower in the experimental group administered with the mPD-L1-mFc protein than in the control group (Fig. 9(b)).

The experiment was repeated by the same method as described above. As a result, reduction of the weight loss (Fig. 10) owing to the alleviation of enteritis by the administration of the mPD-L1-mFc protein in the chronic mouse enteritis model induced by T cells was verified. In addition, the expression of inflammatory cytokines in the colon was measured, and as a result, it was shown that the secretion of INF-g, IL-17 and IL-10 was inhibited (Fig. 11).

As shown in the above Examples, the mouse PD-L1-mFc fusion protein has the effect of inhibiting T cell proliferation, is effective for acute and chronic inflammatory diseases, and inhibits the expression of inflammatory cytokines found in the lesion. Namely, the above results indicate that the PD-L1-Fc protein is highly likely to be used as a therapeutic agent for IBD and an IBD-related inflammatory disease.

**Example 5: Examination of the Effect of mPD-L1-mFc Protein in Treatment of Psoriasis**

40 mg of Imiquimod (IMQ; ALDARA CREAM, 3M) was applied to both ears of experimental mice for 6 days to induce acute psoriasis. Mice were divided into a normal mouse group, a group not treated after induction of psoriasis, and a group administered with the mPD-L1-mFc protein along with induction of psoriasis, and then the therapeutic effect of mPD-L1-mFc was examined. On days 1, 2, 4 and 6, 200 μg of mPD-L1-mFc was administered to each mouse intraperitoneally.

On day 7 after the first administration, the ears were histopathologically examined (Fig. 12(a)), and the pathological tissues findings of psoriasis such as epidermal thickness, etc., in the IMQ-induced psoriasis group were comparatively observed (Fig. 12(b)). As a result, the epidermal thickness in the group administered with mPD-L1-mFc was reduced to a statistically significant level compared to the control group.

In addition, in order to examine the effect of mPD-L1-mFc in the acute psoriasis mouse model, 40 mg of Imiquimod (IMQ) was applied to both ears of experimental mice for 6 days to induce acute psoriasis. Mice were divided into a normal mouse group, a group not treated after induction of psoriasis, a group administered with mouse anti-p40 antibody, a group administered with the mPD-L1-mFc protein, and a group co-treated with anti-P40 antibody and mPD-L1-mFc, and then the therapeutic effect of the mPD-L1-mFc protein was examined. On days 1 and 4, 100 μg of anti-P40 antibody was administered to each mouse intraperitoneally. 200 μg of mPD-L1-mFc was administered to each mouse intraperitoneally on days 1, 2, 4 and 6.

After the first administration, the thickness of the ear was observed every day to examine the phenotype of psoriasis.

As a result, the groups administered with mPD-L1-mFc or anti-P40 antibody showed a statistically significant effect of delaying the development of psoriasis as compared to the untreated group (Fig. 13).

In addition, on day 7, the ears were histopathologically examined, and the pathological tissues of psoriasis such as epidermal thickness in the IMQ-induced psoriasis group were comparatively observed. As a result, it was shown that the epidermal thickness in the group administered with mPD-L1-mFc was reduced to a statistically significant level as compared to other groups not administered with mPD-L1-mFc (Fig. 14).

In addition, it was shown that the epidermal thickness was also reduced to a statistically significant level in the groups administered with mPD-L1-mFc as compared to other groups not administered with mPD-L1-mFc (Fig. 15).

These results indicate that the mPD-L1-mFc fusion protein has a therapeutic effect on psoriasis, and particularly, the use of mPD-L1-mFc in combination with anti-P40 antibody shows the best effect.

**Example 6: Observation of the Effect of mPD-L1-mFc Protein in Treatment of Rheumatoid Arthritis (RA)**

Using CIA (collagen induced arthritis) mouse model as a rheumatoid arthritis (RA) model, the therapeutic effect of mPD-L1-mFc was examined. CIA mice were obtained by administering a 1:1 mixture of complete Freund's adjuvant (CFA) and collagen to normal C57BL/6 mice (The Jackson Laboratory, US) at 2-week intervals to induce RA. The mice in CIA mouse model were divided into a group without drug administration, a group administered with 100 μg or 300 μg of mPD-L1-mFc (obtained in Example 2), and a group administered with 300 μg of anti-TNF-alpha antibody. Each of the drugs was administered intraperitoneally three times a week for 4 weeks, and then the therapeutic effect of mPD-L1-mFc was examined.

On day 37 after the first administration, phenotypes were observed by measuring the clinical score, changes in the ankle thickness, etc. Taking these results together, arthritis scores were determined. As a result, it was shown that the clinical score in the group administered with mPD-L1-mFc was reduced to a statistically significant level compared to other groups. In addition, it was observed that the mPD-L1-mFc fusion protein showed an effect similar to the anti-TNF-alpha antibody commercially available (Fig. 40).

**Example 7: Observation of Effect of mPD-L1-mFc Protein on Immune Tolerance**

To examine whether the mPD-L1-mFc protein can inhibit transplantation rejection responses, the following experiment was conducted using islet-allograft mouse model.

First, healthy islets from other mouse species (C57/BL6) were transplanted to the mice (BALB/c(H-2d)) with streptozotocin (STZ)-induced pancreatic destruction. Mice of the mouse model were divided into a group without drug administration and a group administered with 100 μg of mPD-L1-mFc (obtained in Example 2). Then, mPD-L1-mFc was administered to each mouse on day 0 (the day islets were transplanted) and days 7 and 14 after transplantation. The blood glucose level and body weight of each mouse were measured during a period ranging from 2 weeks prior to the islet transplantation to the end of the experiment, and the results were shown by a graphs. Herein, the reference blood glucose level was shown as a dotted line, and the reference body weight was shown as a gray region.

As a result, in the group not administered with mPD-L1-mFc, it was observed that the blood glucose level persistently increased above the reference blood glucose level and that the body weight also decreased (Fig. 16(a)). However, in the group administered with mPD-L1-mFc (Fig. 16(b)), for 17 days after the first administration of mPD-L1-mFc, the blood glucose level was maintained not more than 200 mg/dl, which is the reference blood glucose level for transplantation rejection responses, and the body weight also increased gradually. Namely, it was found that the transplanted islets from other species could perform the function of blood glucose control without immune rejection reactions, owing to the immune tolerance to the transplantation induced by mPD-L1-mFc.

## II. Examination of *in vitro* Activity of Fusion Protein Comprising Human PD-L1 or Fragment Thereof and Immunoglobulin Fc Region

### Example 8: Preparation of hPD-L1-hyFc (human PD-L1-hyFc) Gene Construct

To obtain a human PD-L1-Fc fusion protein, a modified Fc region, which causes no ADCC and CDC and can increase *in vivo* half-life, was fused to the human PD-L1 gene, to produce a recombinant protein.

Specifically, to prepare an expression vector comprising human PD-L1 gene, a known amino acid sequence of human PD-L1 gene (Accession number: Q9NZQ7) was used. In addition, a construct comprising the extracellular domain alone was prepared. The PD-L1 gene was fused to the modified Fc domain, to obtain a recombinant protein expression vector.

The modified Fc domain is described as hyFc (hybrid Fc) in US Patent No. 7,867,491. The hyFc protein is a hybrid of human IgD Fc and human IgG4 Fc, and when it is coupled to a physiologically active protein, it can significantly increase the *in vivo* half-life as compared to a pre-existing modified immunoglobulin Fc region. Among various types of hyFc proteins, an amino acid sequence of SEQ ID NO: 6 (hyFc) or an amino acid sequence of SEQ ID NO: 7 (hyFcM1) was used in this experiment to prepare a recombinant protein expression vector.

A variety of hPD-L1-hyFc fusion proteins and hPD-L1-hyFcM1 fusion proteins have the amino acid sequences represented by SEQ ID NOS: 12 to 23. Furthermore, nucleic acid sequences encoding the hPD-L1-hyFc fusion protein and hPD-L1-hyFcM1 fusion protein are represented by SEQ ID NOS: 26 to 37.

In addition, hPD-L1-linker-hyFc fusion proteins were prepared. In this Example, GS6 or GS 11 was used as a linker. GS6 refers to a peptide consisting of the amino acid sequence of GSGGGS, and GS 11 refers to a peptide consisting of the amino acid sequence of GSGGGGSGGGS.

First, to determine the start codon of the N-terminal sequence, evaluation of a recombinant protein (*see* SEQ ID NOS: 13 and 19 for the amino acid sequence; and *see* SEQ ID NOS: 27 and 33 for the nucleic acid sequences encoding the recombinant protein) obtained by fusing a fragment (21-239) having a deletion of two amino acids in the N-terminal region of the PD-L1 V domain was conducted. As a result, it was found that the recombinant protein having deletion of two amino acids in the N-terminal region has relatively low productivity (Figs. 17 and 18).

In addition, recombinant expression vectors were constructed to comprise the genes in which various fragments of the PD-L1 extracellular domain (19-239, 19-133, 19-127, 19-120, and 19-114) (*see* SEQ ID NOS: 12, 14 to 18 and 20 to 23 for the amino acid sequences; and *see* SEQ ID NOS: 26, 28 to 32 and 34 to 37 for the nucleic acid sequences encoding the fragments) were fused to the N-terminus of hyFc (Fig. 19). Each of the constructed expression vectors was transfected into CAP-T cell line (CAP-T™ production system) or CHO cell line. Next, the cells were cultured for 7 days, and the productivity of each cell line was examined using the collected culture medium.

### Example 9: Isolation of hPD-L1-hyFc (Human PD-L1-hyFc) Proteins

The expression levels of various PD-L1-hyFc proteins produced using the CAP-T and CHO production systems in Example 8 above were examined by ELISA assay (Fig. 20). To isolate and purify various hPD-L1-hyFc proteins from the culture medium, the protein purification process was monitored by lapse of time using protein A resin at a UV wavelength of 280 nm. As shown in Fig. 22, elution of the target protein was verified by analyzing the peak that appeared when elution buffer (0.1M glycine, pH 3.0) passed through the purification column in SE-HPLC analysis. In addition, the size of the protein was analyzed during SDS-PAGE (Fig. 21).

As a result, hPD-L1 (VC,19-239)-hyFcM1, hPD-L1 (V,19-133)-hyFcM1, hPD-L1 (V,19-130)-hyFcM1, hPD-L1 (V,19-127)-hyFcM1, hPD-L1 (V,19-127)-GS6-hyFcM1 and hPD-L1 (V,19-127)-GS11-hyFcM1 fusion proteins could be obtained from the CAP-T cell line in high yields, and a hPD-L1 (V,19-130)-hyFcM1 fusion protein could be obtained from the CHO cell line in a high yield.

### Example 10: Examination of *in vitro* Activities of hPD-L1-hyFc Fusion Proteins

#### Example 10-1: Comparison of T Cell Proliferation Inhibition and Inflammatory Cytokine Expression

The activities of various forms of hPD-L1-hyFc fusion protein including the V like domain of PD-L1 and the V and C like domains of PD-L1 were examined.

2 $\mu$g/ml of anti-CD3 antibody and each fusion protein (hPD-L1-hyFc) were mixed at a ratio of 1:1 or 1:4 to prepare mixture solutions, which were treated to 5 x $10^5$ cells/well of mouse splenocytes.

72 hr after stimulation of the splenocytes with anti-CD3 antibody, the expression level of the cell proliferation factor Ki-67 in the mouse splenocytes was analyzed by FACS to determine the effect of hPD-L1-hyFc on the cell proliferation.

As a result, it was shown that the PD-L1 extracellular domain (hPD-L1 (V,19-239)-hyFcM1) and a fragment thereof (hPD-L1 (V,19-133)-hyFcM1) inhibited the $CD4^+$ T cell and $CD8^+$ T cell proliferations in the mouse splenocytes (reduced Ki-67 expression; Figs. 23 and 24). Particularly, the concentration-dependent inhibition effect of the protein on $CD8^+$ T cell proliferation was more significant than on $CD4^+$ T cell proliferation.

**Example 10-2: Measurement of Binding Affinity**

It is known that PD-L1 binds to PD-1 to inhibit T cell proliferation or inflammatory cytokine secretion. Thus, the binding affinity between PD-1 and various PD-L1-hyFc fusion proteins as shown in Fig. 16 were measured, to predict the pharmacological activities of the fusion proteins.

It is known that the binding sequences of mouse PD-1 (mPD-1) and human PD-1 (hPD-1) are well conserved, and thus human PD-L1 may bind to mouse PD-1. mPD-1 was coated on the plate surface, and then treated with various concentrations of the hPD-L1-hyFc fusion proteins, after which the binding affinity between mPD-1 and the fusion proteins were examined by ELISA assay.

As a result, it was shown that not only the fusion protein comprising the V domain of PD-L1 alone (hPD-L1 (V,19-133)-hyFcM1), but also the fusion protein comprising the V and C domains of PD-L1 (hPD-L1 (VC,19-239)-hyFcM1), had an excellent ability of binding to PD-1 (Fig. 25(a)).

In addition, the binding affinity was evaluated by SPR assay. First, Protein GLC sensor chips (Bio-Rad, Cat #. 176-5011) coated with hPD-1 were prepared. After completion of the coating, the chips were respectively treated with hPD-L1 (VC,19-239), hPD-L1 (VC,19-239)-hyFcM1 and hPD-L1 (V,19-133)-hyFcM1 at concentrations of 1000, 500, 250, 100 and 50 nM. Each of the fusion proteins was allowed to flow on the hPD-L1-coated chips at a rate of 40 or 50 μl/min for 240 sec or 300 sec. Next, the baseline value was determined using regeneration buffer (10 mM NaOH), and the above step was repeated. Next, the binding curves were obtained using a protein binding analysis device (Proteon XPR36, BIO-RAD, USA).

As a result, it was shown that hPD-L1 (VC,19-239), hPD-L1 (VC,19-239)-hyFcM1 and hPD-L1 (V,19-133)-hyFcM1 all bound to hPD-1 in a concentration-dependent manner (Fig. 25(b)). In addition, based on the association constant (Ka) values and the dissociation constant (Kd) values, it was shown that hPD-L1 (VC,19-239)-hyFcM1 and hPD-L1 (V,19-133)-hyFcM1 had similar binding affinity and that the binding affinity of the PD-L1-Fc fusion protein did not greatly differ from that of the single protein to which no Fc was coupled.

**Example 11: Measurement of *in vivo* Half-Life of PD-L1 Fusion Protein (*see* KR10-2008-0094781A)**

To examine the pharmacokinetics of the fusion protein according to the present invention, the *in vivo* half-life of the fusion protein was measured with reference to the disclosure of KR10-2008-0094781A.

**Example 11-1: Preparation of Fusion Protein**

A PD-L1 fusion protein was prepared by fusing the extracellular domain of PD-L1 or a fragment thereof with immunoglobulin Fc. As the fusion partner Fc, each of wild-type IgG Fc of human origin and the hyFc and hyFcM1 prepared in Example 9 was used.

Herein, as the extracellular domain of PD-L1, a full-length peptide (VC, 19-239), a hPD-L1 (VC, 21-239) fragment comprising the amino acids at positions 21 to 239 of SEQ ID NO: 3, a hPD-L1 (V, 19-133) fragment comprising the amino acids at positions 19 to 133 of SEQ ID NO: 3, a hPD-L1 (V, 19-130) fragment comprising the amino acids at positions 19 to 130 of SEQ ID NO: 3, a hPD-L1 (VC, 19-127) fragment comprising the amino acids at positions 19-127 of SEQ ID NO: 3, a hPD-L1 (V, 19-120) fragment comprising the amino acids at positions 19 to 120 of SEQ ID NO: 3, and a hPD-L1 (V, 19-114) fragments comprising the amino acids at positions 19 to 114 of SEQ ID NO: 3 were used. The preparation was carried out by the same method as described in Examples 5 and 6.

As a result, hPD-L1 (VC,19-239), hPD-L1 (VC,21-239), hPD-L1 (V,19-133), hPD-L1 (V,19-130), hPD-L1 (V,19-127), hPD-L1 (V,19-120), hPD-L1 (V,19-114), hPD-L1 (VC,19-239)-hFc(IgG1), hPD-L1 (VC,21-239)-hFc, hPD-L1 (V,19-133)-hFc, hPD-L1 (V,19-130)-hFc, hPD-L1 (V,19-127)-hFc, hPD-L1 (V,19-120)-hFc, hPD-L1 (V,19-114)-hFc, hPD-L1 (VC,19-239)-hyFc, hPD-L1 (VC,21-239)-hyFc, hPD-L1 (V,19-133)-hyFc, hPD-L1 (V,19-130)-hyFcM, hPD-L1 (V,19-127)-hyFc, hPD-L1 (V,19-120)-hyFc, hPD-L1 (V,19-114)-hyFc, hPD-L1 (VC,19-239)-hyFcM1, hPD-L1 (VC,21-239)-hyFcM1, hPD-L1 (V,19-133)-hyFcM1, hPD-L1 (V,19-130)-hyFcM1, hPD-L1 (V,19-127)-hyFcM1, hPD-L1 (V,19-127)-GS6-hyFcM1, hPD-L1 (V,19-127)-GS11-hyFcM1, hPD-L1 (V,19-120)-hyFcM1, and hPD-L1 (V,19-114)-hyFcM1 were obtained. In this Example, GS6 refers to a peptide consisting of the amino acid sequence of GSGGGS, and GS 11 refers to a peptide consisting of the amino acid sequence of GSGGGGSGGGS.

**Example 11-2: Pharmacokinetic Study of Fusion Protein**

To compare the half-life of the extracellular domain fragment of PD-L1 with that of the fusion protein comprising the extracellular domain, 2 mg/kg of Fc-free PD-L1 protein (Sino Biological Inc., Cat#10084-H08H) as a control was administered to animal groups intravenously, each group consisting of two male Sprague Dawley rats (Charles River Laboratories, Wilmington). Before injection and at 5 min, 30 min, 2 hr, 8 hr, 24 hr, 48 hr, 72 hr, 96 hr, 120 hr, 144 hr, 168 hr, 216 hr, 264 hr and 336 hr after injection, blood was sampled from the animals. The blood samples were incubated at room temperature for 30 min to be coagulated. After centrifugation at 3000 rpm for 10 min, serum was collected from each sample and stored in a deep freezer. Each sample was quantified at several dilution ratios using a test method capable of detecting PD-L1 specifically.

hPD-L1 (VC,19-239), hPD-L1 (VC,21-239), hPD-L1 (V,19-133), hPD-L1 (V,19-130), hPD-L1 (V,19-127), hPD-L1 (V,19-120), hPD-L1 (V,19-114), hPD-L1 (VC,19-239)-hFc(IgG1), hPD-L1 (VC,21-239)-hFc, hPD-L1 (V,19-133)-hFc, hPD-L1 (V,19-130)-hFc, hPD-L1 (V,19-127)-hFc, hPD-L1 (V,19-120)-hFc, hPD-L1 (V,19-114)-hFc, hPD-L1 (VC,19-239)-hyFc, hPD-L1 (VC,21-239)-hyFc, hPD-L1 (V,19-133)-hyFc, hPD-L1 (V,19-130)-hyFcM, hPD-L1 (V,19-127)-hyFc, hPD-L1 (V,19-120)-hyFc, hPD-L1 (V,19-114)-hyFc, hPD-L1 (VC,19-239)-hyFcM1, hPD-L1 (VC,21-239)-hyFcM1, hPD-L1 (V,19-133)-hyFcM1, hPD-L1 (V,19-130)-hyFcM1, hPD-L1 (V,19-127)-hyFcM1, hPD-L1 (V,19-120)-hyFcM1, or hPD-L1 (V,19-114)-hyFcM1 was injected subcutaneously or intravenously.

As a result, it was shown that the half-life of PD-L1 to which Fc, hyFc or hyFcM1 was coupled was longer than the PD-L1 single protein. In addition, it was shown that the half-life of the fusion protein comprising the PD-L1 extracellular domain or a fragment thereof to which hyFc or hyFcM1 was coupled, was significantly longer than that of PD-L1 to which Fc (IgG1) was coupled (Fig. 26).

**Example 12: Comparison of *in vitro* Activity of PD-L1 or Fragment Thereof, or Fusion Protein Comprising the Same - Comparison of T Cell Proliferation Inhibition and Inflammatory Cytokine Expression**

The activities of purified hPD-L1 (VC, 19-239) (SEQ ID NO: 41), hPD-L1 (V, 19-133) (SEQ ID NO: 40), hPD-L1 (V, 19-127) (SEQ ID NO: 39), hPD-L1 (VC, 19-239)-hyFc (SEQ ID NO: 12), hPD-L1 (V, 19-133)-hyFc (SEQ ID NO: 14), hPD-L1 (V, 19-127)-hyFc (SEQ ID NO: 15), hPD-L1 (VC, 19-239)-hyFcM1 (SEQ ID NO: 18), hPD-L1 (VC, 21-239)-hyFcM1 (SEQ ID NO: 19), hPD-L1 (V, 19-133)-hyFcM1 (SEQ ID NO: 20), and hPD-L1 (V, 19-127)-hyFcM1 (SEQ ID NO: 21) were examined. Using the splenocytes of C57BL/6 mice, the T cell proliferation inhibition effects of human PD-L1 were compared *in vitro.*

Anti-CD3 and each hPD-L1-hyFc fusion protein were coated on microbeads at a ratio of 1:1 or 1:4. In addition, the beads and the mouse splenocytes were used at a ratio of 10:1 to stimulate the splenocytes ($5 \times 10^6$ beads: $5 \times 10^5$ splenocytes).

The mouse splenocytes were stimulated in a microwell plate. After 48 hours of stimulation, the expression level of the cell proliferation factor Ki-67 in the mouse splenocytes was analyzed by FACS, to measure the effect of hPD-L1-hyFc on cell proliferation.

As a result, it was shown that the PD-L1 extracellular domain and a fragment thereof, the PD-L1 extracellular domain to which Fc was coupled and a fragment thereof, and the PD-L1 extracellular domain to which hyFc or hyFcM1 was coupled and a fragment thereof inhibited the proliferation of $CD4^+/CD8^+$ T cells in mouse splenocytes (reduced expression of Ki-67) (Fig. 5). About 30.1% of the splenocytes stimulated with the anti-mouse CD3 antibody expressed Ki-67, but in the experimental group treated with hPD-L1 (VC, 19-239)-hyFcM1 (SEQ ID NO: 18), T cell proliferation was inhibited, and led to decrease in the ratio of Ki-67 expressing cells. Particularly, when treated at a ratio of 1:4, the proliferation of the splenocytes was reduced, and thus the ratio of Ki-67 expressing cells decreased to 6.9%.

**Example 13: *In vitro* Experiment on IL-2 Production Inhibition Activity of PD-L1 or Fragment Thereof, or Fusion Protein Comprising the Same**

To examine the ability of the hPD-L1 (VC, 19-239)-hyFcM1 fusion protein to inhibit human T cells, PBMCs obtained from RA (Rheumatoid Arthritis) patients were treated with hPD-L1 (VC, 19-239)-hyFcM1, and then the expression level of IL-2 in the cells was analyzed. IL-2 is a typical cytokine that is expressed in activated T cells. For activation of PBMCs, the cells were treated with 5 μg/ml of PHA (phytohemagglutinin), and at the same time, treated with hPD-L1 (VC, 19-239)-hyFcM1 at concentrations of 0, 10 and 50 μg/ml. At 48 hr after treatment, the expression level of IL-2 in the cells was analyzed by ELISA.

As a result, it was shown that the production of IL-2 in the group administered with 50 μg/ml of hPD-L1 (VC, 19-239)-hyFcM1 was significantly inhibited as compared to the group not administered with hPD-L1 (VC, 19-239)-hyFcM1 (Fig. 27).

In addition, in order to examine human T cell proliferation inhibition, PBMCs obtained from RA patients were respectively administered with 2 μg of hPD-L1 (VC, 19-239)-hyFcM1 and CTLA-4-Ig (Orencia) (control), and after 72 hr, proliferation of the T cells was evaluated by CFSE staining.

As a result, it was shown that T cell proliferation in the group administered with hPD-L1 (VC, 19-239)-hyFcM1 was highly significantly inhibited as compared to the group not administered with hPD-L1 (VC, 19-239)-hyFcM1 (Fig. 28).

The above results show that the hPD-L1 (VC)-hyFc protein exhibited the inhibition effects not only on human T cells but also on the cytokines associated with T cell activation, indicating that it can effectively inhibit T cells that may cause various immune diseases.

Example 14: *In **vitro*** Experiment on IL-6 Production Inhibition Activity of **PD-L1** or Fragment Thereof, or Fusion Protein Comprising the Same

Since PD-1 is expressed in human dendritic cells and monocytes as well as in T cells, the activity of the recombinant protein of the present invention was examined using these cells. Dendritic cells (DCs) were differentiated from human PBMCs by using GM-CSF, and then treated with purified hPD-L1 (VC)-hyFcM1 and CTLA-4-Ig proteins, and the activities of the proteins were examined. Specifically, it is known that IL-6 is a typical proinflammatory cytokine that is secreted from activated DCs. Thus, the expression level of IL-6 was used as a marker for evaluation.

To activate DCs induced form isolated hPBMCs, the DCs were treated with GM-CSF and 5 μg/ml of IL-4 and incubated for 6 days under the condition of LPS stimulation. After the activation procedure, the cells were respectively treated with 2, 10 and 50 μg/ml of hPD-L1 (VC, 19-239)-hyFcM1 or CTLA-4-Ig (Orencia). After 24 hr, the expression level of human IL-6 mRNA in the cells was measured by quantitative real-time PCR. As a result, it was shown that hPD-L1 (VC, 19-239)-hyFcM1 reduced the expression level of IL-6 in the DC cells in a concentration-dependent manner (Fig. 29).

In addition, the analysis result of cytokine production indicates that the production of the proinflammatory cytokine IL-6 was reduced by the PD-L1-hyFcM1 protein. Furthermore, it was shown that treatment with hPD-L1 (VC, 19-239)-hyFcM1 inhibited the expression of mRNA more effectively compared to the treatment with CTLA-4-Ig of the same concentration (Fig. 29).

**Example 15: *In vitro* Experiment on Effect of PD-L1 or Fragment Thereof, or Fusion Protein Comprising the Same in Treatment of Immune disease**

**Example 15-1: Examination of Concentration-Dependent Inhibition Effect of hPD-L1-hyFc Recombinant Protein on IL-2 Production**

Using the cells isolated from mouse spleens, the ability of the hPD-L1 (VC)-hyFcM1 and CTLA-4-Ig proteins to inhibit T cell activity was examined by measuring IL-2 and IFN-gamma. Specifically, isolated mouse splenocytes were cultured in plates coated with anti-CD3 (2 μg/ml) and anti-CD28, and were then respectively treated with 0, 10 and 50 μg/ml of hPD-L1 (VC, 19-239)-hyFcM1, followed by culture for 48 hr. Next, using the culture media, the levels of IL-2 and IFN-gamma, which are the markers of T cell activity, were measured by ELISA assay.

As a result, it was shown that treatment with hPD-L1 (VC, 19-239)-hyFcM1 highly significantly reduced the expression levels of both IL-2 and IFN-gamma in the splenocytes (Fig. 30).

**Example 15-2: Examination of Inhibition Effect of hPD-L1-hyFc Recombinant Protein on IFN-gamma**

T cells isolated from mouse pancreases were treated with the hPD-L1 (19-239)-hyFcM1, hPD-L1 (19-133)-hyFcM1 or hPD-L1 (19-127)-hyFcM1 recombinant protein, and IFN-gamma secretion from the pancreatic cells was measured, to evaluate the inhibition effects of the recombinant proteins on the total T cells and $CD4^+$ T cells. In addition, by the same method, an experiment was also conducted using hPD-L1 (19-114)-hyFc and hPD-L1 (19-120)-hyFc that comprise the V domain fragments.

Particularly, the isolated mouse splenocytes were cultured in plates coated with anti-CD3 (2 μg/ml) and anti-CD28, and then respectively treated with 2 μg of hPD-L1 (VC, 19-239)-hyFc, hPD-L1 (V, 19-133)-hyFc and hPD-L1 (V,19-127), followed by culture for 48 hr. Next, the culture media were collected, and the level of IFN-gamma, a T cell activating cytokine, in the culture media was analyzed by ELISA assay.

As a result, it was shown that all the PD-L1 fusion proteins inhibited the production of IFN-gamma (Fig. 31). The effects of the fusion proteins comprising the PD-L1 IgV domain fragments (19-127, 19-120, and 19-114) were compared, and as a result, it was shown that the cytokine inhibition effect of hPD-L1 (19-127)-hyFcM1 was more excellent than hPD-L1 (19-114)-hyFcM1 and hPD-L1 (19-120)-hyFcM1. This result indicated that the fragment consisting of the amino acids at positions 19 to 127 of SEQ ID NO: 3 plays an important role in exhibiting the immunosuppressive effect of the PD-L1 extracellular domain. In addition, it was shown that the fragments shorter than the fragment consisting of the amino acids at positions 19 to 127 of SEQ ID NO: 3 were slightly inefficient in terms of the productivity of the fusion protein (Fig. 20). Thus, it was considered that the use of a polypeptide having a length equal to or longer than the fragment consisting of the amino acids at positions 19 to 127 of SEQ ID NO: 3 is preferred in developing Fc fusion protein therapeutic agent including the PD-L1 extracellular domain.

**Example 15-3: Examination of Concentration-Dependent Inhibition Effect of hPD-L1-hyFc Recombinant Protein on IFN-gamma**

To examine the ability to inhibit the activity of T cells isolated from mouse pancreases, T cells were treated with the hPD-L1 (VC, 19-239)-hyFcM1 or hPD-L1 (V, 19-133)-hyFcM1 fusion protein. The T cell activity inhibition ability of the fusion proteins was compared by measuring the expression level of IFN-gamma in the cells. Specifically, isolated mouse splenocytes were cultured in plates coated with anti-CD3 (2 μg/ml), and then treated with 250, 500 or 1000 nM of hPD-L1 (VC, 19-239)-hyFcM1 or hPD-L1 (V, 19-133)-hyFcM1. After treatment, the cells were cultured for 48 hr, and the culture media were collected, and the level of IFN-gamma in the culture media was measured by ELISA assay.

As a result, it was shown that both hPD-L1 (VC, 19-239)-hyFcM1 and hPD-L1 (V, 19-133)-hyFcM1 inhibited the production of IFN-gamma in a concentration-dependent manner, which suggests that the fusion proteins are effective in inhibiting T cell activity (Fig. 32).

**Example 15-4: Examination of the Inhibition Effect of hPD-L1-hyFe Recombinant Protein on T Cell Proliferation**

Using T cells isolated from mouse pancreases, the cell proliferation inhibition ability of the hPD-L1 (VC, 19-239)-hyFcM1, hPD-LI (V, 19-133)-hyFcM1 and CTLA-4-Ig (Orencia) proteins was compared by MTT assay.

Specifically, isolated mouse splenocytes were cultured in plates coated with anti-CD3 (2 μg/ml), and were then treated with 250, 500 or 1000 nM of hPD-L1 (VC, 19-239)-hyFcM1, hPD-L1 (V, 19-133)-hyFcM1 or CTLA-4-Ig (Orencia). Next, the cells were cultured for 72 hr, and then MTT assay was to measure the proliferation of T cells.

As a result, it was shown that hPD-L1 (VC, 19-239)-hyFcM1, hPD-L1 (V, 19-133)-hyFcM1 and CTLA-4-Ig (Orencia) all inhibited the proliferation of T cells in a concentration-dependent manner, and that the inhibition ability of all types of the hPD-L1-hyFc fusion protein on T cell proliferation was more excellent than the control CTLA-4-Ig (Orencia) (Fig. 33) at the same concentration.

**Example 15-5: Examination of the Inhibition Effect of hPD-L1-hyFc Recombinant Protein on T Cell Activity**

Using Jurkat cells (Promega, US), a type of immortalized human T cell line, the activities of hPD-L1 (VC, 19-239)-hyFc, hPD-L1 (V, 19-133)-hyFcM1 and CTLA-4-Ig (Orencia) were measured. Jurkat cells are a cell line designed to emit a fluorescence (luciferase) signal when they express IL-2. In this Example, commercially available Jurkat cells were used.

Specifically, $2.5 \times 10^4$ cells/15 μl/well were pretreated with 100 ng/ml of PMA and activated for 24 hr. Next, proteins were respectively added to the cells to the final concentrations of 0, 1, 1.9, 3.9, 7.8, 15.5, 31, 62 and 124 μM. For re-activation, the cells were treated with 1 μg/ml of PHA, and then incubated for 4-5 hr, after which the cells were placed in a luminometer to measure the fluorescence signal intensity.

The result of comparison of IL-2 secretion indicates that hPD-L1 (VC, 19-239)-hyFc, hPD-L1 (V, 19-133)-hyFcM1 and CTLA-4-Ig (Orencia) all inhibited IL-2 secretion of T cells in a concentration-dependent manner, which demonstrates the T cell inhibition effect in a human T cell line (Fig. 34).

**III. Examination of *in vivo* Activity of Fusion Protein Comprising Human PD-L1 or Fragment Thereof and Immunoglobulin Fc Region**

**Example 16: Evaluation of Effect of hPD-L1-hyFc Recombinant Protein in IBD (Inflammatory Bowel Disease) Model**

**Example 16-1: Evaluation of Effect of hPD-L1-hyFc Recombinant Protein in Chronic Inflammatory Enteritis Model**

In chronic mouse enteritis models induced by T cell transplantation, the effect of the hPD-L1 (V, 19-133)-hyFcM1 fusion protein on the inflammatory enteritis was examined.

$CD4^+CD25^-CD45RB^{high}$ T cells were isolated from C57BL/6 mouse splenocytes by FACS. $5 \times 10^5$ isolated

$CD4^+CD25^-CD45RB^{high}$ T cells were injected to Rag-1 KO mice lacking T cells and B cells intraperitoneally.

Evaluation was conducted by the same method as described in Example 4-1. As a result, as shown in Fig. 35, the clinical score of the mice improved significantly (Fig. 35a), and the weight loss symptoms of the experimental group were alleviated in the experimental group treated with the hPD-L1 (V, 19-133)-hyFcM1 fusion protein compared to the control group (Fig. 35b). In addition, the hPD-L1 (V, 19-133)-hyFcM1 fusion protein showed an effect equal to or higher than CTLA4-Ig used as a control, even though it was used in an amount 30 times smaller than CTLA4-Ig.

**Example 16-2: Evaluation of hPD-L1-hyFc Recombinant Protein by Analysis of Survival Rate in Chronic Inflammatory Bowel Disease Model**

To evaluate the *in vivo* activities of the hPD-L1 (VC, 19-239)-hyFcM1 and hPD-L1 (V, 19-133)-hyFcM1 fusion proteins, the effect of PD-L1 protein administration on the alleviation and inhibition of enteritis was evaluated in chronic mouse enteritis model induced by T-cells.

Specifically, $CD4^+CD25^-CD45RB^{high}$ T cells were isolated from C57BL/6 mouse splenocytes by FACS. $5 \times 10^5$ isolated $CD4^+CD25^-CD45RB^{high}$ T cells were injected to Rag-1 KO mice lacking T and B cells intraperitoneally. From the day 3 weeks after T cell injection, 20 or 200 μg of the hPD-L1 (VC, 19-239)-hyFcM1 or hPD-L1 (V, 19-133)-hyFcM1 fusion protein was injected to each mouse intraperitoneally at one-week intervals for a total of three times. Throughout the experimental period, the number of the surviving mice was recorded.

As a result, in all the experimental groups excluding the control group administered with PBS after induction of enteritis and the group administered with 20 μg (lower than effective amount) of the PD-L1 fusion protein, all mice survived up to 60 days after T cell transplantation, which demonstrates the efficacy of the PD-L1 fusion proteins (Fig. 36).

**Example 17: Evaluation of the Effect of hPD-L1-hyFc Recombinant Protein in Treatment of Psoriasis**

To evaluate the effect of hPD-L1-hyFc in acute psoriasis mouse model, 40 mg of Imiquimod (IMQ) was applied to both ears of each experimental mouse for 6 days to induce acute psoriasis. Mice of the mouse model were divided into a normal mouse group, a group not treated after induction of psoriasis, and a group administered with anti-P40 antibody or the hPD-L1 (VC, 19-239)-hyFcM1 fusion protein along with induction of psoriasis, and then the therapeutic effect of the fusion protein was examined. 25 μg of anti-P40 antibody was administered to each mouse intraperitoneally on days 1 and 4. 200 μg of hPD-L1 (F)-hyFc was administered to each mouse intraperitoneally on days 1, 2, 4 and 6.

Following the first administration, the thickness of the ear was observed every day to examine the phenotype of psoriasis.

As a result, the group administered with hPD-L1 (VC, 19-239)-hyFcM1 and anti-P40 antibody showed a statistically significant delay in developing psoriasis compared to the untreated group (Fig. 37). On day 7, the ears were histopathologically examined, and the pathological tissues findings of psoriasis such as epidermal thickness in the IMQ-induced psoriasis group were comparatively observed. As a result, it was shown that the epidermal thickness in the group administered with hPD-L1 (VC, 19-239)-hyFcM1 was reduced to a statistically significant level as compared to other groups not administered with hPD-L1 (VC, 19-239)-hyFcM1 (Fig. 38).

The epidermal thickness was also reduced to a statistically significant level in the group administered with hPD-L1 (VC, 19-239)-hyFcM1 as compared to other groups not administered with hPD-L1 (VC, 19-239)-hyFcM1 (Fig. 39).

The above results indicate that the hPD-L1 fusion protein has a therapeutic effect on psoriasis and exhibits an effect comparable to anti-P40 antibody that is a conventional therapeutic agent.

## Claims

1. A fusion protein comprising an extracellular domain of Programmed Cell Death-Ligand 1 (PD-L1) protein or a fragment thereof and a modified immunoglobulin Fc region.

2. The fusion protein of claim 1, wherein the fusion protein is represented by the following formula (I) or (I'):

$$(FT)_{w1} - X1 - (L1)_{w2} - (X2)_{w3} - (L2)_{w4} - IgFc \qquad (I)$$

$$IgFc - (L2)_{w4} - (FT)_{w1} - X1 - (L1)_{w2} - (X2)_{w3} \qquad (I'),$$

wherein FT is a dipeptide consisting of phenylalanine and threonine;
w1, w2, w3 and w4 are each 0 or 1;
X1 is an Ig V like domain of the extracellular domain of PD-L1, which comprises a polypeptide having the amino acid sequence of SEQ ID NO: 48 or 50;
L1 and L2 are each a linker;
X2 is a polypeptide comprising an immunoglobulin C (Ig C) like domain of the extracellular domain of PD-L1, or a fragment thereof; and
IgFc is a modified immunoglobulin Fc region.

3. The fusion protein of claim 2, wherein the Ig V like domain of the extracellular domain of PD-L1 has the sequence of SEQ ID NO: 39 or 40.

4. The fusion protein of claim 2, wherein the polypeptide comprising the Ig C like domain of the extracellular domain of PD-L1 has the sequence of SEQ ID NO: 47 or 49.

5. The fusion protein of claim 2, wherein L1 consists of 1 to 10 amino acids.

6. The fusion protein of claim 5, wherein the amino acid is selected from the group consisting of leucine (Leu, L), isoleucine (Ile, I), alanine (Ala, A), valine (Val, V), proline (Pro, P), lysine (Lys, K), arginine (Arg, R), asparagine (Asn, N), and glutamine (Gln, Q).

7. The fusion protein of claim 5, wherein L1 has the sequence of SEQ ID NO: 9, 45 or 46.

**8.** The fusion protein of claim 2, wherein L2 is:

a polypeptide consisting of 10 to 20 amino acids, which consists of glycine (Gly, G) and serine (Ser, S) residues; or a polypeptide consisting of 1 to 10 amino acids selected from the group consisting of leucine (Leu, L), isoleucine (Ile, I), alanine (Ala, A), valine (Val,V), proline (Pro, P), lysine (Lys, K), arginine (Arg, R), asparagine (Asn, N), and glutamine (Gln, Q).

**9.** The fusion protein of claim 8, wherein L2 is the amino acid sequence of SEQ ID NO: 8, 9, 45 or 46.

**10.** The fusion protein of claim 2, wherein w2 is 0.

**11.** The fusion protein of claim 2, wherein the formula (I) or (I') is represented by the following formula (I-a) or (I'-a):

$$(FT)_{w1}\text{-X1-L1-X2-}(L2)_{w4}\text{-IgFc} \qquad \text{(I-a)},$$

or

$$\text{IgFc-}(L2)_{w4}\text{-}(FT)_{w1}\text{-X1-L1-X2} \qquad \text{(I'-a)},$$

wherein FT-X1-L1-X2 is the amino acid sequence of SEQ ID NO: 41.

**12.** The fusion protein of claim 2, wherein the modified immunoglobulin Fc region is selected from the Fc regions of IgG1, IgG2, IgG3, IgD and IgG4, and a combination thereof.

**13.** The fusion protein of claim 12, wherein:

the modified immunoglobulin Fc region comprises a hinge region, a CH2 domain and a CH3 domain, which are arranged in the direction of from the N-terminus to the C-terminus, wherein,
the hinge region comprises a human IgD hinge region;
the CH2 domain comprises an amino acid residue portion of the CH2 domain of human IgD and an amino acid residue portion of the CH2 domain of human IgG4; and
the CH3 domain comprises an amino acid residue portion of the CH3 domain of human IgG4.

**14.** The fusion protein of claim 13, wherein the modified immunoglobulin Fc region is represented by the following formula (II):

$$\text{N'-(Z1)p-Y-Z2-Z3-Z4-C'} \qquad \text{(II)},$$

wherein, N' is the N-terminus of a polypeptide, and C' is the C-terminus of a polypeptide;
p is an integer of 0 or 1; and
Z1 is an amino acid sequence having 5 to 9 consecutive amino acid residues counted from position 98 in the direction to the N-terminus, among the amino acid residues at positions 90 to 98 of SEQ ID NO: 4,
Y is an amino acid sequence having 5 to 64 consecutive amino acid residues counted from position 162 in the direction to the N-terminus, among the amino acid residues at positions 99 to 162 of SEQ ID NO: 4,
Z2 is an amino acid sequence having 4 to 37 consecutive amino acid residues counted from position 163 in the direction to the C-terminus, among the amino acid residues at positions 163 to 199 of SEQ ID NO: 4,
Z3 is an amino acid sequence having 71 to 106 consecutive amino acid residues counted from position 220 in the direction to the N-terminus, among the amino acid residues at positions 115 to 220 of SEQ ID NO: 5, and
Z4 is an amino acid sequence having 80 to 107 consecutive amino acid residues counted from position 221 in the direction to the C-terminus, among the amino acid residues at positions 221 to 327 of SEQ ID NO: 5.

**15.** The fusion protein of claim 2, wherein the modified immunoglobulin Fc region comprises a polypeptide having the amino acid sequence of SEQ ID NO: 6, 7, 42, 43, or 44.

**16.** The fusion protein of claim 1, wherein the modified immunoglobulin Fc region comprises a polypeptide having the amino acid sequence of SEQ ID NO: 2.

**17.** An isolated nucleic acid molecule encoding the fusion protein of any one of claims 1 to 16.

**18.** The isolated nucleic acid molecule of claim 17, wherein the nucleic acid molecule encodes a polypeptide having an amino acid sequence selected from the group consisting of SEQ ID NOS: 10 to 23.

**19.** The isolated nucleic acid molecule of claim 18, wherein the nucleic acid molecule comprises a polynucleotide having a nucleotide sequence selected from the group consisting of SEQ ID NOS: 24 to 37.

**20.** The isolated nucleic acid molecule of claim 19, wherein the nucleic acid molecule further comprises a signal sequence or a leader sequence.

**21.** The isolated nucleic acid molecule of claim 20, wherein the signal sequence is tPa signal sequence.

**22.** The isolated nucleic acid molecule of claim 21, wherein the tPa signal sequence comprises the nucleic acid sequence of SEQ ID NO: 38.

**23.** An expression vector comprising the isolated nucleic acid molecule of any one of claims 17 to 22.

**24.** A host cell comprising the expression vector of claim 23.

**25.** A pharmaceutical composition for preventing or treating an immune disease, which comprises the fusion protein of any one of claims 1 to 16.

**26.** The pharmaceutical composition of claim 25, further comprising a pharmaceutically acceptable carrier.

**27.** The pharmaceutical composition of claim 25, wherein the immune disease is selected from the group consisting of an autoimmune disease, an inflammatory disease, and a transplantation rejection disease of a cell, a tissue or an organ.

**28.** The pharmaceutical composition of claim 27, wherein the autoimmune disease is selected from the group consisting of arthritis, psoriasis, autoimmune diabetes, and inflammatory bowel disease.

**29.** A composition for inducing immune tolerance, which comprises the fusion protein of any one of claims 1 to 16.

**30.** A method for preventing or treating an immune disease, which comprises administering the fusion protein of any one of claims 1 to 16 and a pharmaceutically acceptable carrier to a subject.

**31.** The method of claim 30, wherein the immune disease is selected from the group consisting of an autoimmune disease, an inflammatory disease, and a transplantation rejection disease of a cell, a tissue or an organ.

**32.** The method of claim 31, wherein the autoimmune disease is selected from the group consisting of arthritis, psoriasis, autoimmune diabetes, and inflammatory bowel disease.

**33.** A method for inducing immune tolerance, which comprises administering the fusion protein of any one of claims 1 to 16 and a pharmaceutically acceptable carrier to a subject.

Fig. 1

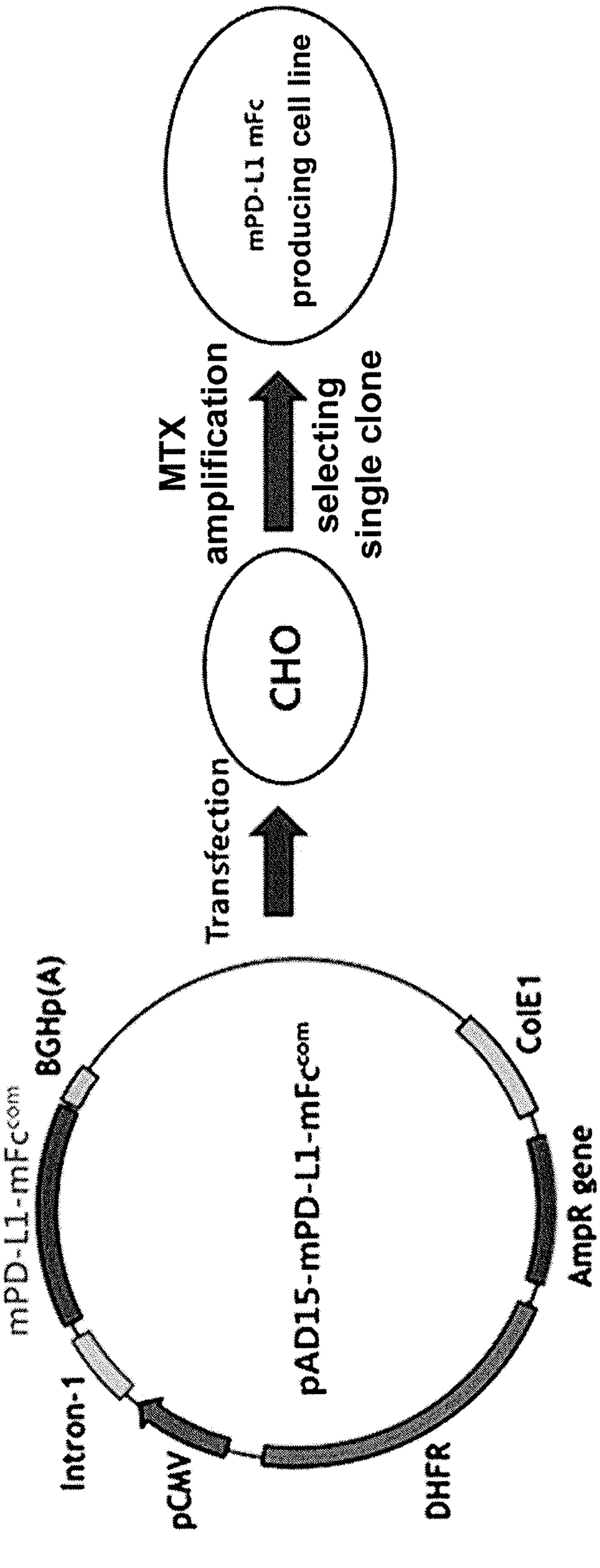
BGHp(A)
mPD-L1-mFc$^{com}$
Intron-1
pCMV
pAD15-mPD-L1-mFc$^{com}$
DHFR
AmpR gene
ColE1
Transfection
CHO
MTX
amplification
selecting
single clone
mPD-L1 mFc
producing cell line

Fig. 2

(a)

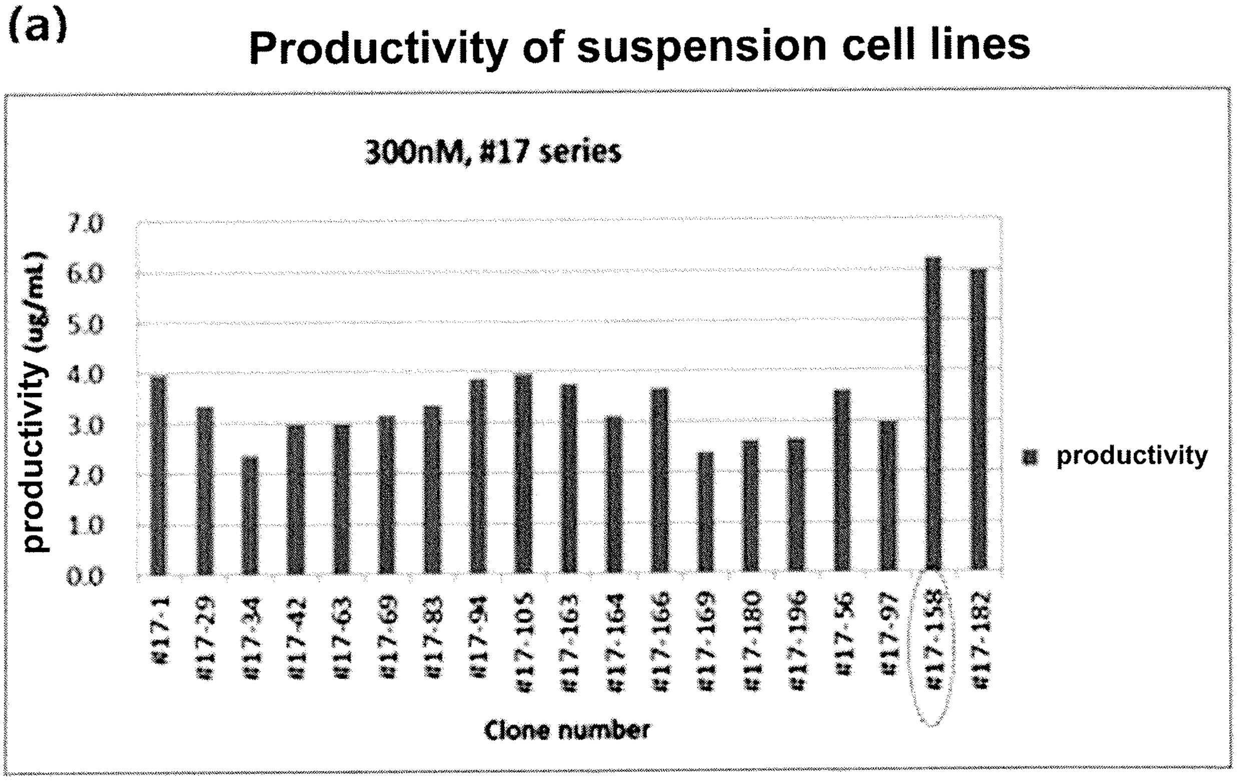
Productivity of suspension cell lines
300nM, #17 series
productivity (ug/mL)
7.0
6.0
5.0
4.0
3.0
2.0
1.0
0.0
#17-1
#17-29
#17-34
#17-42
#17-63
#17-69
#17-83
#17-94
#17-105
#17-163
#17-164
#17-166
#17-169
#17-180
#17-196
#17-56
#17-97
#17-158
#17-182
Clone number
productivity

(b)

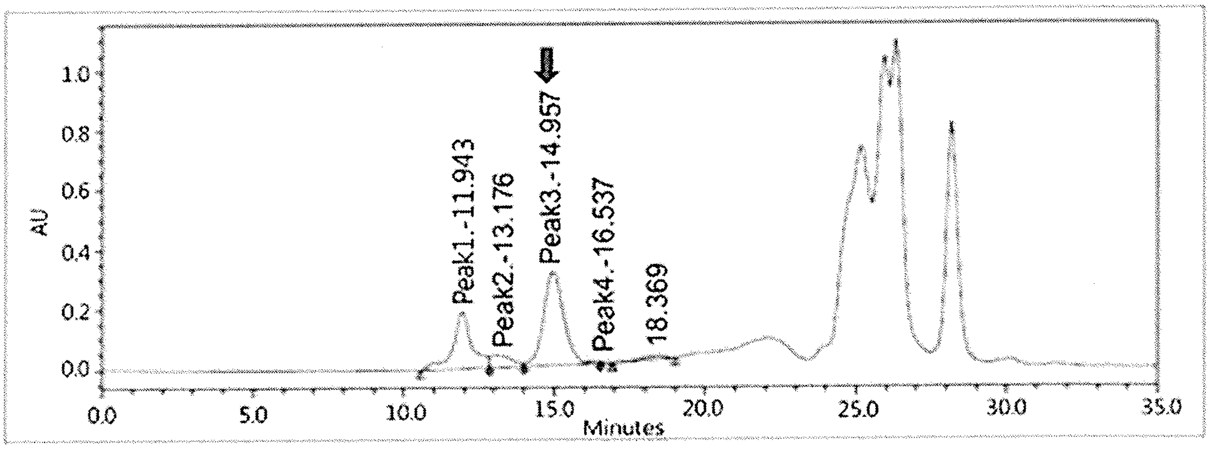
AU
1.0
0.8
0.6
0.4
0.2
0.0
Peak1.-11.943
Peak2.-13.176
Peak3.-14.957
Peak4.-16.537
18.369
0.0
5.0
10.0
15.0
20.0
25.0
30.0
35.0
Minutes

Fig. 3

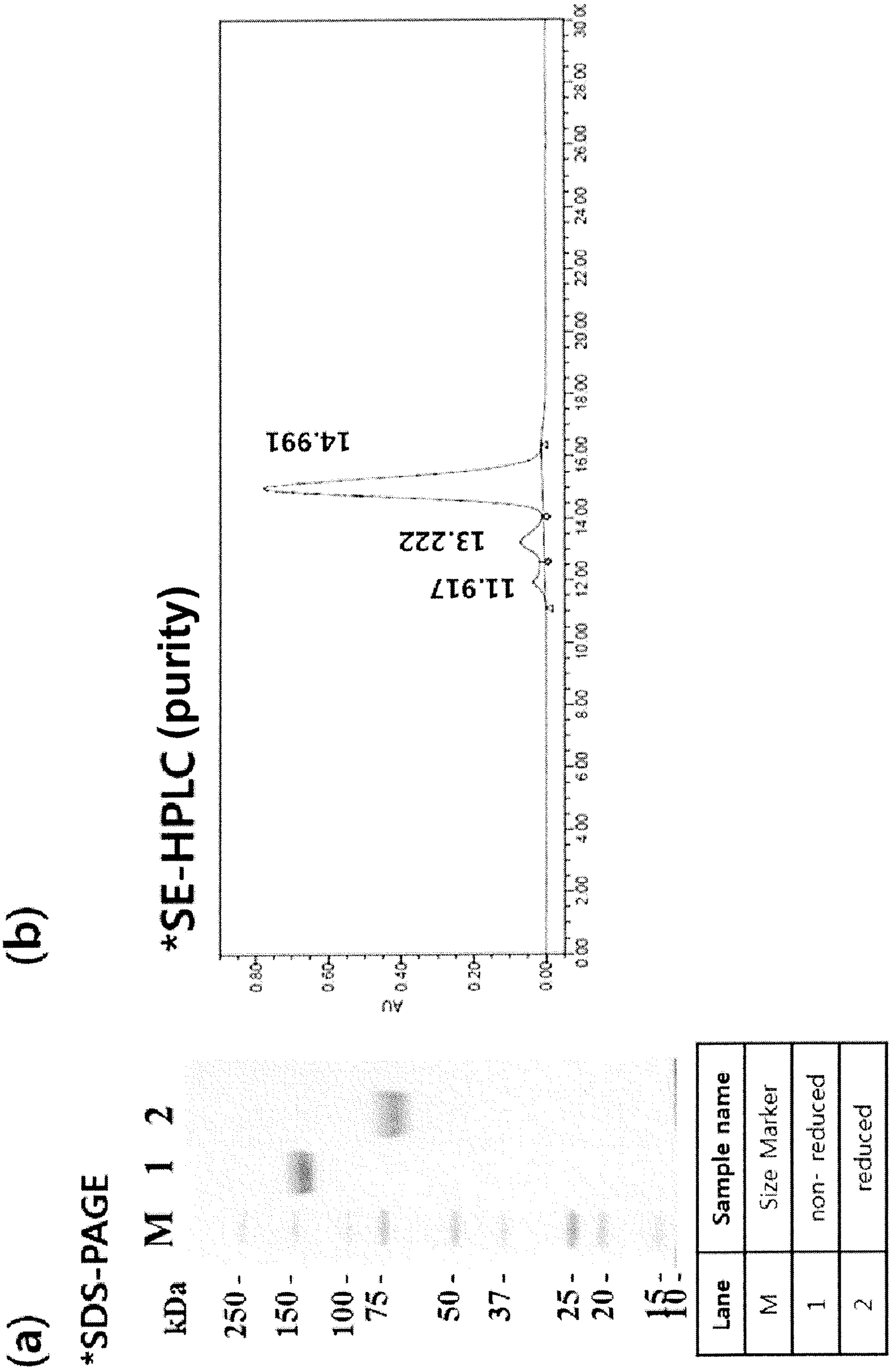
(a)
*SDS-PAGE
kDa
M 1 2
250 -
150 -
100 -
75 -
50 -
37 -
25 -
20 -
15 -
10 -
Lane | Sample name
M | Size Marker
1 | non- reduced
2 | reduced
(b)
*SE-HPLC (purity)
AU
14.991
13.222
11.917

Fig. 4

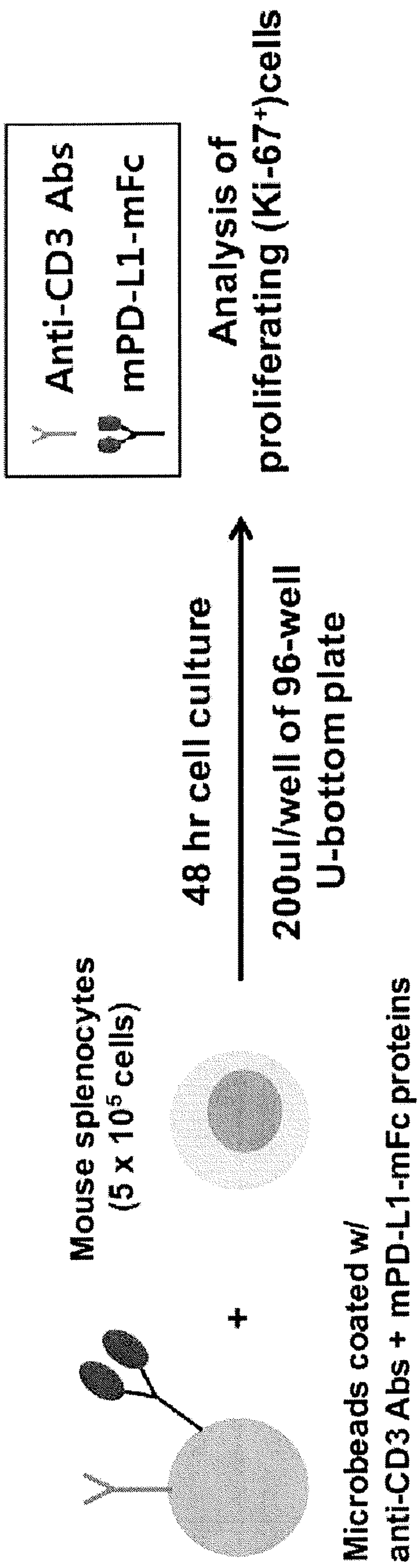
Microbeads coated w/
anti-CD3 Abs + mPD-L1-mFc proteins
+
Mouse splenocytes
(5 x 10⁵ cells)
48 hr cell culture
200ul/well of 96-well
U-bottom plate
Analysis of
proliferating (Ki-67⁺)cells
Anti-CD3 Abs
mPD-L1-mFc

Fig. 5

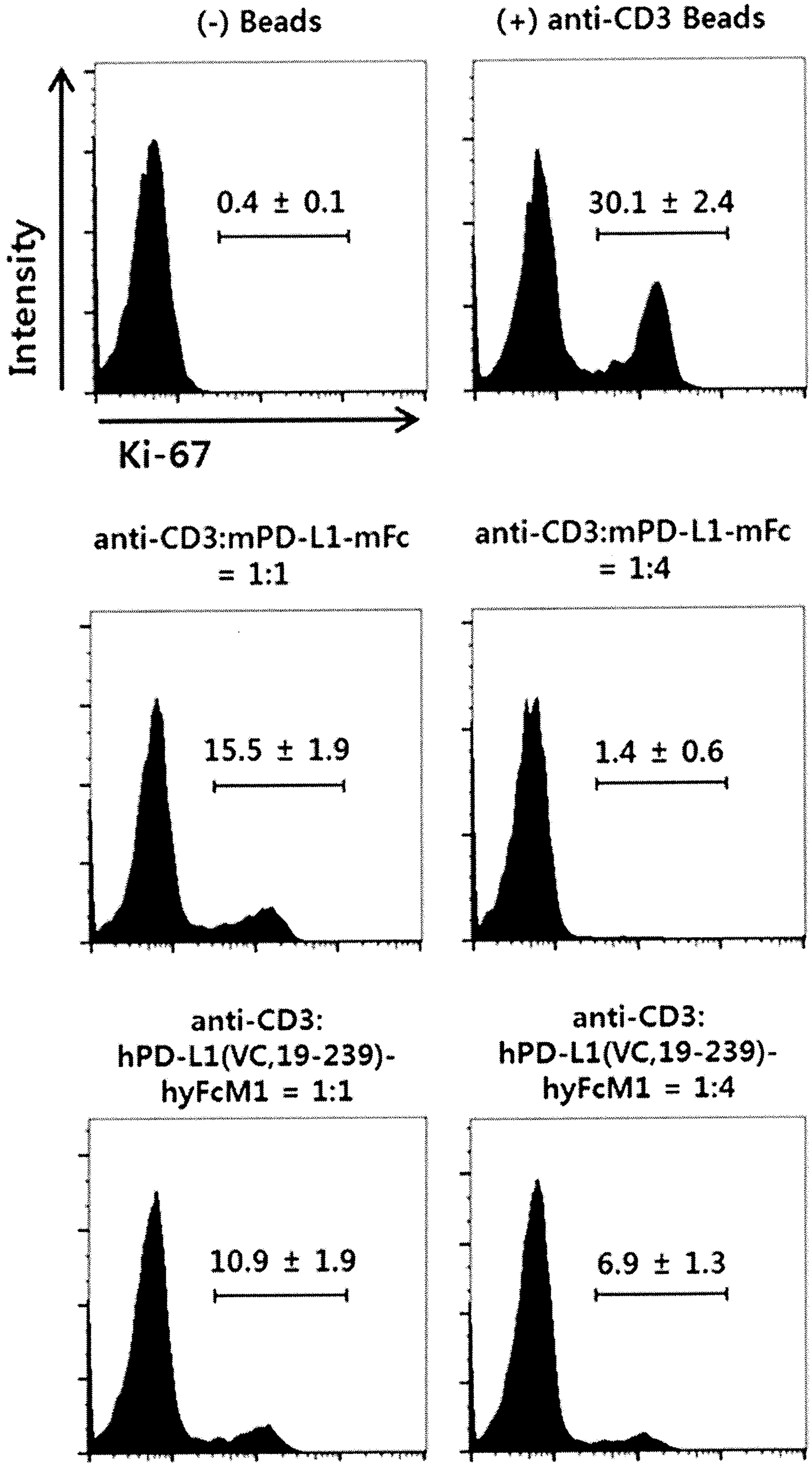
(-) Beads
(+) anti-CD3 Beads
Intensity
Ki-67
0.4 ± 0.1
30.1 ± 2.4
anti-CD3:mPD-L1-mFc = 1:1
anti-CD3:mPD-L1-mFc = 1:4
15.5 ± 1.9
1.4 ± 0.6
anti-CD3: hPD-L1(VC,19-239)-hyFcM1 = 1:1
anti-CD3: hPD-L1(VC,19-239)-hyFcM1 = 1:4
10.9 ± 1.9
6.9 ± 1.3

Fig. 6

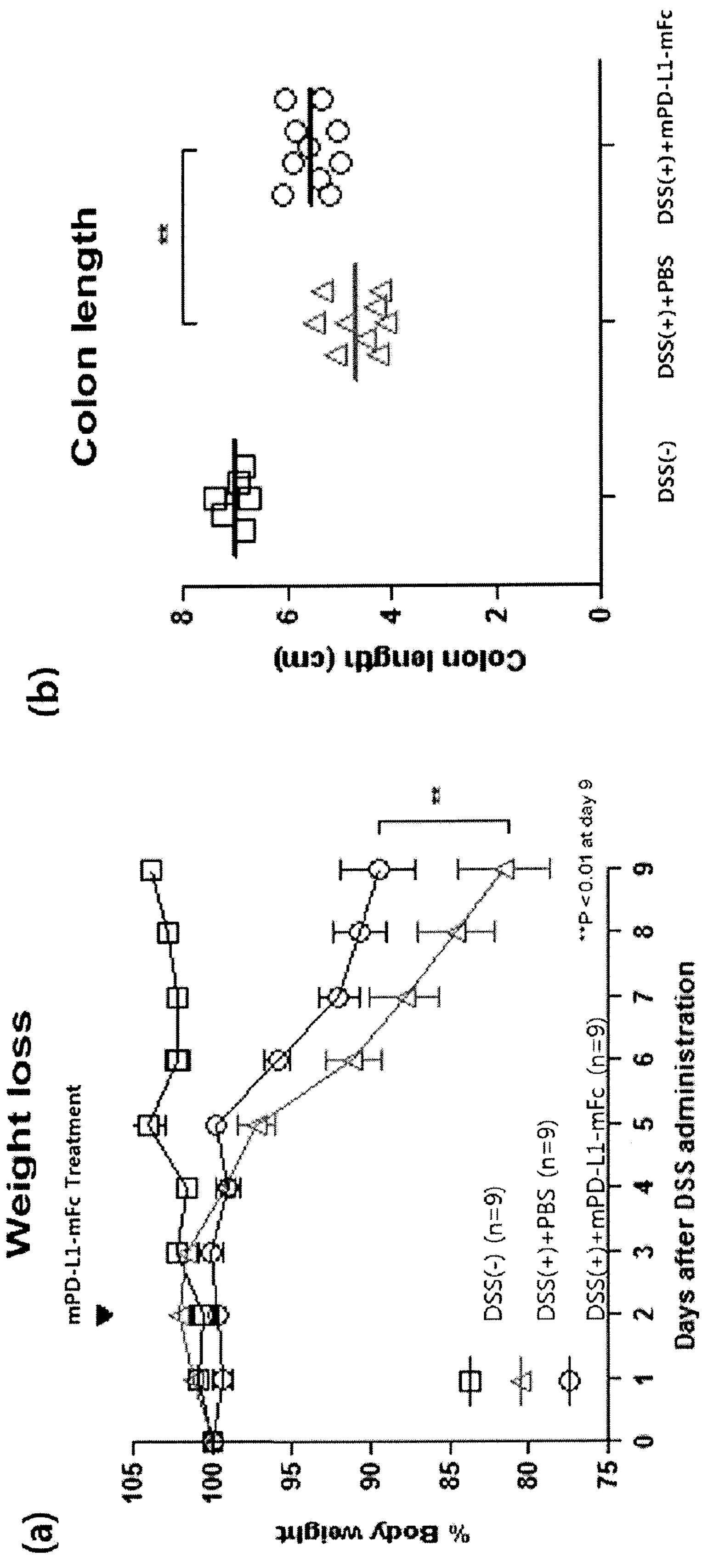
(a)
Weight loss
mPD-L1-mFc Treatment
% Body weight
105
100
95
90
85
80
75
0
1
2
3
4
5
6
7
8
9
Days after DSS administration
DSS(-) (n=9)
DSS(+)+PBS (n=9)
DSS(+)+mPD-L1-mFc (n=9)
**
**P < 0.01 at day 9
(b)
Colon length
Colon length (cm)
8
6
4
2
0
**
DSS(-)
DSS(+)+PBS
DSS(+)+mPD-L1-mFc

Fig. 7

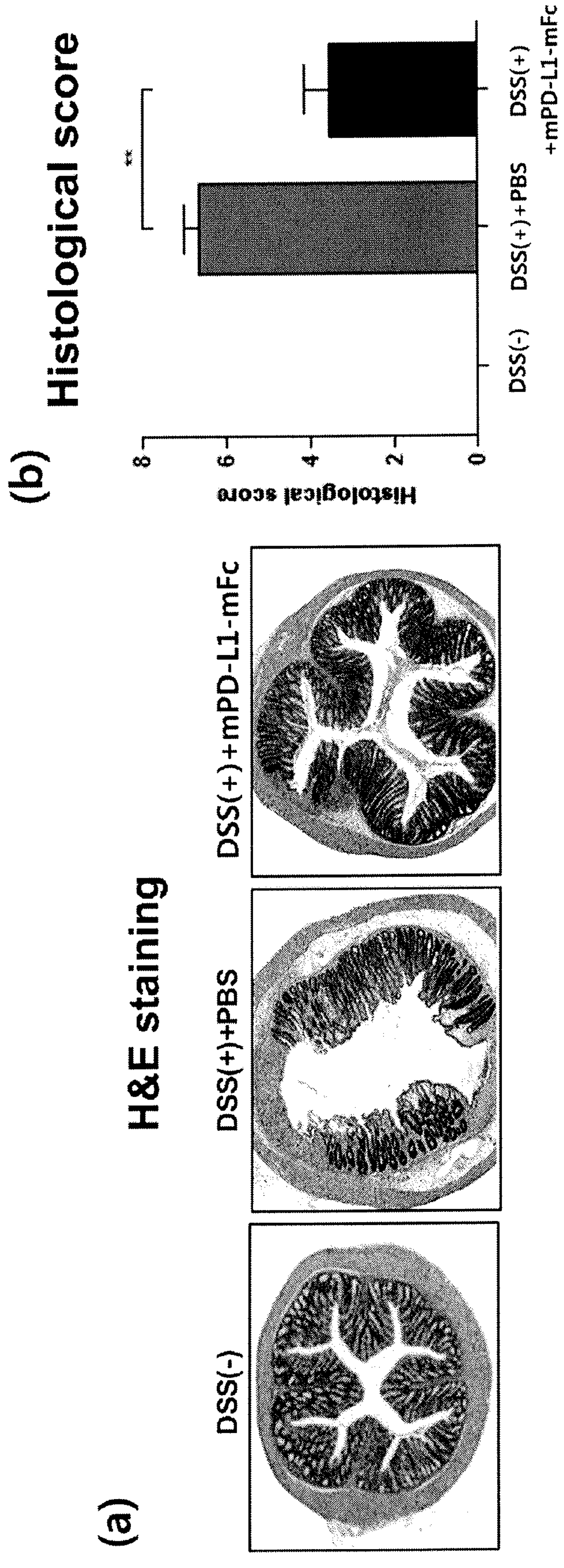
(a)
H&E staining
DSS(-)
DSS(+)+PBS
DSS(+)+mPD-L1-mFc
(b)
Histological score
**
Histological score
0
2
4
6
8
DSS(-)
DSS(+)+PBS
DSS(+)
+mPD-L1-mFc

Fig. 8

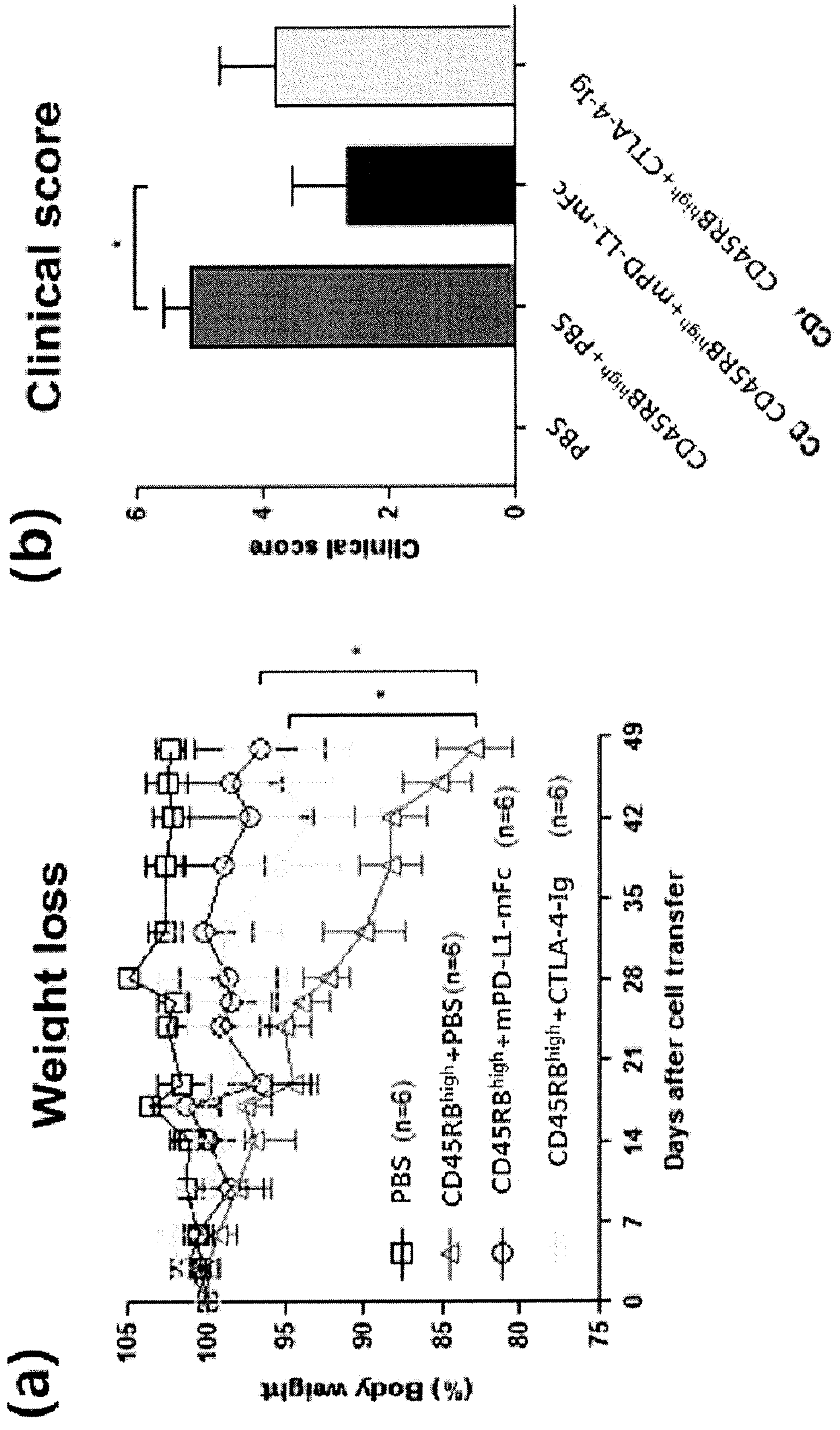
(a)
Weight loss
Body weight (%)
105
100
95
90
85
80
75
0
7
14
21
28
35
42
49
Days after cell transfer
PBS (n=6)
CD45RBhigh+PBS (n=6)
CD45RBhigh+mPD-L1-mFc (n=6)
CD45RBhigh+CTLA-4-Ig (n=6)
*
*
(b)
Clinical score
Clinical score
6
4
2
0
*
PBS
CD45RBhigh+PBS
CD45RBhigh+mPD-L1-mFc
CD45RBhigh+CTLA-4-Ig

Fig. 9

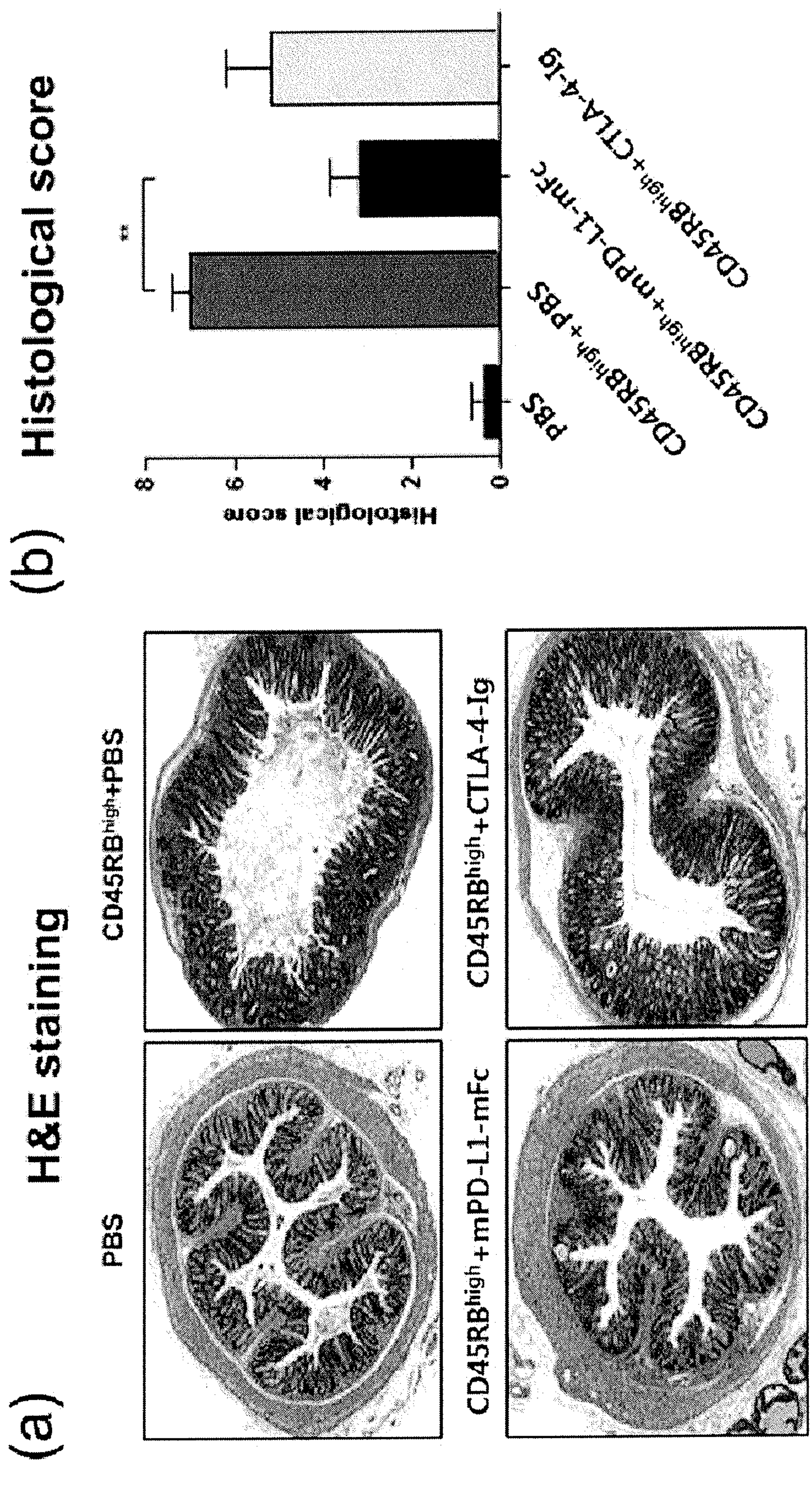
(a)
H&E staining
PBS
CD45RB$^{high}$+PBS
CD45RB$^{high}$+mPD-L1-mFc
CD45RB$^{high}$+CTLA-4-Ig
(b)
Histological score
Histological score
0
2
4
6
8
**
PBS
CD45RB$^{high}$+PBS
CD45RB$^{high}$+mPD-L1-mFc
CD45RB$^{high}$+CTLA-4-Ig

Fig. 10

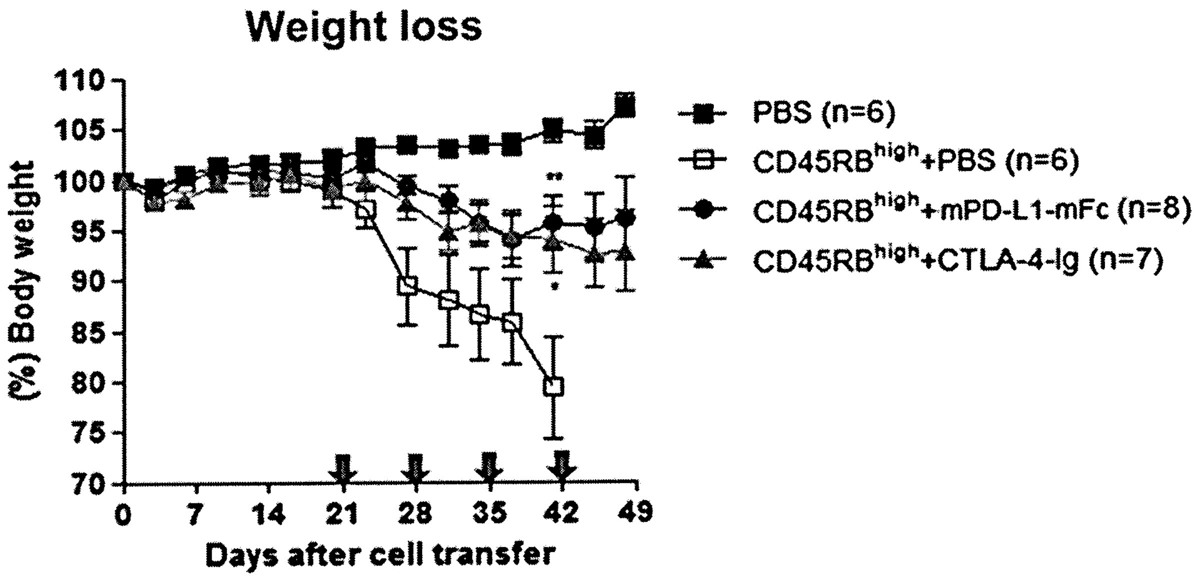


*$P$ < 0.05, **$P$ < 0.01
compared to CD45RB$^{high}$+PBS group at day 42

Fig. 11

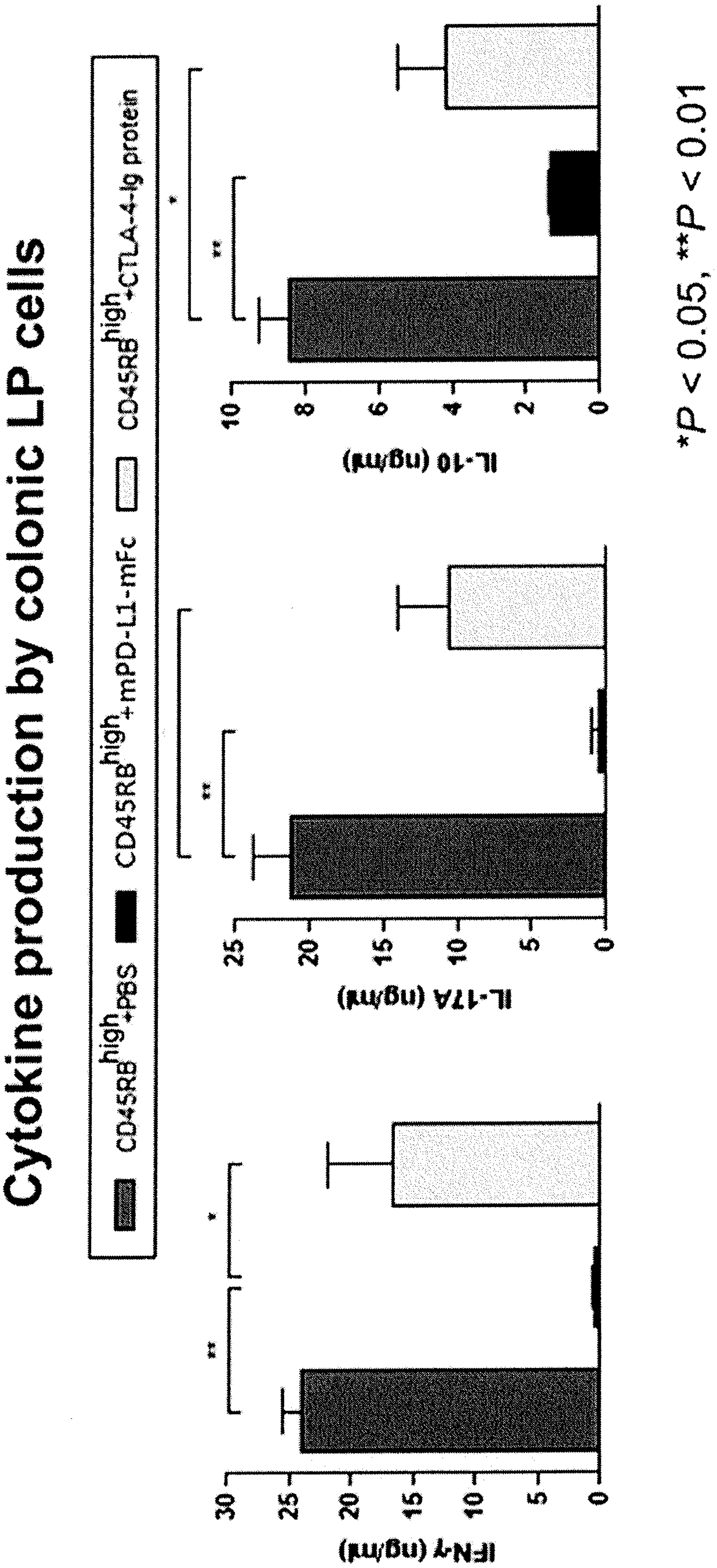
Cytokine production by colonic LP cells
CD45RBhigh+PBS
CD45RBhigh+mPD-L1-mFc
CD45RBhigh+CTLA-4-Ig protein
IFN-γ (ng/ml)
IL-17A (ng/ml)
IL-10 (ng/ml)
*P < 0.05, **P < 0.01

Fig. 12

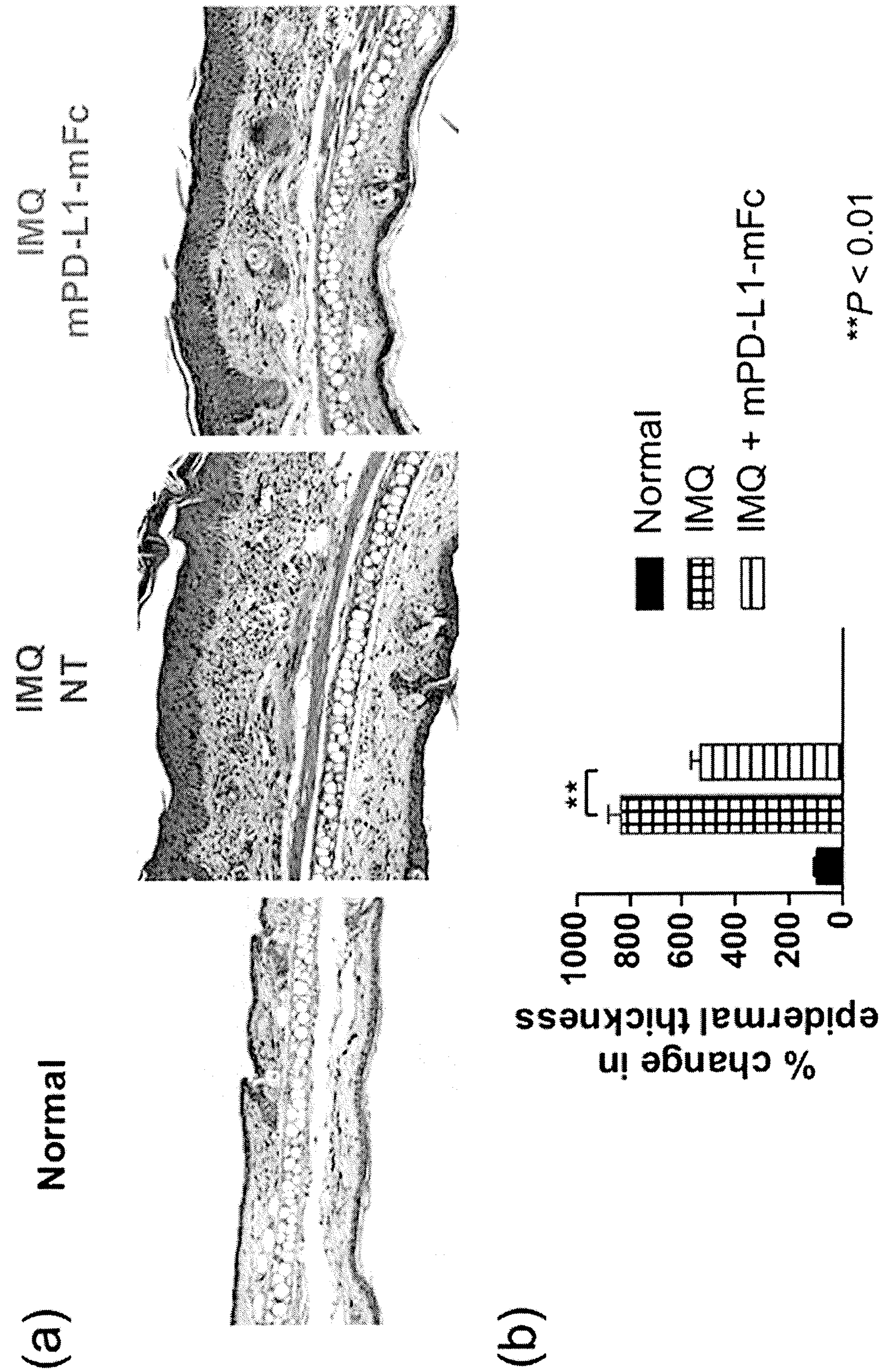
(a)
Normal
IMQ
NT
IMQ
mPD-L1-mFc
(b)
% change in
epidermal thickness
1000
800
600
400
200
0
**
Normal
IMQ
IMQ + mPD-L1-mFc
**P < 0.01

Fig. 13

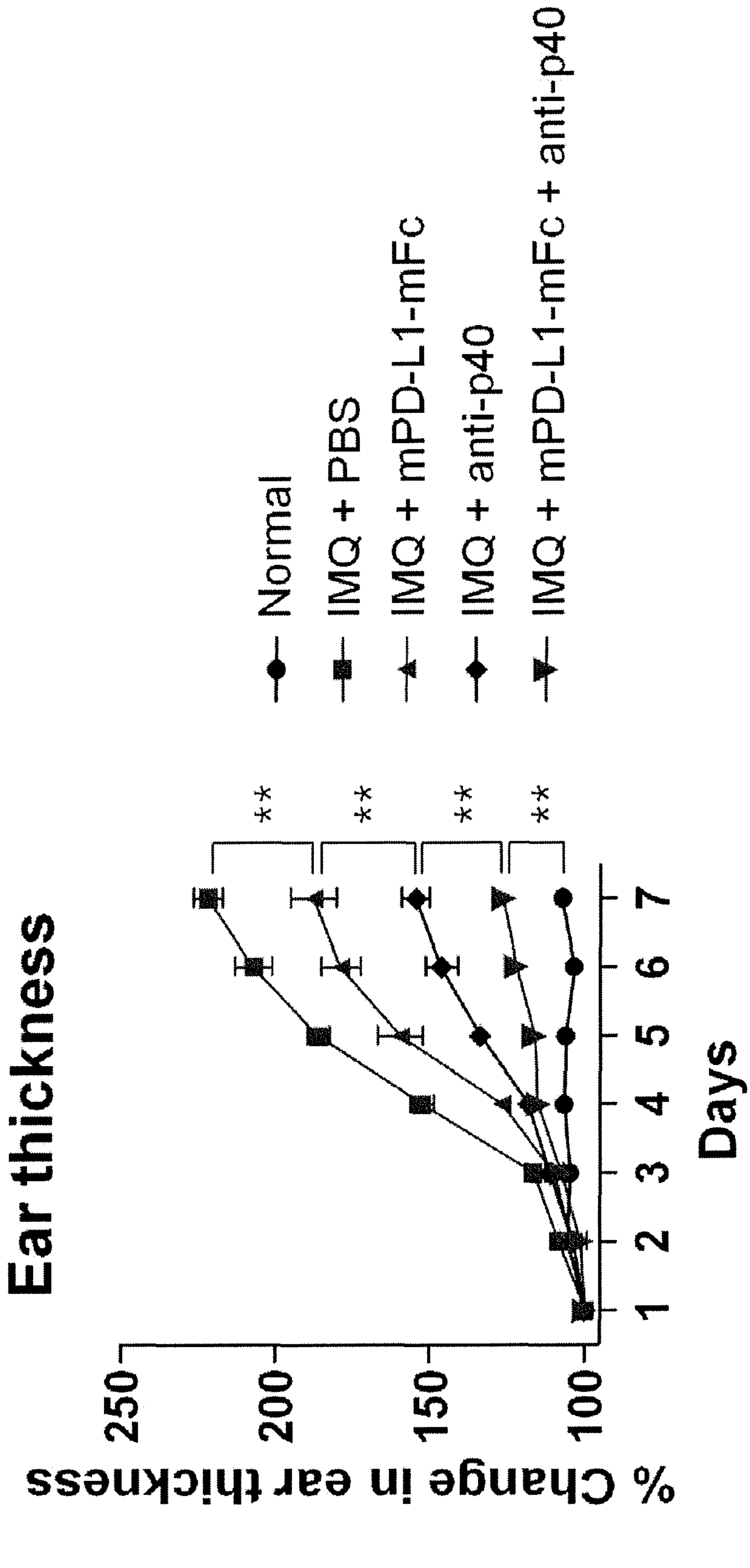
Ear thickness
% Change in ear thickness
250
200
150
100
1
2
3
4
5
6
7
Days
**
**
**
**
Normal
IMQ + PBS
IMQ + mPD-L1-mFc
IMQ + anti-p40
IMQ + mPD-L1-mFc + anti-p40

Fig. 14

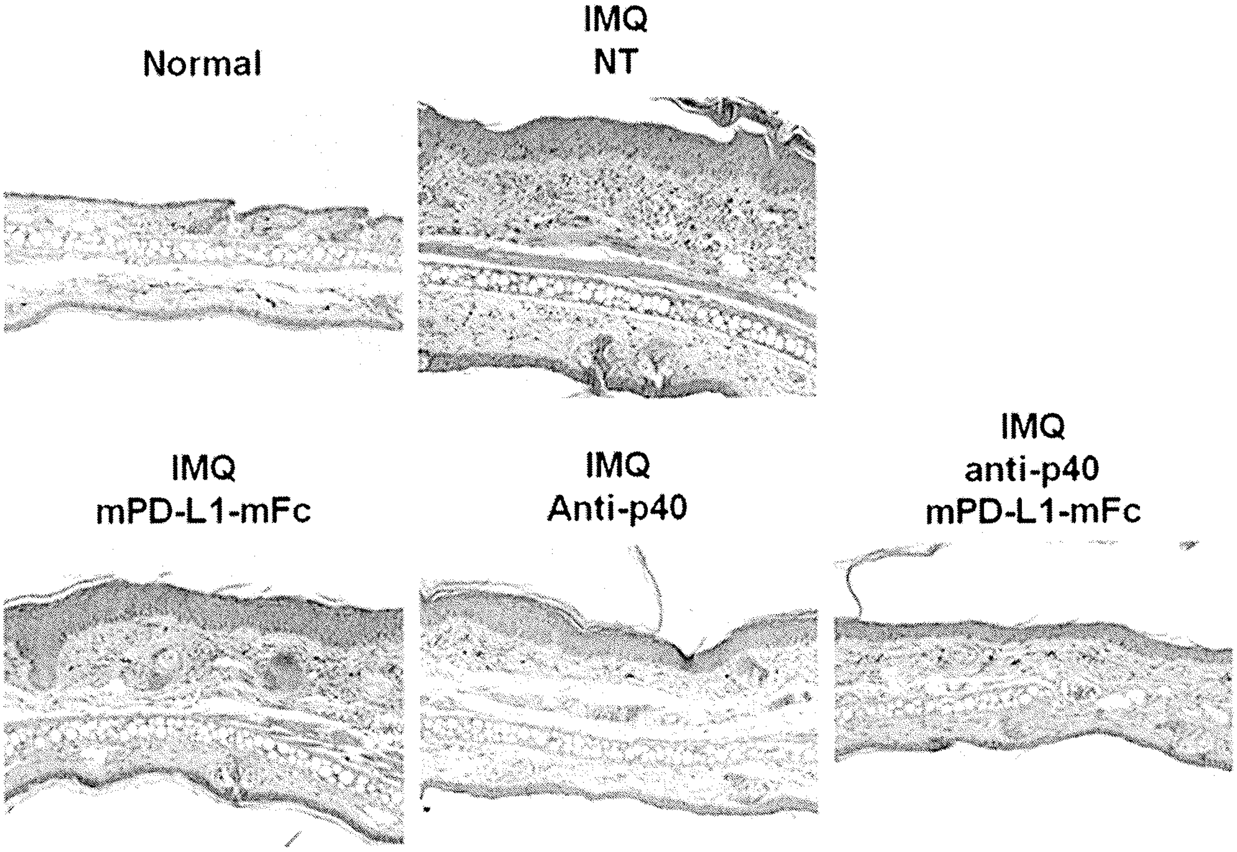
Normal
IMQ
NT
IMQ
mPD-L1-mFc
IMQ
Anti-p40
IMQ
anti-p40
mPD-L1-mFc

Fig. 15

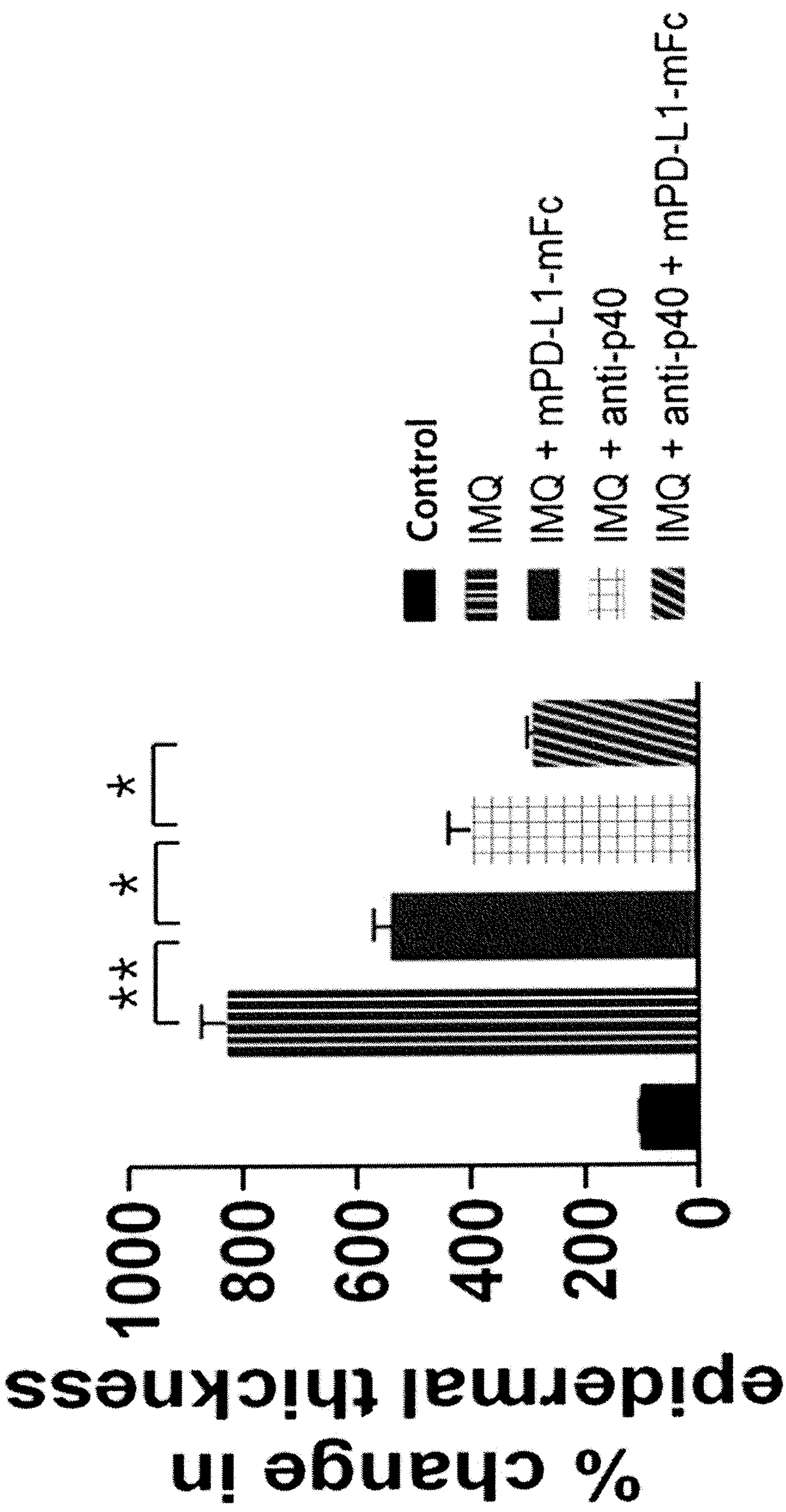
% change in epidermal thickness
1000
800
600
400
200
0
**
*
*
Control
IMQ
IMQ + mPD-L1-mFc
IMQ + anti-p40
IMQ + anti-p40 + mPD-L1-mFc

Fig. 16

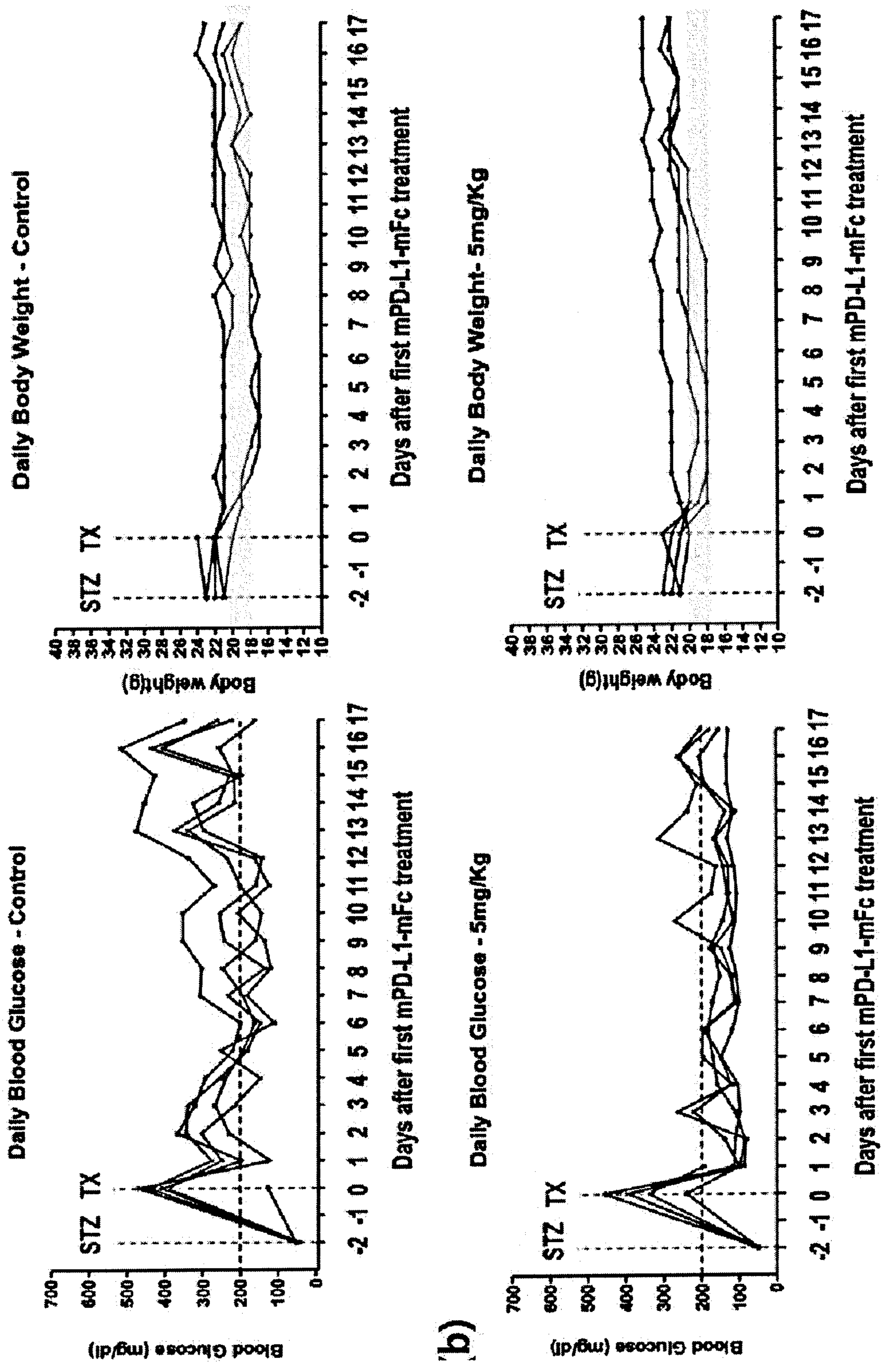
Daily Blood Glucose - Control
Daily Body Weight - Control
b)
Daily Blood Glucose - 5mg/Kg
Daily Body Weight- 5mg/Kg
Blood Glucose (mg/dl)
Body weight(g)
STZ
TX
Days after first mPD-L1-mFc treatment

Fig. 17

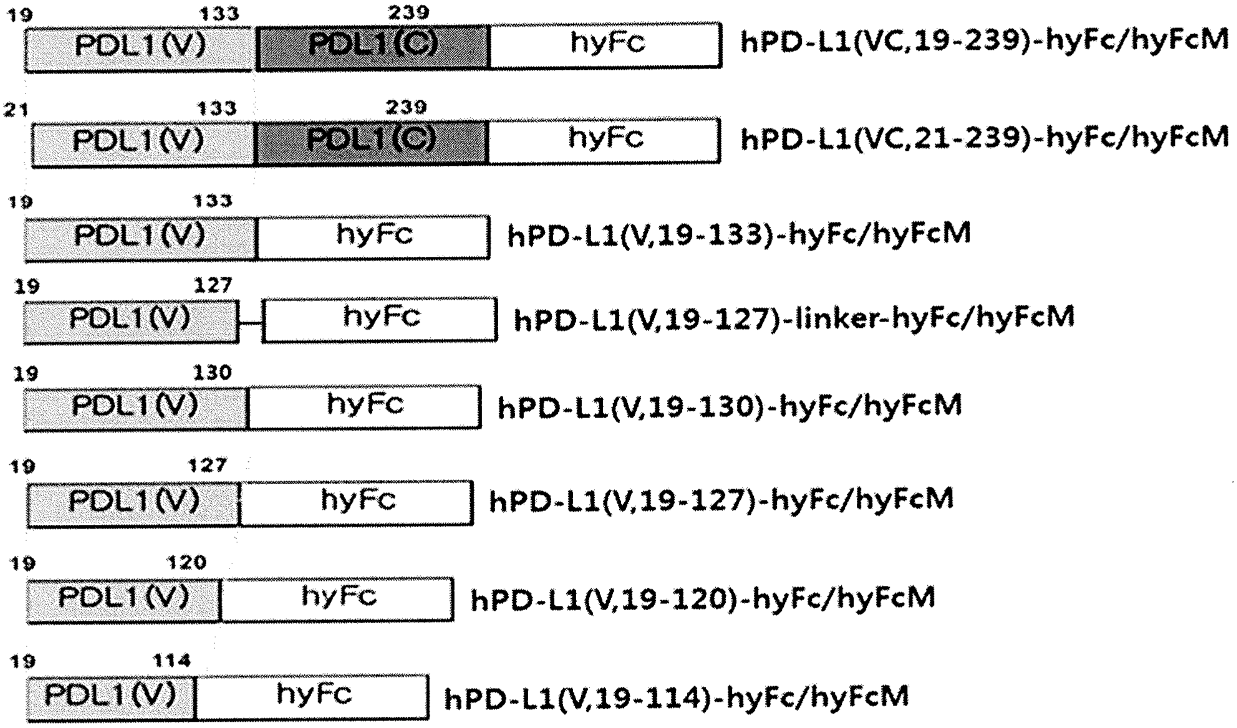
19
133
239
PDL1 (V)
PDL1 (C)
hyFc
hPD-L1(VC,19-239)-hyFc/hyFcM
21
133
239
PDL1 (V)
PDL1 (C)
hyFc
hPD-L1(VC,21-239)-hyFc/hyFcM
19
133
PDL1 (V)
hyFc
hPD-L1(V,19-133)-hyFc/hyFcM
19
127
PDL1 (V)
hyFc
hPD-L1(V,19-127)-linker-hyFc/hyFcM
19
130
PDL1 (V)
hyFc
hPD-L1(V,19-130)-hyFc/hyFcM
19
127
PDL1 (V)
hyFc
hPD-L1(V,19-127)-hyFc/hyFcM
19
120
PDL1 (V)
hyFc
hPD-L1(V,19-120)-hyFc/hyFcM
19
114
PDL1 (V)
hyFc
hPD-L1(V,19-114)-hyFc/hyFcM

Fig. 18

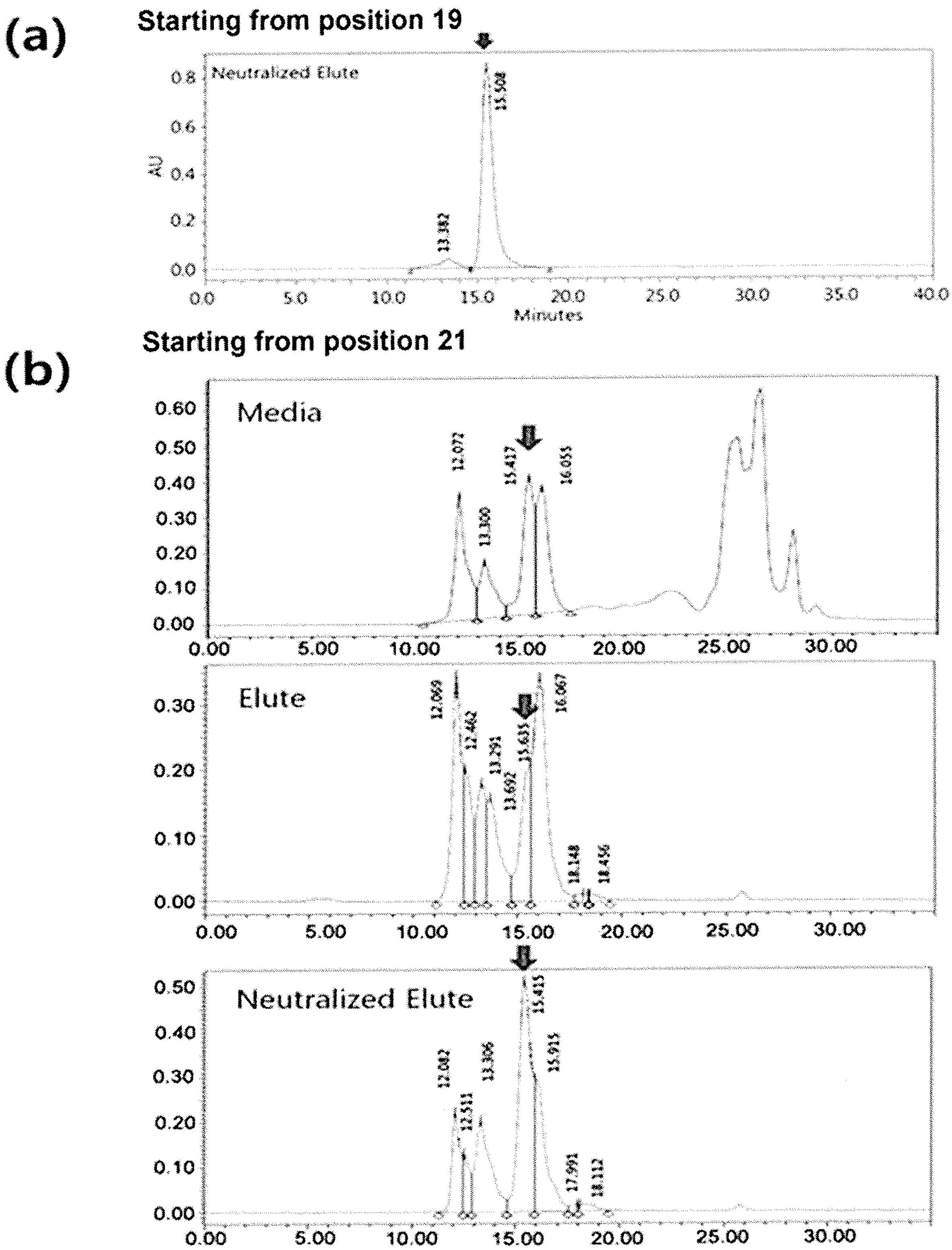
(a)
Starting from position 19
Neutralized Elute
AU
Minutes
(b)
Starting from position 21
Media
Elute
Neutralized Elute

Fig. 19

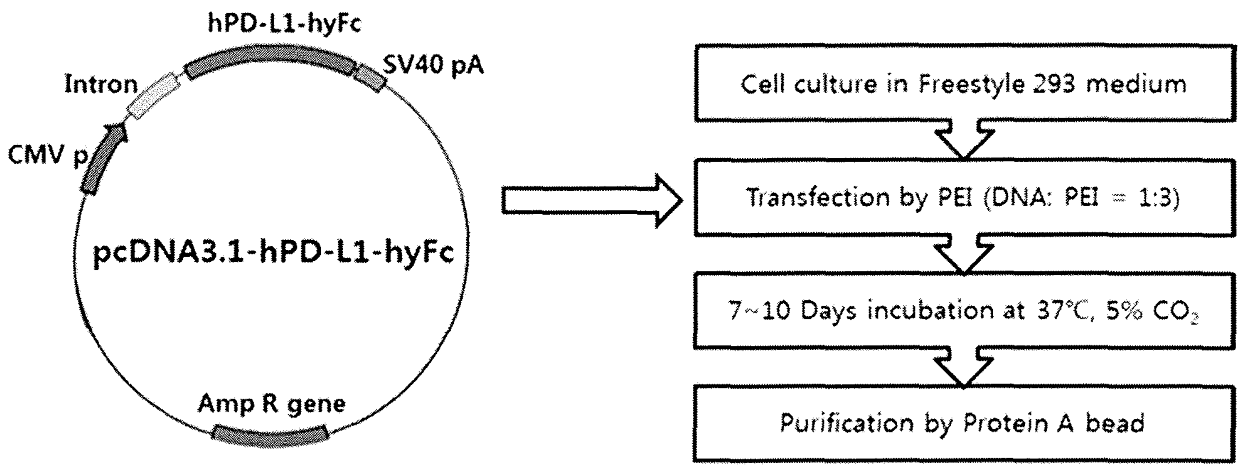
hPD-L1-hyFc
SV40 pA
Intron
CMV p
pcDNA3.1-hPD-L1-hyFc
Amp R gene
Cell culture in Freestyle 293 medium
Transfection by PEI (DNA: PEI = 1:3)
7~10 Days incubation at 37℃, 5% CO2
Purification by Protein A bead

Fig. 20

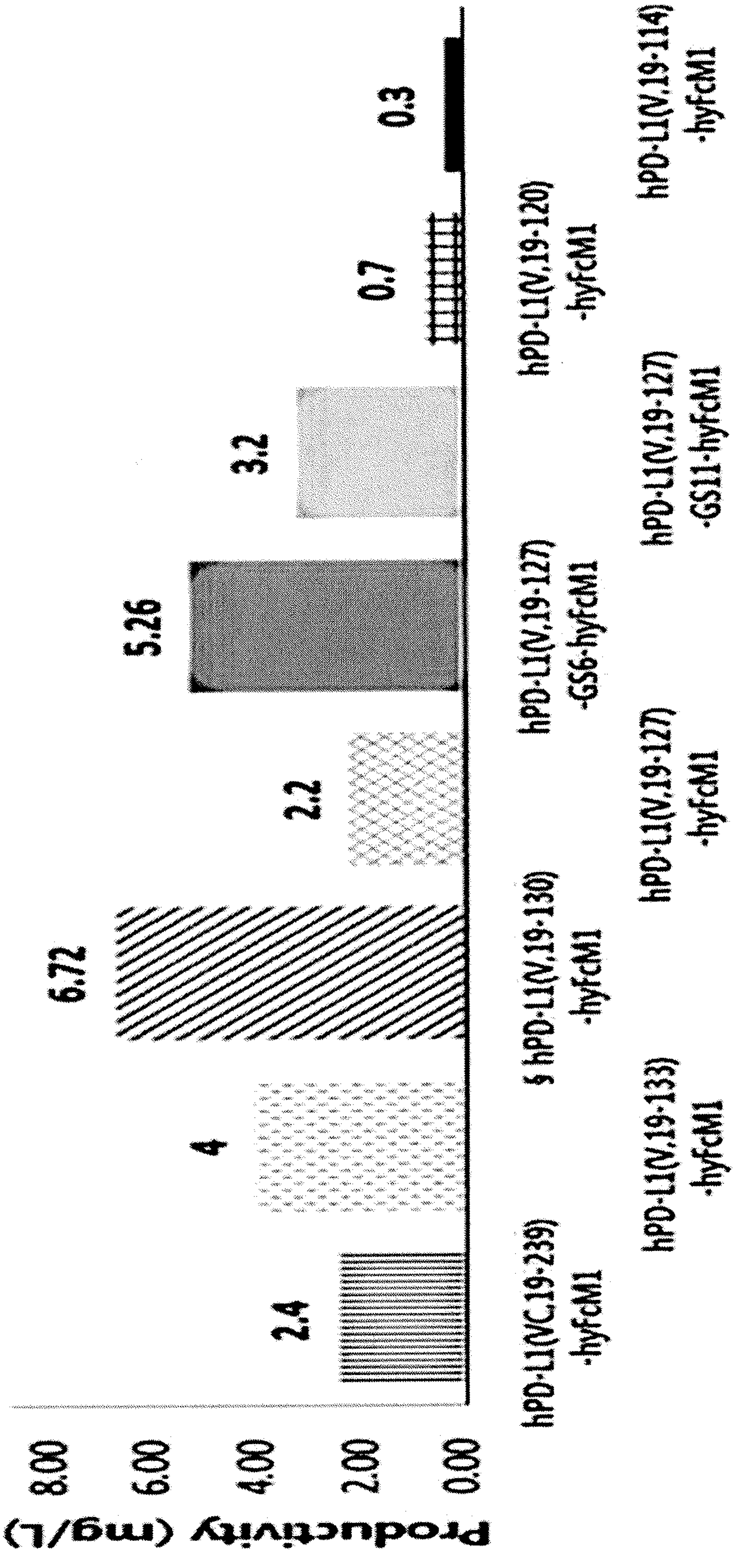
Productivity (mg/L)
8.00
6.00
4.00
2.00
0.00
2.4
4
6.72
2.2
5.26
3.2
0.7
0.3
hPD-L1(VC,19-239)-hyfcM1
hPD-L1(V,19-133)-hyfcM1
$ hPD-L1(V,19-130)-hyfcM1
hPD-L1(V,19-127)-hyfcM1
hPD-L1(V,19-127)-GS6-hyfcM1
hPD-L1(V,19-127)-GS11-hyfcM1
hPD-L1(V,19-120)-hyfcM1
hPD-L1(V,19-114)-hyfcM1

Fig. 21

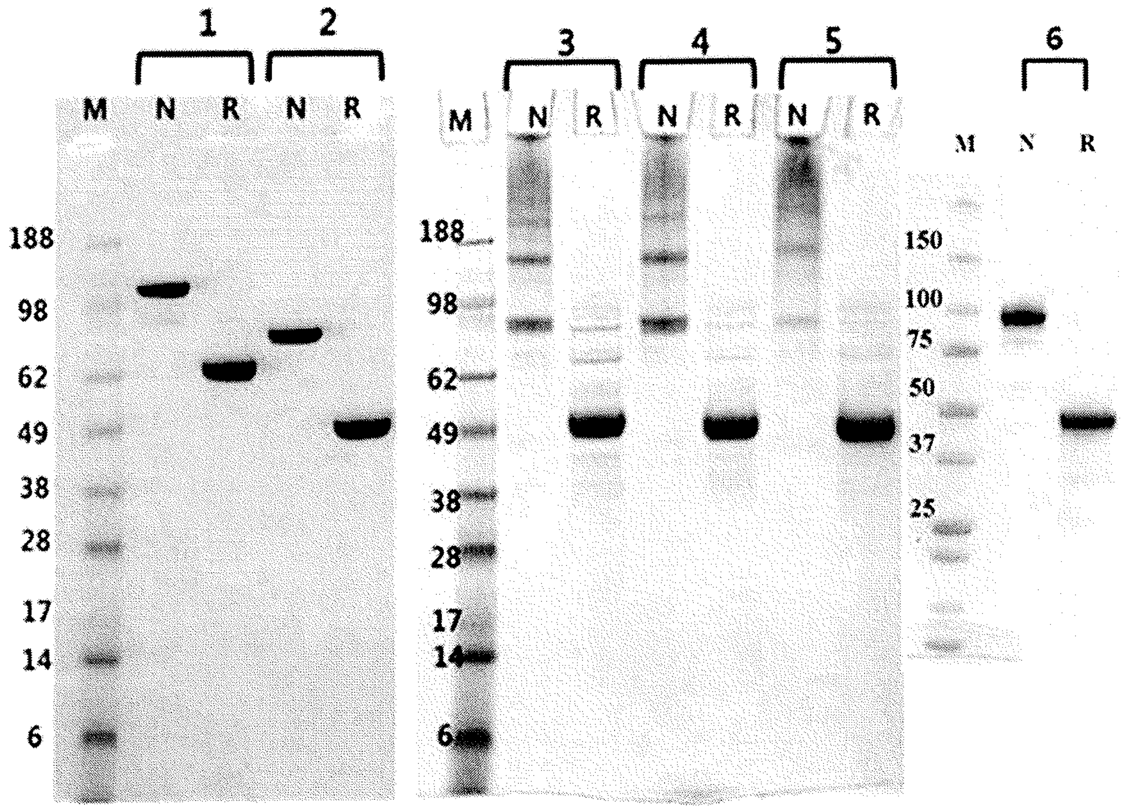


M: size marker
N: non-reduced
R: reduced
Lane 1: hPD-L1(VC,19-239)-hyFcM1
Lane 2: hPD-L1(V,19-133)-hyFcM1
Lane 3: hPD-L1(V,19-127)-hyFcM1
Lane 4: hPD-L1(V,19-120)-hyFcM1
Lane 5: hPD-L1(V,19-114)-hyFcM1
Lane 6: hPD-L1(V,19-130)-hyFcM1

Fig. 22

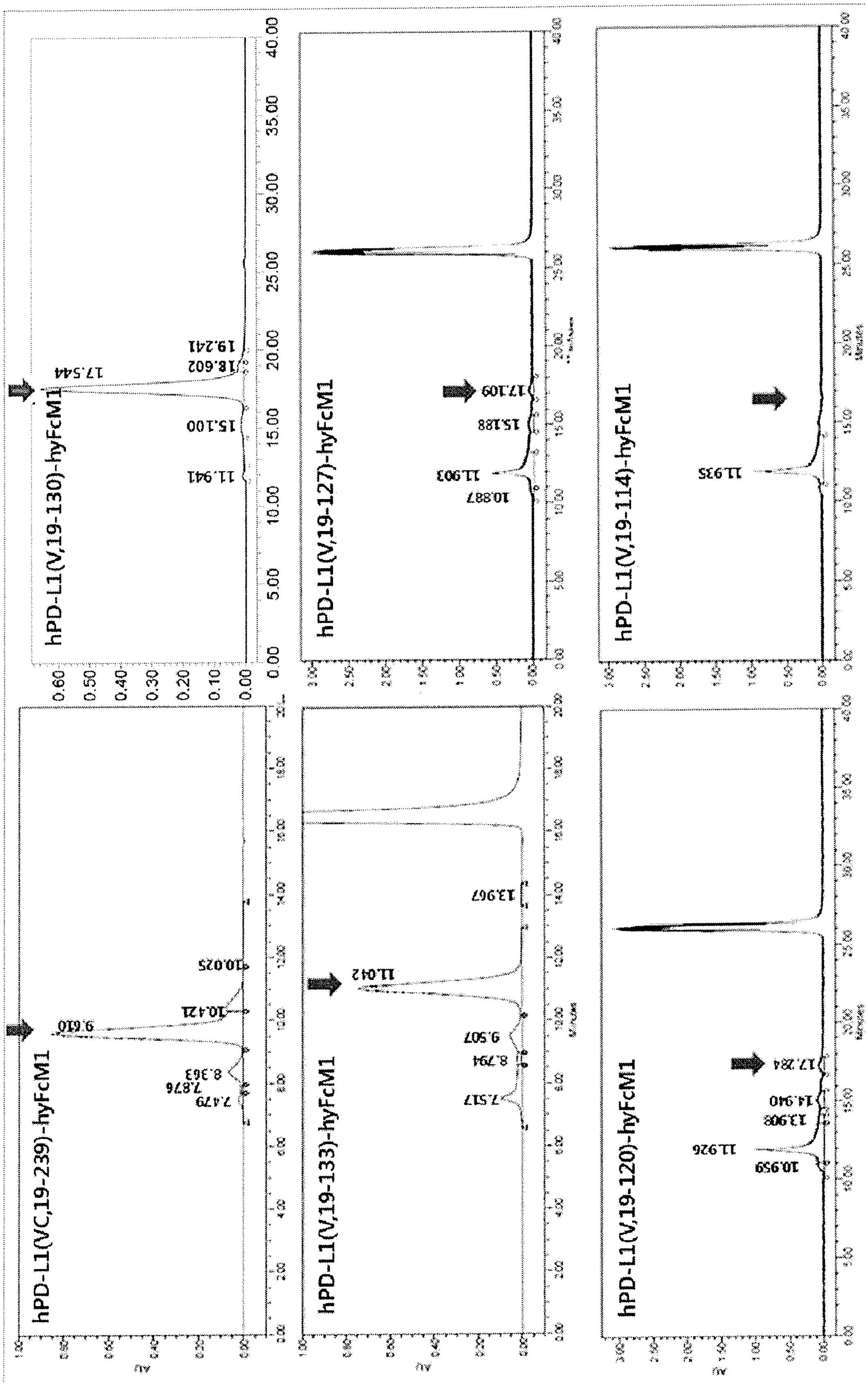
hPD-L1(V,19-130)-hyFcM1
hPD-L1(V,19-127)-hyFcM1
hPD-L1(V,19-114)-hyFcM1
hPD-L1(VC,19-239)-hyFcM1
hPD-L1(V,19-133)-hyFcM1
hPD-L1(V,19-120)-hyFcM1

Fig. 23

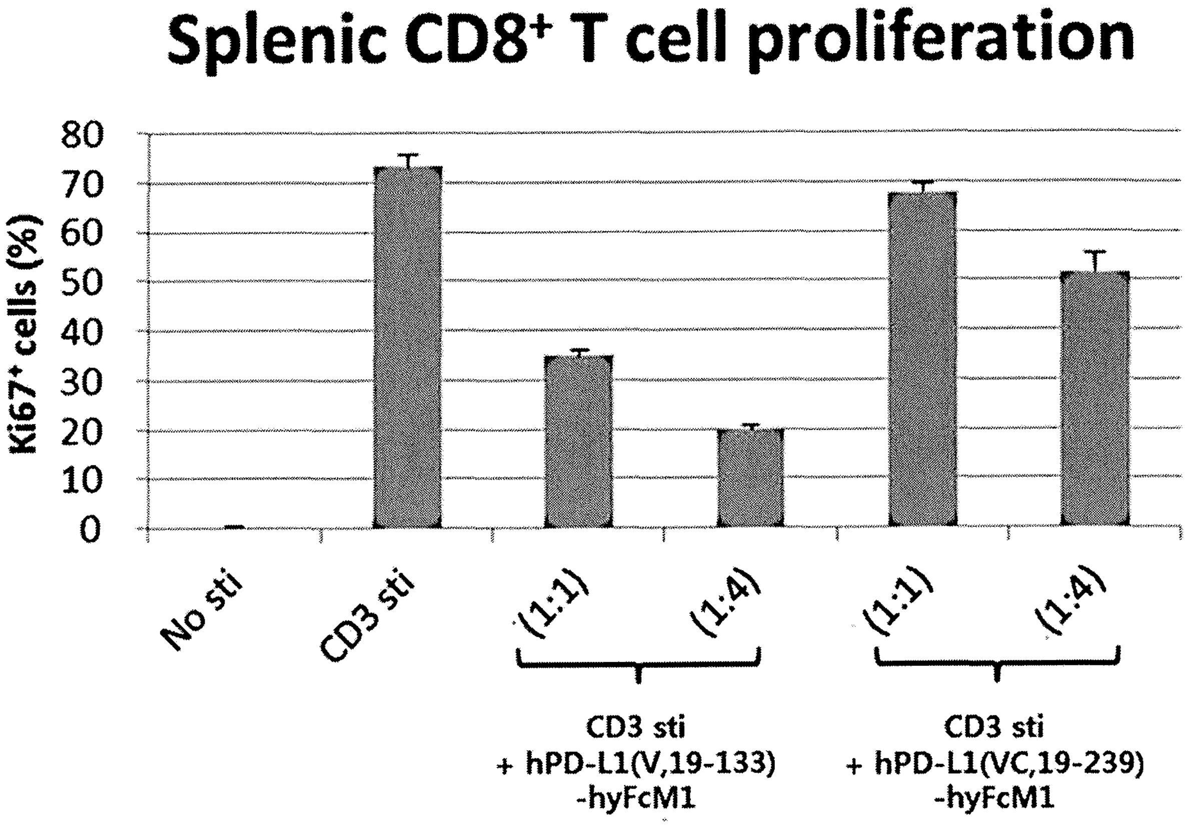
Splenic CD8+ T cell proliferation
Ki67+ cells (%)
80
70
60
50
40
30
20
10
0
No sti
CD3 sti
(1:1)
(1:4)
(1:1)
(1:4)
CD3 sti
+ hPD-L1(V,19-133)
-hyFcM1
CD3 sti
+ hPD-L1(VC,19-239)
-hyFcM1

Fig. 24

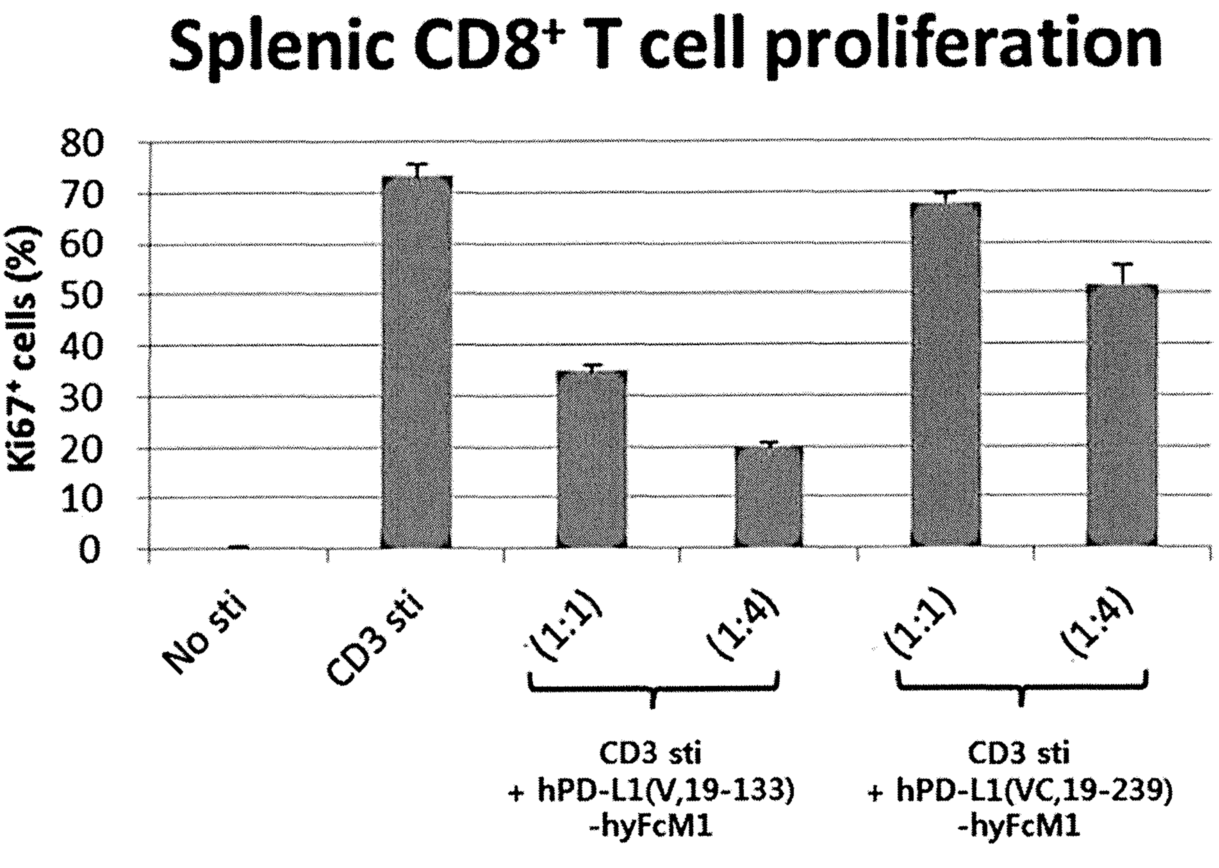
Splenic CD8+ T cell proliferation
Ki67+ cells (%)
80
70
60
50
40
30
20
10
0
No sti
CD3 sti
(1:1)
(1:4)
(1:1)
(1:4)
CD3 sti
+ hPD-L1(V,19-133)
-hyFcM1
CD3 sti
+ hPD-L1(VC,19-239)
-hyFcM1

Fig. 25

(a)

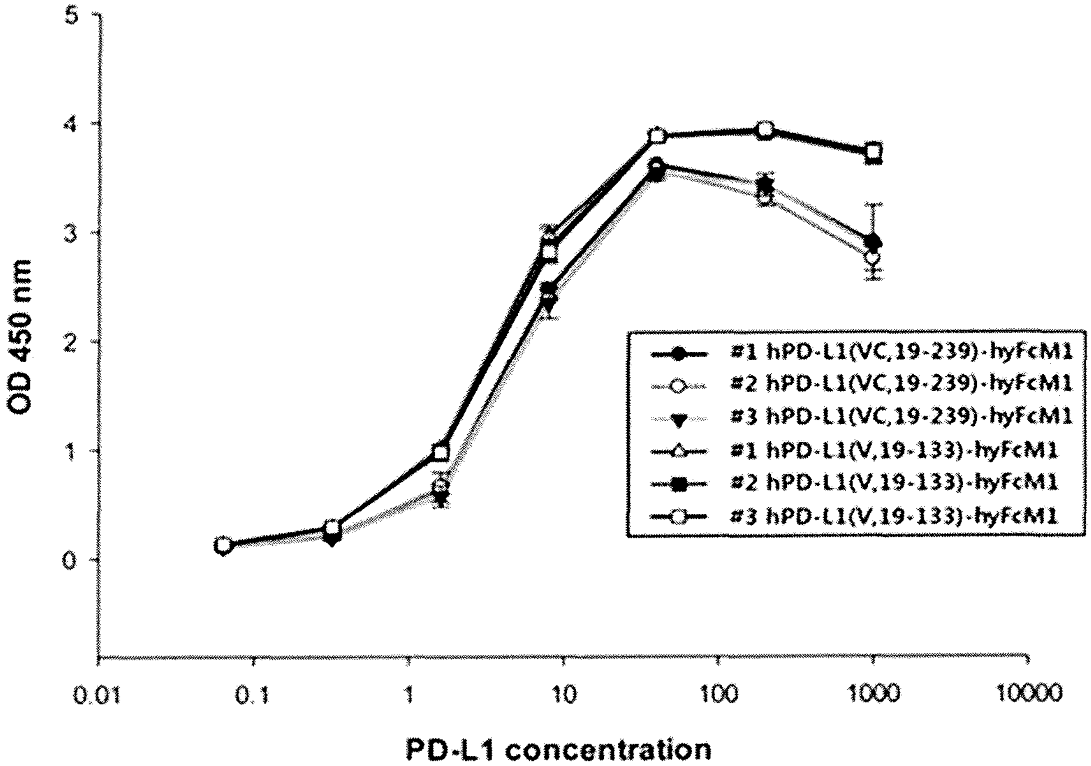


(b)

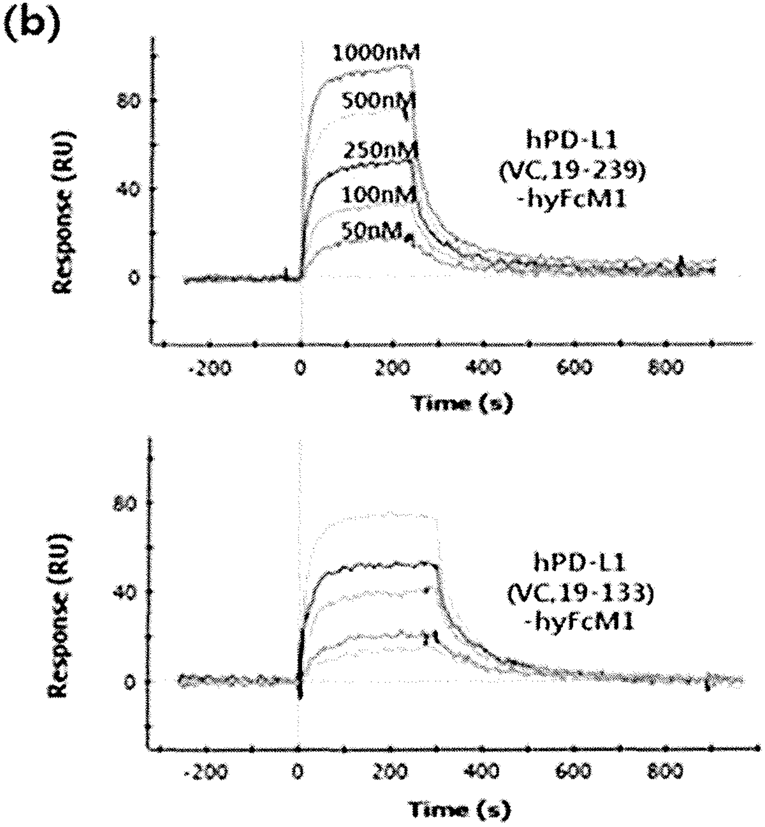


| Relative KA/KD | % for KA | % for KD |
|---|---|---|
| hPD-L1 | 100 | 100 |
| hPD-L1 (VC,19-239) -hyFcM1 | 88.4 | 86.8 |
| hPD-L1 (VC,19-133 )-hyFcM1 | 86.4 | 73.9 |

Fig. 26

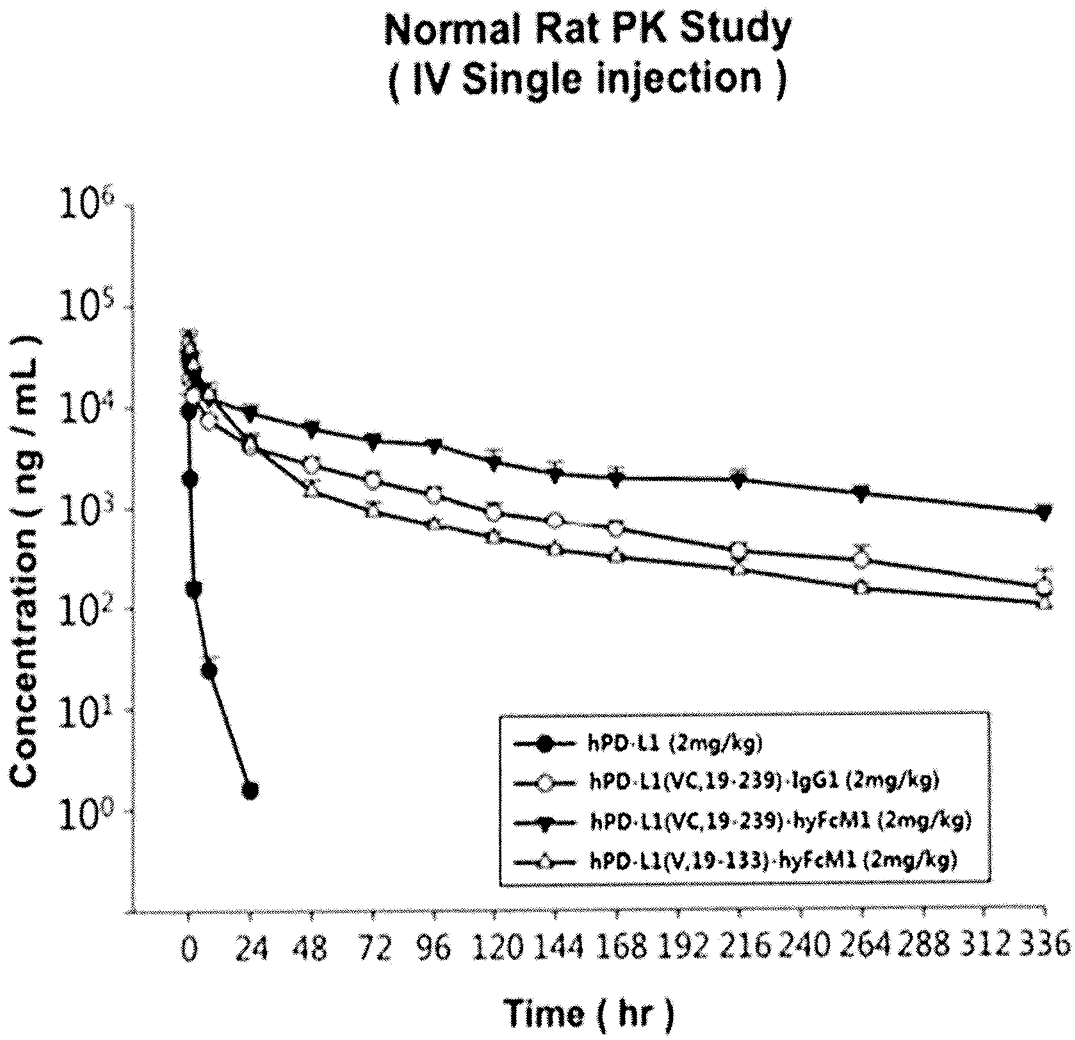


| | AUClast (ug*hr/ml) |
|---|---|
| hPD-L1 | 5.1 ± 1.2 |
| hPD-L1(VC,19-239)-IgG1 | 477.9 ± 85.9 |
| hPD-L1(VC,19-239)-hyFcM1 | 1168.9 ± 186.9 |
| hPD-L1(V,19-133)-hyFcM1 | 512.0 ± 133.7 |

Fig. 27

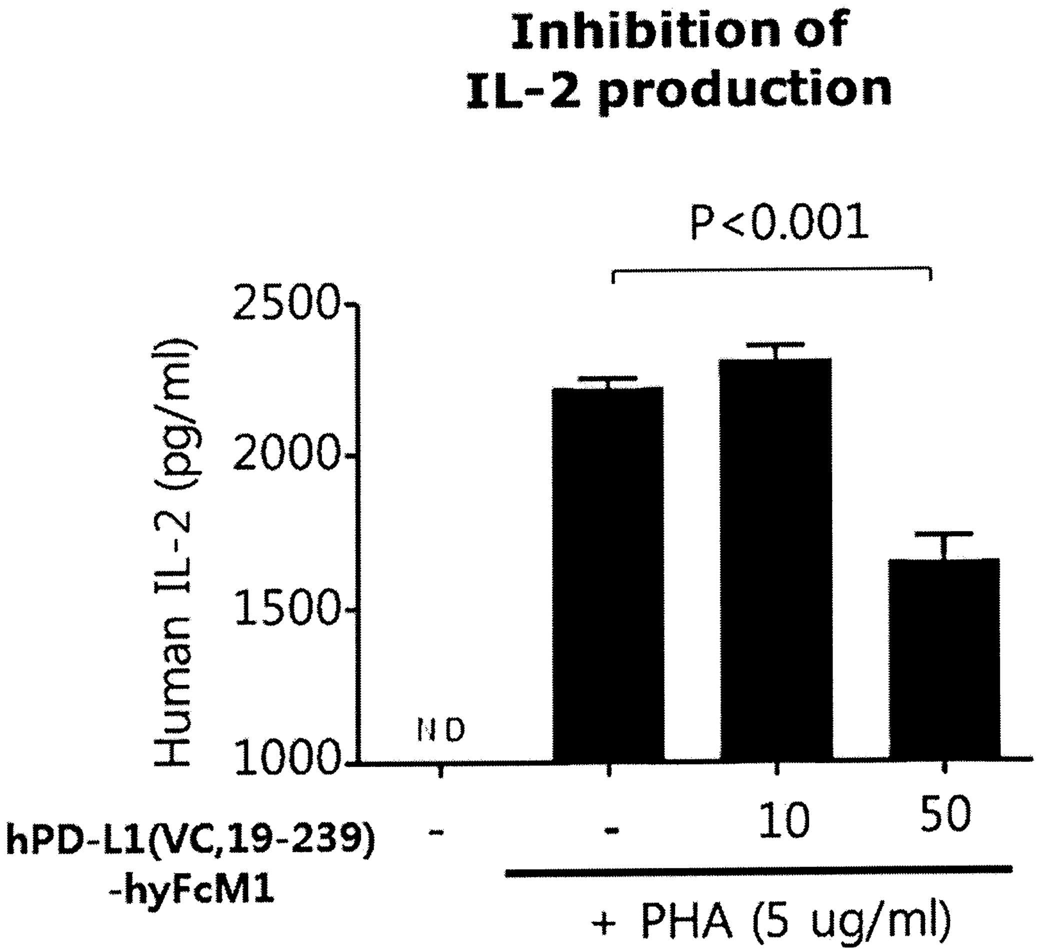
Inhibition of
IL-2 production
P<0.001
Human IL-2 (pg/ml)
2500
2000
1500
1000
ND
hPD-L1(VC,19-239)
-hyFcM1
-
-
10
50
+ PHA (5 ug/ml)

Fig. 28

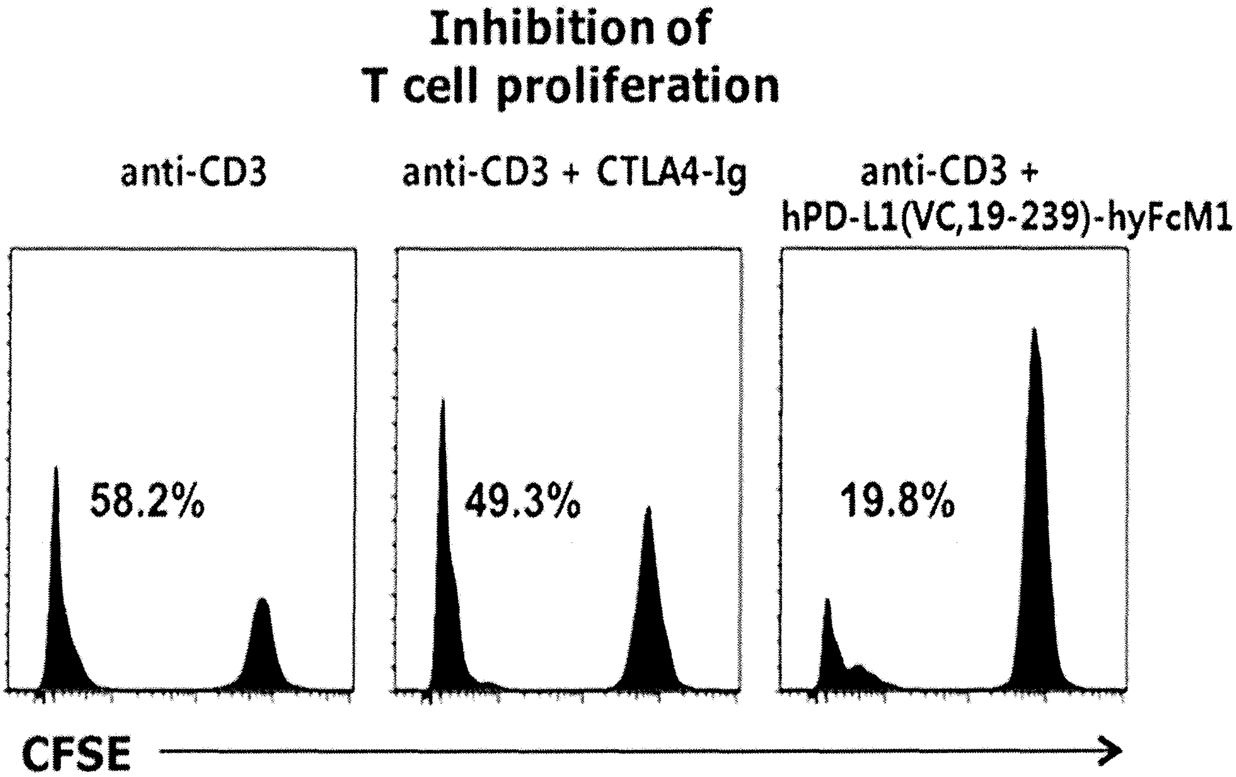
Inhibition of
T cell proliferation
anti-CD3
anti-CD3 + CTLA4-Ig
anti-CD3 +
hPD-L1(VC,19-239)-hyFcM1
58.2%
49.3%
19.8%
CFSE

Fig. 29

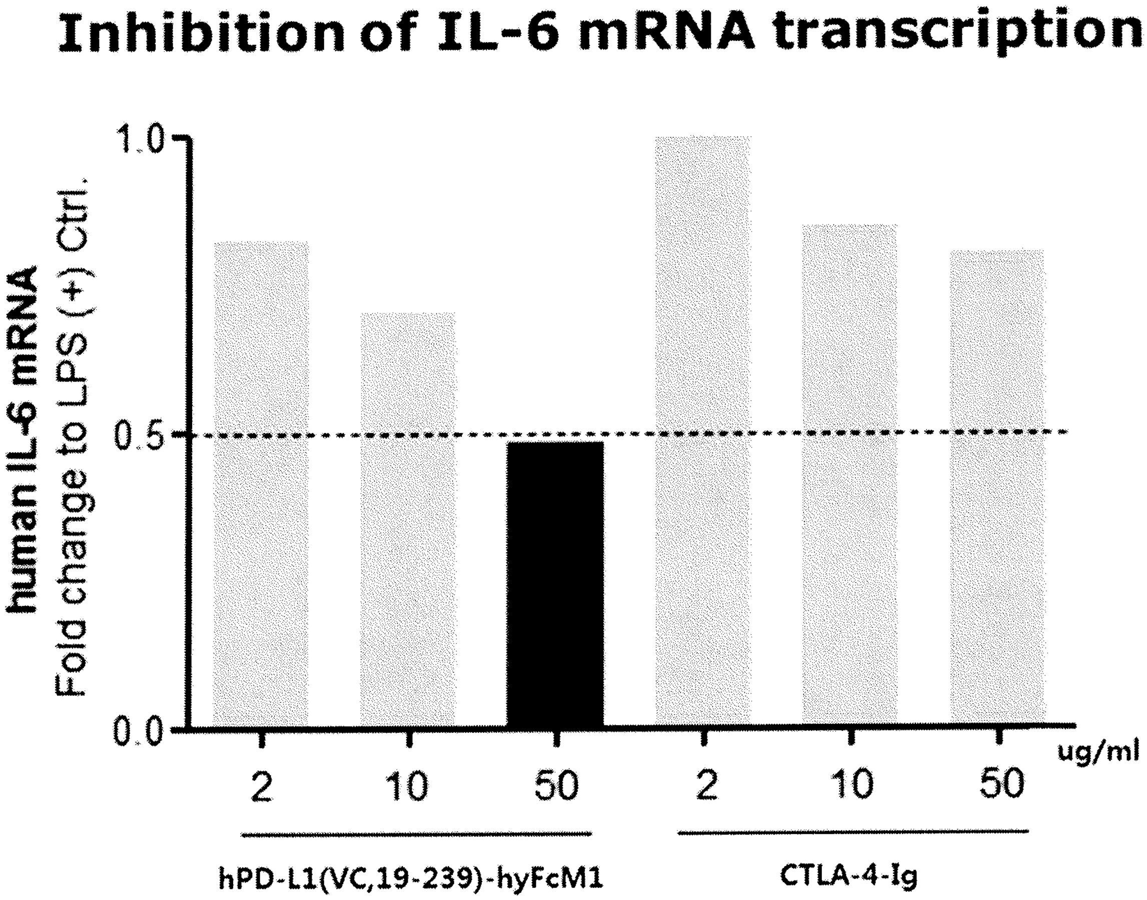
Inhibition of IL-6 mRNA transcription
human IL-6 mRNA
Fold change to LPS (+) Ctrl.
1.0
0.5
0.0
2
10
50
2
10
50
ug/ml
hPD-L1(VC,19-239)-hyFcM1
CTLA-4-Ig

Fig. 30

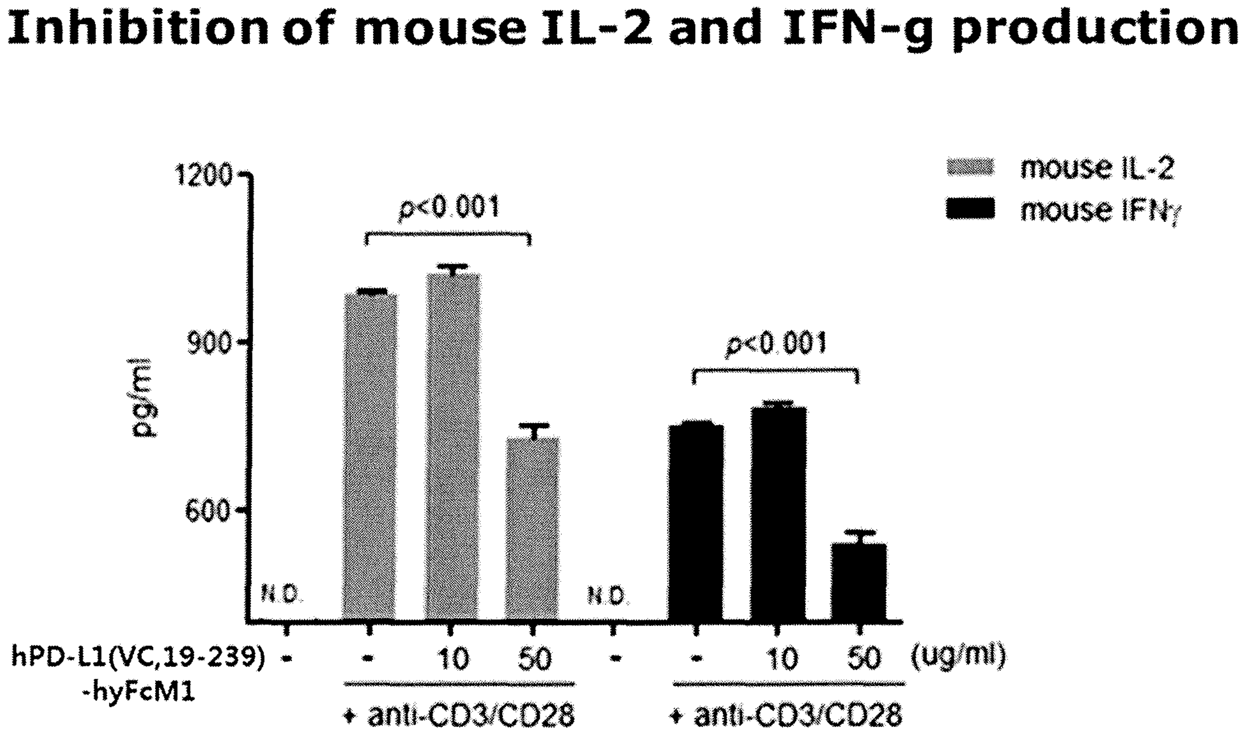
Inhibition of mouse IL-2 and IFN-g production
mouse IL-2
mouse IFNγ
1200
900
600
pg/ml
p<0.001
p<0.001
N.D.
N.D.
hPD-L1(VC,19-239)
-hyFcM1
- - 10 50 - - 10 50 (ug/ml)
+ anti-CD3/CD28
+ anti-CD3/CD28

Fig. 31

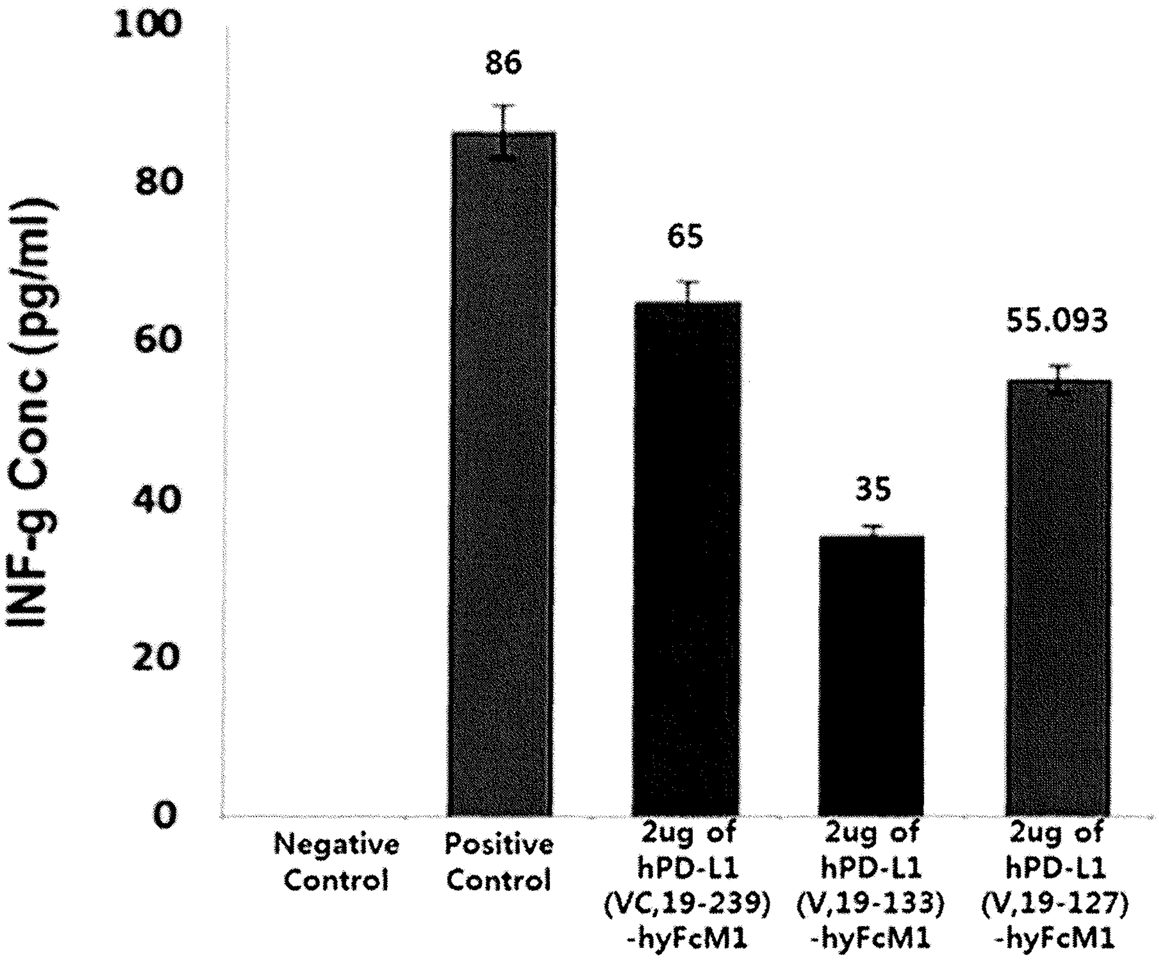
100
80
60
40
20
0
INF-g Conc (pg/ml)
86
65
35
55.093
Negative Control
Positive Control
2ug of hPD-L1 (VC,19-239) -hyFcM1
2ug of hPD-L1 (V,19-133) -hyFcM1
2ug of hPD-L1 (V,19-127) -hyFcM1

Fig. 32

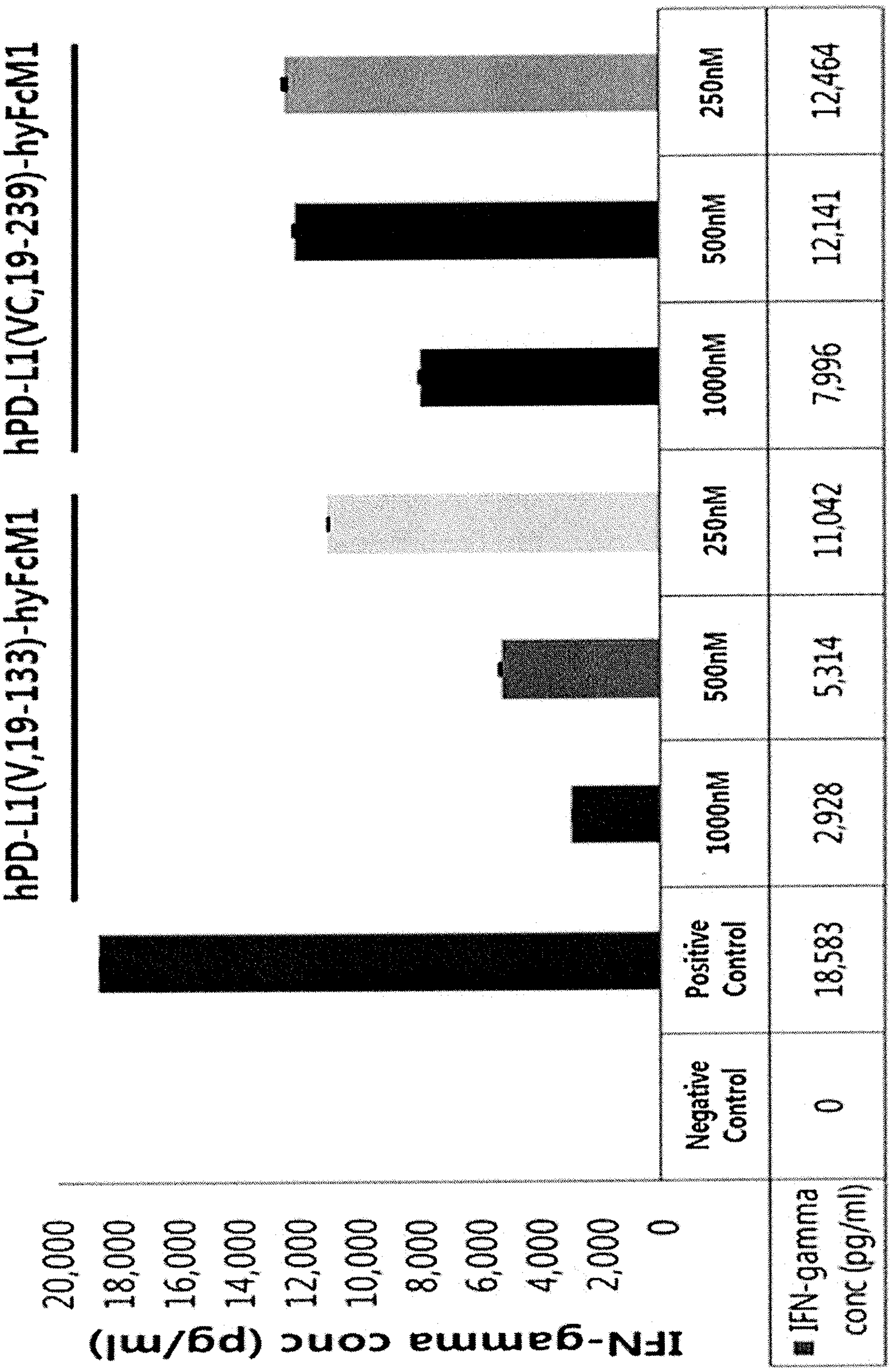
hPD-L1(V,19-133)-hyFcM1
hPD-L1(VC,19-239)-hyFcM1
IFN-gamma conc (pg/ml)
20,000
18,000
16,000
14,000
12,000
10,000
8,000
6,000
4,000
2,000
0
Negative Control
Positive Control
1000nM
500nM
250nM
1000nM
500nM
250nM
■ IFN-gamma conc (pg/ml)
0
18,583
2,928
5,314
11,042
7,996
12,141
12,464

Fig. 33

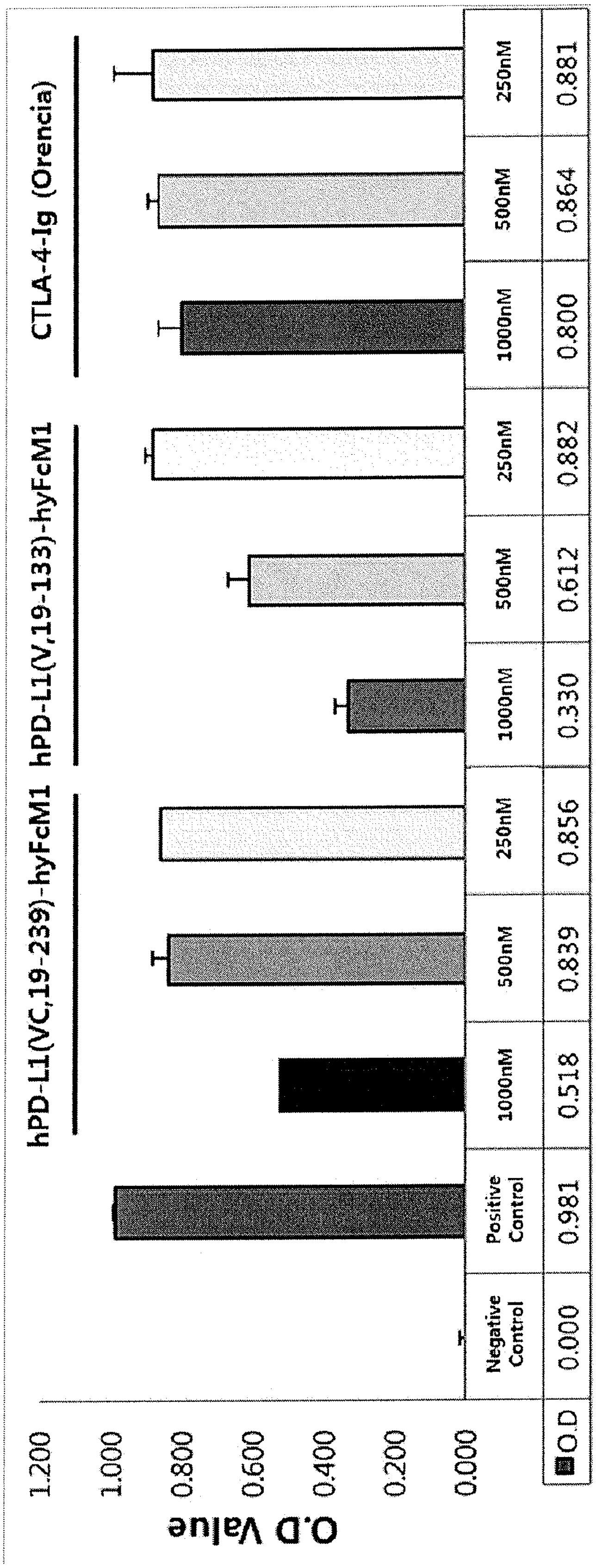
O.D Value
1.200
1.000
0.800
0.600
0.400
0.200
0.000
hPD-L1(VC,19-239)-hyFcM1
hPD-L1(V,19-133)-hyFcM1
CTLA-4-Ig (Orencia)
Negative Control
Positive Control
1000nM
500nM
250nM
1000nM
500nM
250nM
1000nM
500nM
250nM
O.D
0.000
0.981
0.518
0.839
0.856
0.330
0.612
0.882
0.800
0.864
0.881

Fig. 34

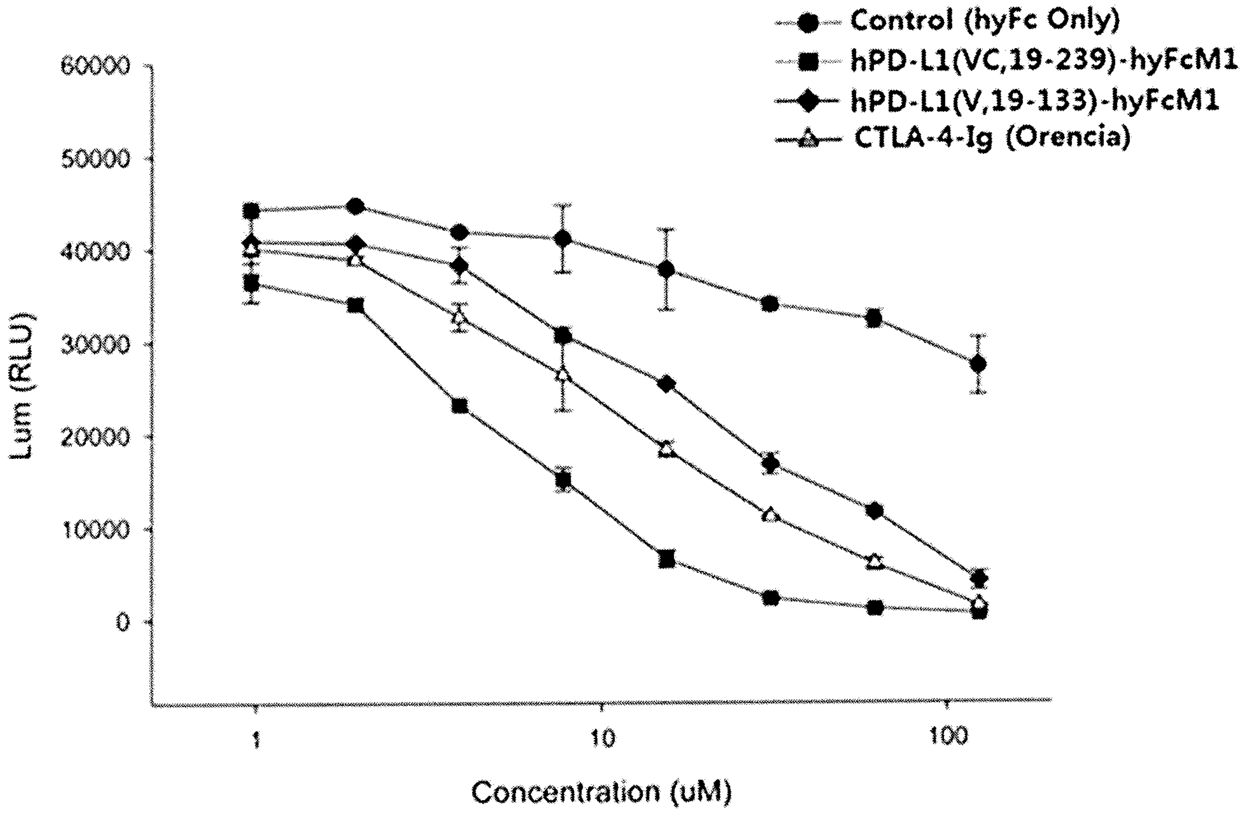


| Relative potency | | | |
|---|---|---|---|
| | hPD-L1(VC,19-239)-hyFcM1 | hPD-L1(V,19-133)-hyFcM1 | CTLA-4-Ig (Orencia) |
| **Log$IC_{50}$** | 3.96 | 15.52 | 7.88 |
| **Relative %** | 100 | 26 | 50 |

Fig. 35

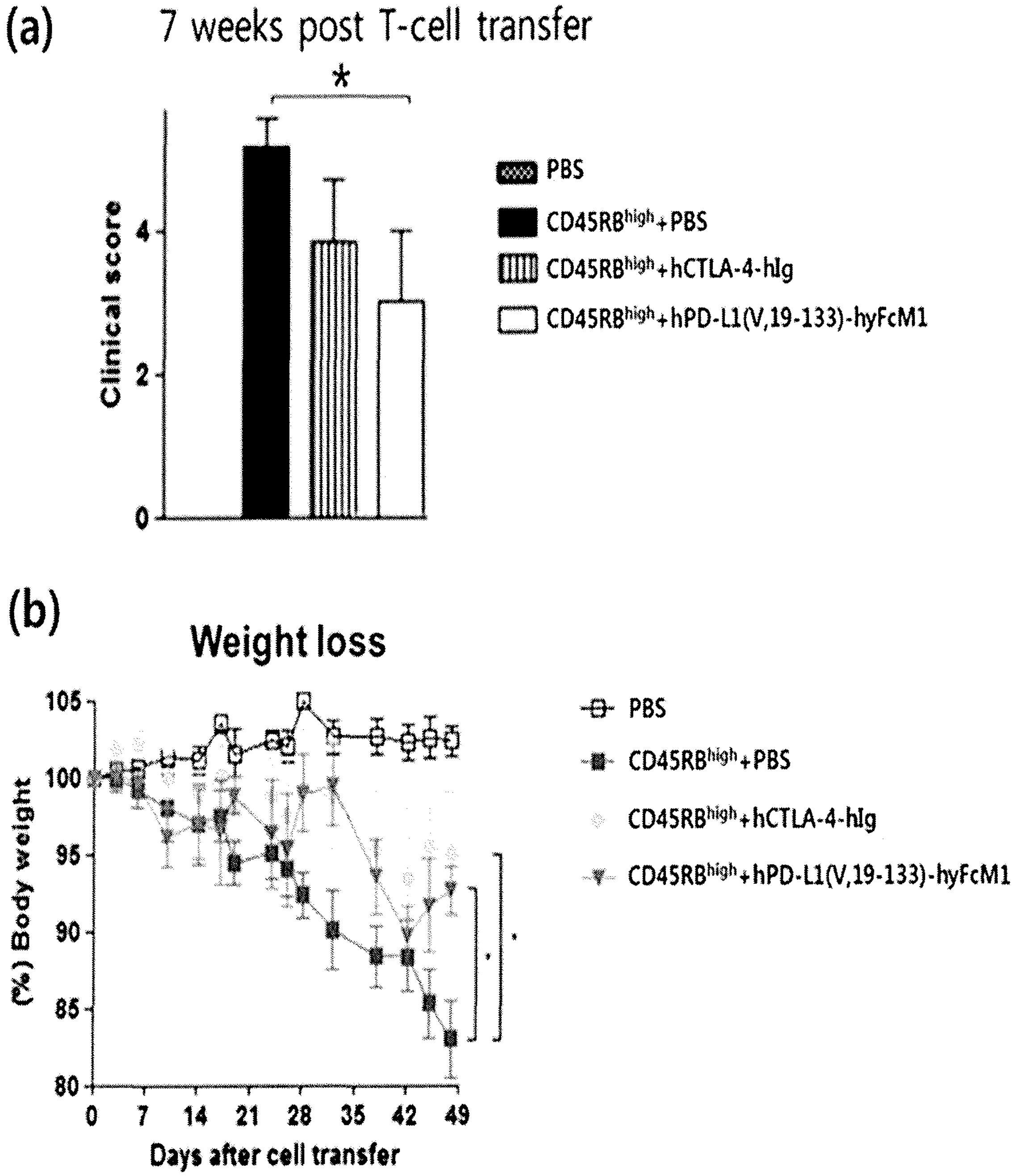
(a)
7 weeks post T-cell transfer
*
Clinical score
0
2
4
PBS
CD45RBhigh+PBS
CD45RBhigh+hCTLA-4-hIg
CD45RBhigh+hPD-L1(V,19-133)-hyFcM1
(b)
Weight loss
(%) Body weight
80
85
90
95
100
105
0
7
14
21
28
35
42
49
Days after cell transfer
PBS
CD45RBhigh+PBS
CD45RBhigh+hCTLA-4-hIg
CD45RBhigh+hPD-L1(V,19-133)-hyFcM1

Fig. 36

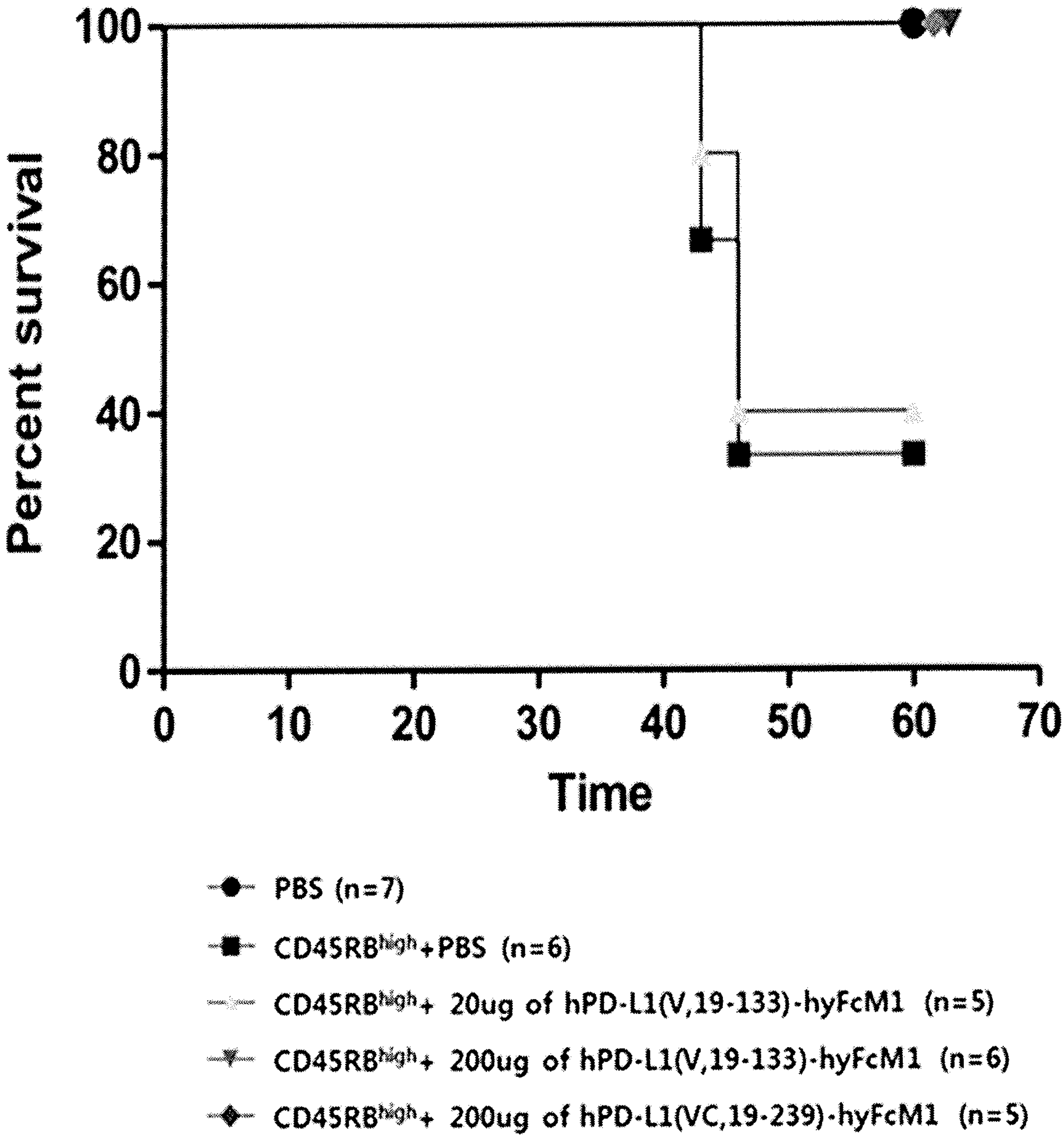
100
80
60
40
20
0
Percent survival
0
10
20
30
40
50
60
70
Time
PBS (n=7)
CD45RBhigh+PBS (n=6)
CD45RBhigh+ 20ug of hPD-L1(V,19-133)-hyFcM1 (n=5)
CD45RBhigh+ 200ug of hPD-L1(V,19-133)-hyFcM1 (n=6)
CD45RBhigh+ 200ug of hPD-L1(VC,19-239)-hyFcM1 (n=5)

Fig. 37

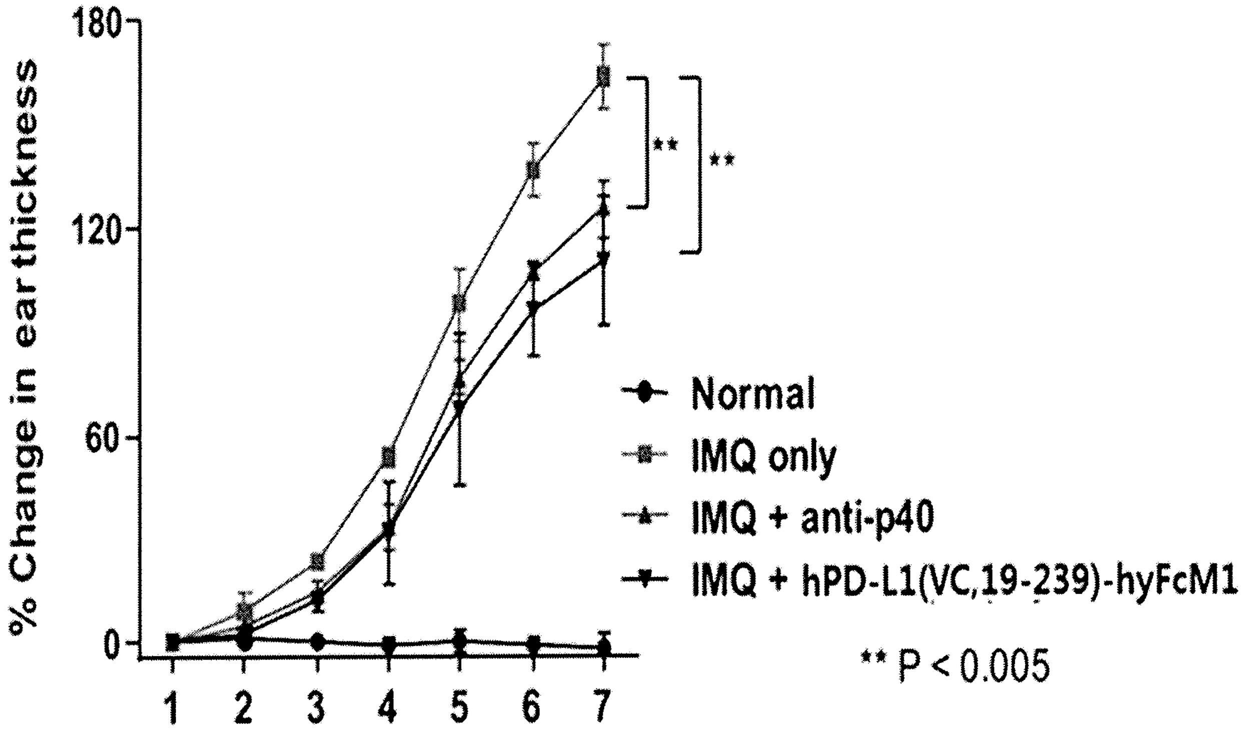
% Change in ear thickness
180
120
60
0
1
2
3
4
5
6
7
**
**
Normal
IMQ only
IMQ + anti-p40
IMQ + hPD-L1(VC,19-239)-hyFcM1
** P < 0.005

Fig. 38

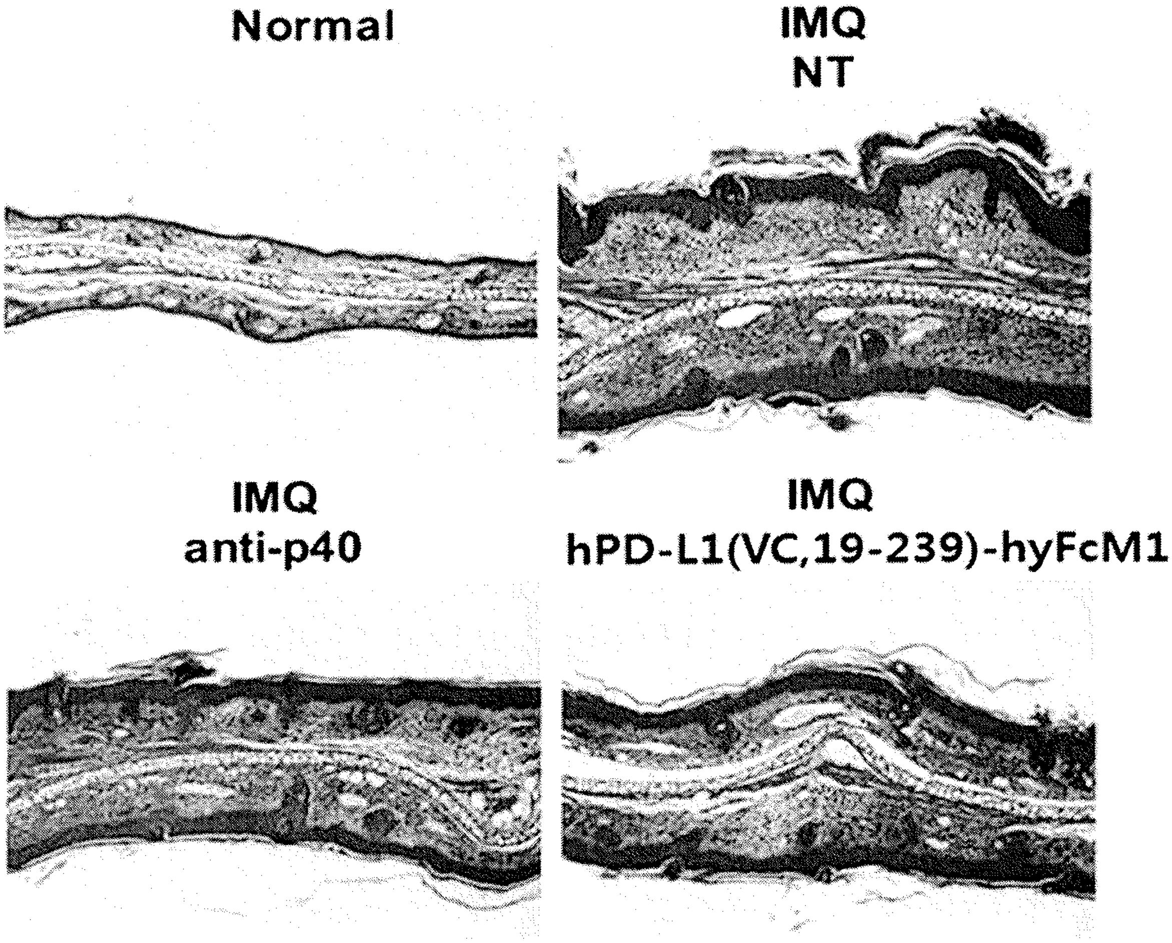

Fig. 39

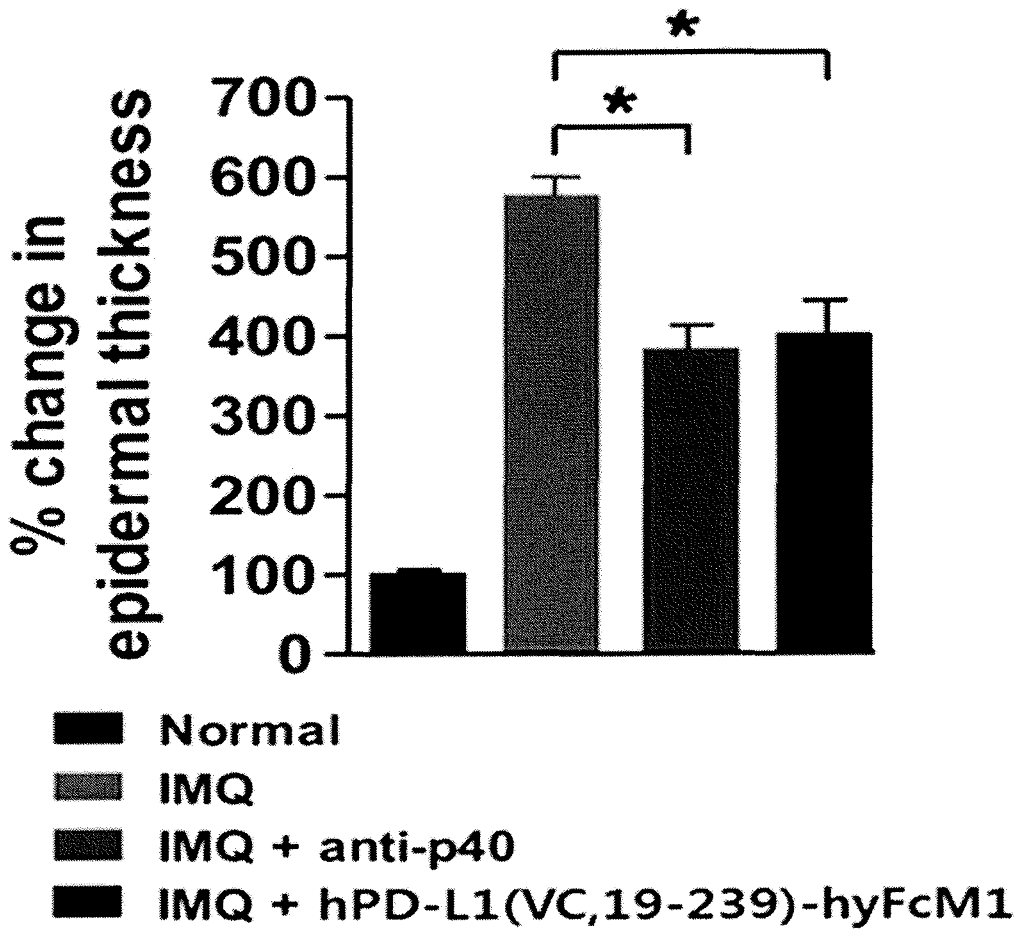
% change in epidermal thickness
700
600
500
400
300
200
100
0
*
*
Normal
IMQ
IMQ + anti-p40
IMQ + hPD-L1(VC,19-239)-hyFcM1

Fig. 40

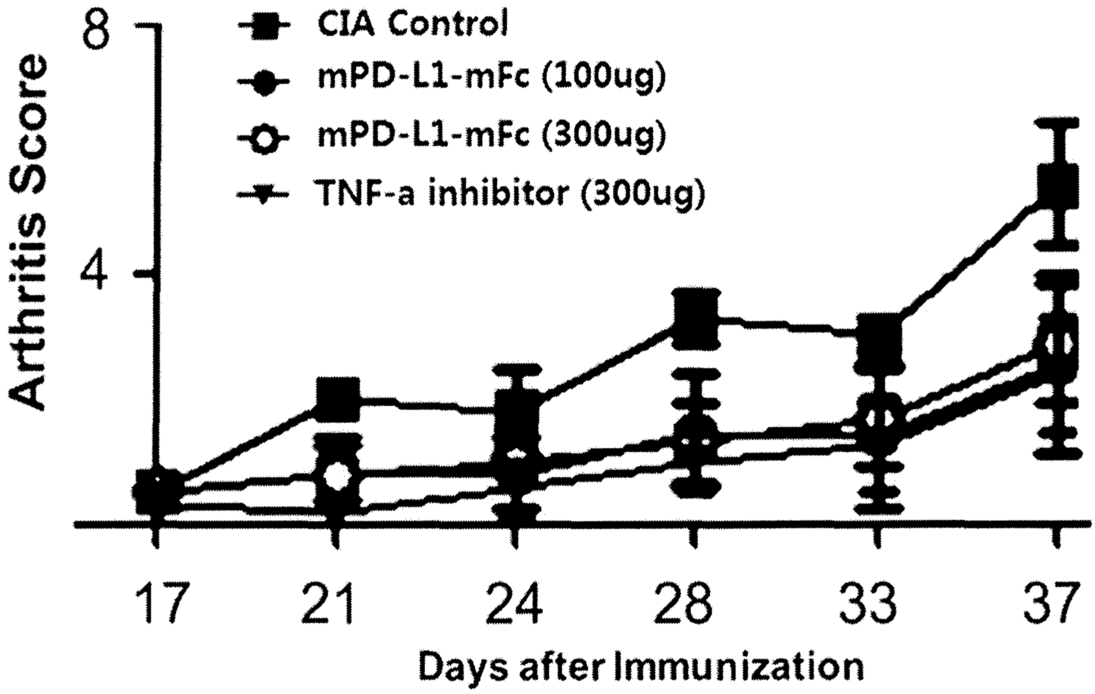
8
4
Arthritis Score
CIA Control
mPD-L1-mFc (100ug)
mPD-L1-mFc (300ug)
TNF-a inhibitor (300ug)
17
21
24
28
33
37
Days after Immunization

INTERNATIONAL SEARCH REPORT

International application No.
**PCT/KR2015/005256**

A. CLASSIFICATION OF SUBJECT MATTER

***C07K 19/00(2006.01)i, C12N 15/62(2006.01)i, A61K 38/17(2006.01)i, A61K 39/395(2006.01)i, A61P 37/02(2006.01)i***

According to International Patent Classification (IPC) or to both national classification and IPC

B. FIELDS SEARCHED

Minimum documentation searched (classification system followed by classification symbols)
C07K 19/00; C12N 15/62; A61K 38/17; A61K 39/395; A61P 37/02

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: PD-L1, extracellular domain, Fc region, fusion protein, immune tolerance, cell-mediated cytotoxicity, ADCC, CDC

C. DOCUMENTS CONSIDERED TO BE RELEVANT

| Category* | Citation of document, with indication, where appropriate, of the relevant passages | Relevant to claim No. |
|---|---|---|
| X | WATSON et al., "Differential effects of costimulatory pathway modulation on corneal allograft survival" Investigative Ophthalmology and Visual Science, Vol. 47, No. 8, pp. 3417-3422 (2006)<br>See abstract; and pages 3418-3421. | 1-22,25-29 |
| A | IgG-Fc engineering for therapeutic use, Issue from InvivoGen Insight Newletters (2006) <URL: http://www.invivogen.com/docs/Insight200605.pdf><br>See the entire document. | 1-22,25-29 |
| A | PARK et al., "B7-H1/CD80 interaction is required for the induction and maintenance of peripheral T-cell tolerance" Blood, Vol. 116, No. 8, pp. 1291-1298 (2010)<br>See abstract; and page 1292. | 1-22,25-29 |
| A | WO 2011-109789 A2 (THE JOHNS HOPKINS UNIVERSITY) 09 September 2011<br>See abstract; and claims 1, 2. | 1-22,25-29 |
| A | NCBI, Genbank assession No. CAA04843.1 (27 April 2010)<br>See the entire document. | 1-22,25-29 |

☐ Further documents are listed in the continuation of Box C.    ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 AUGUST 2015 (21.08.2015) | **24 AUGUST 2015 (24.08.2015)** |

| Name and mailing address of the ISA/**KR** | Authorized officer |
|---|---|
| Korean Intellectual Property Office<br>Government Complex-Daejeon, 189 Seonsa-ro, Daejeon 302-701, Republic of Korea<br>Facsimile No. 82-42-472-7140 | Telephone No. |

Form PCT/ISA/210 (second sheet) (July 2009)

INTERNATIONAL SEARCH REPORT

International application No.
**PCT/KR2015/005256**

**Box No. II Observations where certain claims were found unsearchable (Continuation of item 2 of first sheet)**

This international search report has not been established in respect of certain claims under Article 17(2)(a) for the following reasons:

1. ☒ Claims Nos.: **30-33**
because they relate to subject matter not required to be searched by this Authority, namely:
Claims 30-33 pertain to a method for treatment of the human body, and thus pertain to subject matter on which the International Searching Authority is not required to carry out an international search under the provisions of PCT Article 17 (2)(a)(i) and PCT Rule 39.1(iv).

2. ☒ Claims Nos.: **24**
because they relate to parts of the international application that do not comply with the prescribed requirements to such an extent that no meaningful international search can be carried out, specifically:
Claim 24 refers to a claim which violates the manner of claiming a multiple dependent claim, and thus it is not clear which feature is claimed (PCT Article 6).

3. ☒ Claims Nos.: **23**
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

INTERNATIONAL SEARCH REPORT
Information on patent family members

International application No.
**PCT/KR2015/005256**

| Patent document cited in search report | Publication date | Patent family member | Publication date |
|---|---|---|---|
| WO 2011-109789 A2 | 09/09/2011 | EP 2542590 A2 | 09/01/2013 |
| | | EP 2542590 A4 | 31/07/2013 |
| | | JP 2013-521311 A | 10/06/2013 |
| | | US 2013-0039911 A1 | 14/02/2013 |
| | | US 8993524 B2 | 31/03/2015 |
| | | WO 2011-109789 A3 | 05/04/2012 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7867491 B
- KR 1020080094781 A

### Non-patent literature cited in the description

- *Immunity,* July 2007, vol. 27 (1), 111-22
- *Immunol Rev.,* July 2010, vol. 236, 219-42
- *Rheumatol Int.,* April 2011, vol. 31 (4), 513-9
- **GILLIES et al.** *J. Immunol. Meth,* 1989, vol. 125, 191-202
- **SAKANO et al.** *Nature,* 1980, vol. 286, 676-683
- **WATSON et al.** *Nucleic Acid Research,* 1984, vol. 12, 5145-5164
- *International Journal of inflammation,* 2012, 412178

<110> Genexine, Inc.
Postech academy-industry foundation

<120> PD-L1 Fused protein and use thereof

<130> PCB505033GNX

<150> KR 10-2014-0062399
<151> 2014-05-23

<150> KR 10-2015-0071820
<151> 2015-05-22

<160> 50

<170> KopatentIn 2.0

<210> 1
<211> 290
<212> PRT
<213> Artificial Sequence

<220>
<223> amino acid sequence of mouse PD-L1 (Accession number : Q9EP73)

<400> 1
```
Met Arg Ile Phe Ala Gly Ile Ile Phe Thr Ala Cys Cys His Leu Leu
  1               5                  10                  15

Arg Ala Phe Thr Ile Thr Ala Pro Lys Asp Leu Tyr Val Val Glu Tyr
             20                  25                  30

Gly Ser Asn Val Thr Met Glu Cys Arg Phe Pro Val Glu Arg Glu Leu
         35                  40                  45

Asp Leu Leu Ala Leu Val Val Tyr Trp Glu Lys Glu Asp Glu Gln Val
     50                  55                  60

Ile Gln Phe Val Ala Gly Glu Glu Asp Leu Lys Pro Gln His Ser Asn
 65                  70                  75                  80

Phe Arg Gly Arg Ala Ser Leu Pro Lys Asp Gln Leu Leu Lys Gly Asn
                 85                  90                  95

Ala Ala Leu Gln Ile Thr Asp Val Lys Leu Gln Asp Ala Gly Val Tyr
            100                 105                 110

Cys Cys Ile Ile Ser Tyr Gly Gly Ala Asp Tyr Lys Arg Ile Thr Leu
        115                 120                 125

Lys Val Asn Ala Pro Tyr Arg Lys Ile Asn Gln Arg Ile Ser Val Asp
    130                 135                 140

Pro Ala Thr Ser Glu His Glu Leu Ile Cys Gln Ala Glu Gly Tyr Pro
145                 150                 155                 160

Glu Ala Glu Val Ile Trp Thr Asn Ser Asp His Gln Pro Val Ser Gly
                165                 170                 175

Lys Arg Ser Val Thr Thr Ser Arg Thr Glu Gly Met Leu Leu Asn Val
            180                 185                 190

Thr Ser Ser Leu Arg Val Asn Ala Thr Ala Asn Asp Val Phe Tyr Cys
        195                 200                 205

Thr Phe Trp Arg Ser Gln Pro Gly Gln Asn His Thr Ala Glu Leu Ile
    210                 215                 220
```

Ile Pro Glu Leu Pro Ala Thr His Pro Pro Gln Asn Arg Thr His Trp
225 230 235 240

Val Leu Leu Gly Ser Ile Leu Leu Phe Leu Ile Val Val Ser Thr Val
245 250 255

Leu Leu Phe Leu Arg Lys Gln Val Arg Met Leu Asp Val Glu Lys Cys
260 265 270

Gly Val Glu Asp Thr Ser Ser Lys Asn Arg Asn Asp Thr Gln Phe Glu
275 280 285

Glu Thr
290

<210> 2
<211> 243
<212> PRT
<213> Artificial Sequence

<220>
<223> amino acid sequence of mouse IgG2a Fc variant

<400> 2
Ala Ser Ala Glu Pro Arg Gly Pro Thr Ile Lys Pro Cys Pro Pro Cys
1 5 10 15

Lys Cys Pro Ala Pro Asn Leu Glu Gly Gly Pro Ser Val Phe Ile Phe
20 25 30

Pro Pro Lys Ile Lys Asp Val Leu Met Ile Ser Leu Ser Pro Ile Val
35 40 45

Thr Cys Val Val Val Asp Val Ser Glu Asp Asp Pro Asp Val Gln Ile
50 55 60

Ser Trp Phe Val Asn Asn Val Glu Val His Thr Ala Gln Thr Gln Thr
65 70 75 80

His Arg Glu Asp Tyr Asn Ser Thr Leu Arg Val Val Ser Ala Leu Pro
85 90 95

Ile Gln His Gln Asp Trp Met Ser Gly Lys Ala Phe Ala Cys Ala Val
100 105 110

Asn Asn Lys Asp Leu Pro Ala Pro Ile Glu Arg Thr Ile Ser Lys Pro
115 120 125

Lys Gly Ser Val Arg Ala Pro Gln Val Tyr Val Leu Pro Pro Pro Glu
130 135 140

Glu Glu Met Thr Lys Lys Gln Val Thr Leu Thr Cys Met Val Thr Asp
145 150 155 160

Phe Met Pro Glu Asp Ile Tyr Val Glu Trp Thr Asn Asn Gly Lys Thr
165 170 175

Glu Leu Asn Tyr Lys Asn Thr Glu Pro Val Leu Asp Ser Asp Gly Ser
180 185 190

Tyr Phe Met Tyr Ser Lys Leu Arg Val Glu Lys Lys Asn Trp Val Glu
195 200 205

Arg Asn Ser Tyr Ser Cys Ser Val Val His Glu Gly Leu His Asn His
210 215 220

His Thr Thr Lys Ser Phe Ser Arg Thr Pro Gly Lys Gly Gly Gly Asn
225 230 235 240

Ser Gly Ser

<210> 3
<211> 290
<212> PRT
<213> Artificial Sequence

<220>
<223> amino acid sequence of human PD-L1(Accession number : Q9NZQ7)

<400> 3
Met Arg Ile Phe Ala Val Phe Ile Phe Met Thr Tyr Trp His Leu Leu
1 5 10 15

Asn Ala Phe Thr Val Thr Val Pro Lys Asp Leu Tyr Val Val Glu Tyr
20 25 30

Gly Ser Asn Met Thr Ile Glu Cys Lys Phe Pro Val Glu Lys Gln Leu
35 40 45

Asp Leu Ala Ala Leu Ile Val Tyr Trp Glu Met Glu Asp Lys Asn Ile
50 55 60

Ile Gln Phe Val His Gly Glu Glu Asp Leu Lys Val Gln His Ser Ser
65 70 75 80

Tyr Arg Gln Arg Ala Arg Leu Leu Lys Asp Gln Leu Ser Leu Gly Asn
85 90 95

Ala Ala Leu Gln Ile Thr Asp Val Lys Leu Gln Asp Ala Gly Val Tyr
100 105 110

Arg Cys Met Ile Ser Tyr Gly Gly Ala Asp Tyr Lys Arg Ile Thr Val
115 120 125

Lys Val Asn Ala Pro Tyr Asn Lys Ile Asn Gln Arg Ile Leu Val Val
130 135 140

Asp Pro Val Thr Ser Glu His Glu Leu Thr Cys Gln Ala Glu Gly Tyr
145 150 155 160

Pro Lys Ala Glu Val Ile Trp Thr Ser Ser Asp His Gln Val Leu Ser
165 170 175

Gly Lys Thr Thr Thr Thr Asn Ser Lys Arg Glu Glu Lys Leu Phe Asn
180 185 190

Val Thr Ser Thr Leu Arg Ile Asn Thr Thr Thr Asn Glu Ile Phe Tyr
195 200 205

Cys Thr Phe Arg Arg Leu Asp Pro Glu Glu Asn His Thr Ala Glu Leu
210 215 220

Val Ile Pro Glu Leu Pro Leu Ala His Pro Pro Asn Glu Arg Thr His
225 230 235 240

Leu Val Ile Leu Gly Ala Ile Leu Leu Cys Leu Gly Val Ala Leu Thr
245 250 255

Phe Ile Phe Arg Leu Arg Lys Gly Arg Met Met Asp Val Lys Lys Cys
260 265 270

Gly Ile Gln Asp Thr Asn Ser Lys Lys Gln Ser Asp Thr His Leu Glu
275 280 285

Glu Thr
290

<210> 4
<211> 384
<212> PRT
<213> Artificial Sequence

<220>
<223> amino acid sequence of human IgD constant region (Genbank accession No. P01880)

<400> 4
Ala Pro Thr Lys Ala Pro Asp Val Phe Pro Ile Ile Ser Gly Cys Arg
1 5 10 15

His Pro Lys Asp Asn Ser Pro Val Val Leu Ala Cys Leu Ile Thr Gly
20 25 30

Tyr His Pro Thr Ser Val Thr Val Thr Trp Tyr Met Gly Thr Gln Ser
35 40 45

Gln Pro Gln Arg Thr Phe Pro Glu Ile Gln Arg Arg Asp Ser Tyr Tyr
50 55 60

Met Thr Ser Ser Gln Leu Ser Thr Pro Leu Gln Gln Trp Arg Gln Gly
65 70 75 80

Glu Tyr Lys Cys Val Val Gln His Thr Ala Ser Lys Ser Lys Lys Glu
85 90 95

Ile Phe Arg Trp Pro Glu Ser Pro Lys Ala Gln Ala Ser Ser Val Pro
100 105 110

Thr Ala Gln Pro Gln Ala Glu Gly Ser Leu Ala Lys Ala Thr Thr Ala
115 120 125

Pro Ala Thr Thr Arg Asn Thr Gly Arg Gly Gly Glu Glu Lys Lys Lys
130 135 140

Glu Lys Glu Lys Glu Glu Gln Glu Glu Arg Glu Thr Lys Thr Pro Glu
145 150 155 160

Cys Pro Ser His Thr Gln Pro Leu Gly Val Tyr Leu Leu Thr Pro Ala
165 170 175

Val Gln Asp Leu Trp Leu Arg Asp Lys Ala Thr Phe Thr Cys Phe Val
180 185 190

Val Gly Ser Asp Leu Lys Asp Ala His Leu Thr Trp Glu Val Ala Gly
195 200 205

Lys Val Pro Thr Gly Gly Val Glu Glu Gly Leu Leu Glu Arg His Ser
210 215 220

Asn Gly Ser Gln Ser Gln His Ser Arg Leu Thr Leu Pro Arg Ser Leu
225 230 235 240

Trp Asn Ala Gly Thr Ser Val Thr Cys Thr Leu Asn His Pro Ser Leu
245 250 255

Pro Pro Gln Arg Leu Met Ala Leu Arg Glu Pro Ala Ala Gln Ala Pro

```
                260                     265                     270

Val Lys Leu Ser Leu Asn Leu Leu Ala Ser Ser Asp Pro Pro Glu Ala
        275                     280                     285

Ala Ser Trp Leu Leu Cys Glu Val Ser Gly Phe Ser Pro Pro Asn Ile
    290                     295                     300

Leu Leu Met Trp Leu Glu Asp Gln Arg Glu Val Asn Thr Ser Gly Phe
305                     310                     315                     320

Ala Pro Ala Arg Pro Pro Pro Gln Pro Gly Ser Thr Thr Phe Trp Ala
                325                     330                     335

Trp Ser Val Leu Arg Val Pro Ala Pro Pro Ser Pro Gln Pro Ala Thr
            340                     345                     350

Tyr Thr Cys Val Val Ser His Glu Asp Ser Arg Thr Leu Leu Asn Ala
        355                     360                     365

Ser Arg Ser Leu Glu Val Ser Tyr Val Thr Asp His Gly Pro Met Lys
    370                     375                     380


<210>   5
<211>   327
<212>   PRT
<213>   Artificial Sequence

<220>
<223>   amino acid sequence of Partial human IgG4 constant region
        (Genbank accession No. AAH25985)

<400>   5
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
```

165 170 175

Asn Ser Thr Tyr Arg Val Val Ser Val Leu Thr Val Leu His Gln Asp
180 185 190

Trp Leu Asn Gly Lys Glu Tyr Lys Cys Lys Val Ser Asn Lys Gly Leu
195 200 205

Pro Ser Ser Ile Glu Lys Thr Ile Ser Lys Ala Lys Gly Gln Pro Arg
210 215 220

Glu Pro Gln Val Tyr Thr Leu Pro Pro Ser Gln Glu Glu Met Thr Lys
225 230 235 240

Asn Gln Val Ser Leu Thr Cys Leu Val Lys Gly Phe Tyr Pro Ser Asp
245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Leu Gly Lys
325

<210> 6
<211> 245
<212> PRT
<213> Artificial Sequence

<220>
<223> amino acid sequence of hyFc

<400> 6
Arg Asn Thr Gly Arg Gly Gly Glu Glu Lys Lys Lys Glu Lys Glu Lys
1 5 10 15

Glu Glu Gln Glu Glu Arg Glu Thr Lys Thr Pro Glu Cys Pro Ser His
20 25 30

Thr Gln Pro Leu Gly Val Phe Leu Phe Pro Pro Lys Pro Lys Asp Thr
35 40 45

Leu Met Ile Ser Arg Thr Pro Glu Val Thr Cys Val Val Val Asp Val
50 55 60

Ser Gln Glu Asp Pro Glu Val Gln Phe Asn Trp Tyr Val Asp Gly Val
65 70 75 80

Glu Val His Asn Ala Lys Thr Lys Pro Arg Glu Glu Gln Phe Asn Ser
85 90 95

Thr Tyr Arg Val Val Ser Val Leu Thr Val Leu His Gln Asp Trp Leu
100 105 110

Asn Gly Lys Glu Tyr Lys Cys Lys Val Ser Asn Lys Gly Leu Pro Ser
115 120 125

Ser Ile Glu Lys Thr Ile Ser Lys Ala Lys Gly Gln Pro Arg Glu Pro

130 135 140

Gln Val Tyr Thr Leu Pro Pro Ser Gln Glu Glu Met Thr Lys Asn Gln
145 150 155 160

Val Ser Leu Thr Cys Leu Val Lys Gly Phe Tyr Pro Ser Asp Ile Ala
165 170 175

Val Glu Trp Glu Ser Asn Gly Gln Pro Glu Asn Asn Tyr Lys Thr Thr
180 185 190

Pro Pro Val Leu Asp Ser Asp Gly Ser Phe Phe Leu Tyr Ser Arg Leu
195 200 205

Thr Val Asp Lys Ser Arg Trp Gln Glu Gly Asn Val Phe Ser Cys Ser
210 215 220

Val Met His Glu Ala Leu His Asn His Tyr Thr Gln Lys Ser Leu Ser
225 230 235 240

Leu Ser Leu Gly Lys
245

<210> 7
<211> 245
<212> PRT
<213> Artificial Sequence

<220>
<223> amino acid sequence of hyFcM1

<400> 7
Arg Asn Thr Gly Arg Gly Gly Glu Glu Lys Lys Gly Gly Lys Glu Lys
1 5 10 15

Glu Glu Gln Glu Glu Arg Glu Thr Lys Thr Pro Glu Cys Pro Ser His
20 25 30

Thr Gln Pro Leu Gly Val Phe Leu Phe Pro Pro Lys Pro Lys Asp Thr
35 40 45

Leu Met Ile Ser Arg Thr Pro Glu Val Thr Cys Val Val Val Asp Val
50 55 60

Ser Gln Glu Asp Pro Glu Val Gln Phe Asn Trp Tyr Val Asp Gly Val
65 70 75 80

Glu Val His Asn Ala Lys Thr Lys Pro Arg Glu Glu Gln Phe Asn Ser
85 90 95

Thr Tyr Arg Val Val Ser Val Leu Thr Val Leu His Gln Asp Trp Leu
100 105 110

Asn Gly Lys Glu Tyr Lys Cys Lys Val Ser Asn Lys Gly Leu Pro Ser
115 120 125

Ser Ile Glu Lys Thr Ile Ser Lys Ala Lys Gly Gln Pro Arg Glu Pro
130 135 140

Gln Val Tyr Thr Leu Pro Pro Ser Gln Glu Glu Met Thr Lys Asn Gln
145 150 155 160

Val Ser Leu Thr Cys Leu Val Lys Gly Phe Tyr Pro Ser Asp Ile Ala
165 170 175

Val Glu Trp Glu Ser Asn Gly Gln Pro Glu Asn Asn Tyr Lys Thr Thr

180 185 190

Pro Pro Val Leu Asp Ser Asp Gly Ser Phe Phe Leu Tyr Ser Arg Leu
195 200 205

Thr Val Asp Lys Ser Arg Trp Gln Glu Gly Asn Val Phe Ser Cys Ser
210 215 220

Val Met His Glu Ala Leu His Asn His Tyr Thr Gln Lys Ser Leu Ser
225 230 235 240

Leu Ser Leu Gly Lys
245

<210> 8
<211> 15
<212> PRT
<213> Artificial Sequence

<220>
<223> Gly-Ser linker

<400> 8
Gly Gly Gly Gly Ser Gly Gly Gly Gly Ser Gly Gly Gly Ser Gly
1 5 10 15

<210> 9
<211> 5
<212> PRT
<213> Artificial Sequence

<220>
<223> PD-L1 linker

<400> 9
Val Lys Val Asn Ala
1 5

<210> 10
<211> 463
<212> PRT
<213> Artificial Sequence

<220>
<223> Amino acid sequence of mouse PD-L1 VC like domain fused mouse IgG2a Fc variant

<400> 10
Phe Thr Ile Thr Ala Pro Lys Asp Leu Tyr Val Val Glu Tyr Gly Ser
1 5 10 15

Asn Val Thr Met Glu Cys Arg Phe Pro Val Glu Arg Glu Leu Asp Leu
20 25 30

Leu Ala Leu Val Val Tyr Trp Glu Lys Glu Asp Glu Gln Val Ile Gln
35 40 45

Phe Val Ala Gly Glu Glu Asp Leu Lys Pro Gln His Ser Asn Phe Arg
50 55 60

Gly Arg Ala Ser Leu Pro Lys Asp Gln Leu Leu Lys Gly Asn Ala Ala
65 70 75 80

Leu Gln Ile Thr Asp Val Lys Leu Gln Asp Ala Gly Val Tyr Cys Cys
85 90 95

Ile Ile Ser Tyr Gly Gly Ala Asp Tyr Lys Arg Ile Thr Leu Lys Val
100 105 110

Asn Ala Pro Tyr Arg Lys Ile Asn Gln Arg Ile Ser Val Asp Pro Ala
115 120 125

Thr Ser Glu His Glu Leu Ile Cys Gln Ala Glu Gly Tyr Pro Glu Ala
130 135 140

Glu Val Ile Trp Thr Asn Ser Asp His Gln Pro Val Ser Gly Lys Arg
145 150 155 160

Ser Val Thr Thr Ser Arg Thr Glu Gly Met Leu Leu Asn Val Thr Ser
165 170 175

Ser Leu Arg Val Asn Ala Thr Ala Asn Asp Val Phe Tyr Cys Thr Phe
180 185 190

Trp Arg Ser Gln Pro Gly Gln Asn His Thr Ala Glu Leu Ile Ile Pro
195 200 205

Glu Leu Pro Ala Thr His Pro Pro Gln Asn Arg Thr Ala Ser Ala Glu
210 215 220

Pro Arg Gly Pro Thr Ile Lys Pro Cys Pro Pro Cys Lys Cys Pro Ala
225 230 235 240

Pro Asn Leu Glu Gly Gly Pro Ser Val Phe Ile Phe Pro Pro Lys Ile
245 250 255

Lys Asp Val Leu Met Ile Ser Leu Ser Pro Ile Val Thr Cys Val Val
260 265 270

Val Asp Val Ser Glu Asp Asp Pro Asp Val Gln Ile Ser Trp Phe Val
275 280 285

Asn Asn Val Glu Val His Thr Ala Gln Thr Gln Thr His Arg Glu Asp
290 295 300

Tyr Asn Ser Thr Leu Arg Val Val Ser Ala Leu Pro Ile Gln His Gln
305 310 315 320

Asp Trp Met Ser Gly Lys Ala Phe Ala Cys Ala Val Asn Asn Lys Asp
325 330 335

Leu Pro Ala Pro Ile Glu Arg Thr Ile Ser Lys Pro Lys Gly Ser Val
340 345 350

Arg Ala Pro Gln Val Tyr Val Leu Pro Pro Pro Glu Glu Glu Met Thr
355 360 365

Lys Lys Gln Val Thr Leu Thr Cys Met Val Thr Asp Phe Met Pro Glu
370 375 380

Asp Ile Tyr Val Glu Trp Thr Asn Asn Gly Lys Thr Glu Leu Asn Tyr
385 390 395 400

Lys Asn Thr Glu Pro Val Leu Asp Ser Asp Gly Ser Tyr Phe Met Tyr
405 410 415

Ser Lys Leu Arg Val Glu Lys Lys Asn Trp Val Glu Arg Asn Ser Tyr
420 425 430

Ser Cys Ser Val Val His Glu Gly Leu His Asn His His Thr Thr Lys

435 440 445

Ser Phe Ser Arg Thr Pro Gly Lys Gly Gly Gly Asn Ser Gly Ser
450 455 460

<210> 11
<211> 358
<212> PRT
<213> Artificial Sequence

<220>
<223> Amino acid sequence of mouse PD-L1 V like domain fused mouse IgG2a Fc variant

<400> 11
Phe Thr Ile Thr Ala Pro Lys Asp Leu Tyr Val Val Glu Tyr Gly Ser
1 5 10 15

Asn Val Thr Met Glu Cys Arg Phe Pro Val Glu Arg Glu Leu Asp Leu
20 25 30

Leu Ala Leu Val Val Tyr Trp Glu Lys Glu Asp Glu Gln Val Ile Gln
35 40 45

Phe Val Ala Gly Glu Glu Asp Leu Lys Pro Gln His Ser Asn Phe Arg
50 55 60

Gly Arg Ala Ser Leu Pro Lys Asp Gln Leu Leu Lys Gly Asn Ala Ala
65 70 75 80

Leu Gln Ile Thr Asp Val Lys Leu Gln Asp Ala Gly Val Tyr Cys Cys
85 90 95

Ile Ile Ser Tyr Gly Gly Ala Asp Tyr Lys Arg Ile Thr Leu Lys Val
100 105 110

Asn Ala Pro Ala Ser Ala Glu Pro Arg Gly Pro Thr Ile Lys Pro Cys
115 120 125

Pro Pro Cys Lys Cys Pro Ala Pro Asn Leu Glu Gly Gly Pro Ser Val
130 135 140

Phe Ile Phe Pro Pro Lys Ile Lys Asp Val Leu Met Ile Ser Leu Ser
145 150 155 160

Pro Ile Val Thr Cys Val Val Val Asp Val Ser Glu Asp Asp Pro Asp
165 170 175

Val Gln Ile Ser Trp Phe Val Asn Asn Val Glu Val His Thr Ala Gln
180 185 190

Thr Gln Thr His Arg Glu Asp Tyr Asn Ser Thr Leu Arg Val Val Ser
195 200 205

Ala Leu Pro Ile Gln His Gln Asp Trp Met Ser Gly Lys Ala Phe Ala
210 215 220

Cys Ala Val Asn Asn Lys Asp Leu Pro Ala Pro Ile Glu Arg Thr Ile
225 230 235 240

Ser Lys Pro Lys Gly Ser Val Arg Ala Pro Gln Val Tyr Val Leu Pro
245 250 255

Pro Pro Glu Glu Glu Met Thr Lys Lys Gln Val Thr Leu Thr Cys Met

260 265 270

Val Thr Asp Phe Met Pro Glu Asp Ile Tyr Val Glu Trp Thr Asn Asn
275 280 285

Gly Lys Thr Glu Leu Asn Tyr Lys Asn Thr Glu Pro Val Leu Asp Ser
290 295 300

Asp Gly Ser Tyr Phe Met Tyr Ser Lys Leu Arg Val Glu Lys Lys Asn
305 310 315 320

Trp Val Glu Arg Asn Ser Tyr Ser Cys Ser Val Val His Glu Gly Leu
325 330 335

His Asn His His Thr Thr Lys Ser Phe Ser Arg Thr Pro Gly Lys Gly
340 345 350

Gly Gly Asn Ser Gly Ser
355

<210> 12
<211> 466
<212> PRT
<213> Artificial Sequence

<220>
<223> Amino acid sequence of human PD-L1 VC like domain(19-239) fused hyFc

<400> 12
Phe Thr Val Thr Val Pro Lys Asp Leu Tyr Val Val Glu Tyr Gly Ser
1 5 10 15

Asn Met Thr Ile Glu Cys Lys Phe Pro Val Glu Lys Gln Leu Asp Leu
20 25 30

Ala Ala Leu Ile Val Tyr Trp Glu Met Glu Asp Lys Asn Ile Ile Gln
35 40 45

Phe Val His Gly Glu Glu Asp Leu Lys Val Gln His Ser Ser Tyr Arg
50 55 60

Gln Arg Ala Arg Leu Leu Lys Asp Gln Leu Ser Leu Gly Asn Ala Ala
65 70 75 80

Leu Gln Ile Thr Asp Val Lys Leu Gln Asp Ala Gly Val Tyr Arg Cys
85 90 95

Met Ile Ser Tyr Gly Gly Ala Asp Tyr Lys Arg Ile Thr Val Lys Val
100 105 110

Asn Ala Pro Tyr Asn Lys Ile Asn Gln Arg Ile Leu Val Val Asp Pro
115 120 125

Val Thr Ser Glu His Glu Leu Thr Cys Gln Ala Glu Gly Tyr Pro Lys
130 135 140

Ala Glu Val Ile Trp Thr Ser Ser Asp His Gln Val Leu Ser Gly Lys
145 150 155 160

Thr Thr Thr Thr Asn Ser Lys Arg Glu Glu Lys Leu Phe Asn Val Thr
165 170 175

Ser Thr Leu Arg Ile Asn Thr Thr Thr Asn Glu Ile Phe Tyr Cys Thr
180 185 190

Phe Arg Arg Leu Asp Pro Glu Glu Asn His Thr Ala Glu Leu Val Ile
195 200 205

Pro Glu Leu Pro Leu Ala His Pro Pro Asn Glu Arg Thr Arg Asn Thr
210 215 220

Gly Arg Gly Gly Glu Glu Lys Lys Lys Glu Lys Glu Lys Glu Glu Gln
225 230 235 240

Glu Glu Arg Glu Thr Lys Thr Pro Glu Cys Pro Ser His Thr Gln Pro
245 250 255

Leu Gly Val Phe Leu Phe Pro Pro Lys Pro Lys Asp Thr Leu Met Ile
260 265 270

Ser Arg Thr Pro Glu Val Thr Cys Val Val Val Asp Val Ser Gln Glu
275 280 285

Asp Pro Glu Val Gln Phe Asn Trp Tyr Val Asp Gly Val Glu Val His
290 295 300

Asn Ala Lys Thr Lys Pro Arg Glu Glu Gln Phe Asn Ser Thr Tyr Arg
305 310 315 320

Val Val Ser Val Leu Thr Val Leu His Gln Asp Trp Leu Asn Gly Lys
325 330 335

Glu Tyr Lys Cys Lys Val Ser Asn Lys Gly Leu Pro Ser Ser Ile Glu
340 345 350

Lys Thr Ile Ser Lys Ala Lys Gly Gln Pro Arg Glu Pro Gln Val Tyr
355 360 365

Thr Leu Pro Pro Ser Gln Glu Glu Met Thr Lys Asn Gln Val Ser Leu
370 375 380

Thr Cys Leu Val Lys Gly Phe Tyr Pro Ser Asp Ile Ala Val Glu Trp
385 390 395 400

Glu Ser Asn Gly Gln Pro Glu Asn Asn Tyr Lys Thr Thr Pro Pro Val
405 410 415

Leu Asp Ser Asp Gly Ser Phe Phe Leu Tyr Ser Arg Leu Thr Val Asp
420 425 430

Lys Ser Arg Trp Gln Glu Gly Asn Val Phe Ser Cys Ser Val Met His
435 440 445

Glu Ala Leu His Asn His Tyr Thr Gln Lys Ser Leu Ser Leu Ser Leu
450 455 460

Gly Lys
465

<210> 13
<211> 464
<212> PRT
<213> Artificial Sequence

<220>
<223> Amino acid sequence of human PD-L1 VC like domain(21-239) fused hyFc

<400> 13
Val Thr Val Pro Lys Asp Leu Tyr Val Val Glu Tyr Gly Ser Asn Met
1 5 10 15

Thr Ile Glu Cys Lys Phe Pro Val Glu Lys Gln Leu Asp Leu Ala Ala
20 25 30

Leu Ile Val Tyr Trp Glu Met Glu Asp Lys Asn Ile Ile Gln Phe Val
35 40 45

His Gly Glu Glu Asp Leu Lys Val Gln His Ser Ser Tyr Arg Gln Arg
50 55 60

Ala Arg Leu Leu Lys Asp Gln Leu Ser Leu Gly Asn Ala Ala Leu Gln
65 70 75 80

Ile Thr Asp Val Lys Leu Gln Asp Ala Gly Val Tyr Arg Cys Met Ile
85 90 95

Ser Tyr Gly Gly Ala Asp Tyr Lys Arg Ile Thr Val Lys Val Asn Ala
100 105 110

Pro Tyr Asn Lys Ile Asn Gln Arg Ile Leu Val Val Asp Pro Val Thr
115 120 125

Ser Glu His Glu Leu Thr Cys Gln Ala Glu Gly Tyr Pro Lys Ala Glu
130 135 140

Val Ile Trp Thr Ser Ser Asp His Gln Val Leu Ser Gly Lys Thr Thr
145 150 155 160

Thr Thr Asn Ser Lys Arg Glu Glu Lys Leu Phe Asn Val Thr Ser Thr
165 170 175

Leu Arg Ile Asn Thr Thr Thr Asn Glu Ile Phe Tyr Cys Thr Phe Arg
180 185 190

Arg Leu Asp Pro Glu Glu Asn His Thr Ala Glu Leu Val Ile Pro Glu
195 200 205

Leu Pro Leu Ala His Pro Pro Asn Glu Arg Thr Arg Asn Thr Gly Arg
210 215 220

Gly Gly Glu Glu Lys Lys Lys Glu Lys Glu Lys Glu Glu Gln Glu Glu
225 230 235 240

Arg Glu Thr Lys Thr Pro Glu Cys Pro Ser His Thr Gln Pro Leu Gly
245 250 255

Val Phe Leu Phe Pro Pro Lys Pro Lys Asp Thr Leu Met Ile Ser Arg
260 265 270

Thr Pro Glu Val Thr Cys Val Val Val Asp Val Ser Gln Glu Asp Pro
275 280 285

Glu Val Gln Phe Asn Trp Tyr Val Asp Gly Val Glu Val His Asn Ala
290 295 300

Lys Thr Lys Pro Arg Glu Glu Gln Phe Asn Ser Thr Tyr Arg Val Val
305 310 315 320

Ser Val Leu Thr Val Leu His Gln Asp Trp Leu Asn Gly Lys Glu Tyr
325 330 335

Lys Cys Lys Val Ser Asn Lys Gly Leu Pro Ser Ser Ile Glu Lys Thr
340 345 350

Ile Ser Lys Ala Lys Gly Gln Pro Arg Glu Pro Gln Val Tyr Thr Leu
355 360 365

Pro Pro Ser Gln Glu Glu Met Thr Lys Asn Gln Val Ser Leu Thr Cys

370 375 380

Leu Val Lys Gly Phe Tyr Pro Ser Asp Ile Ala Val Glu Trp Glu Ser
385 390 395 400

Asn Gly Gln Pro Glu Asn Asn Tyr Lys Thr Thr Pro Pro Val Leu Asp
405 410 415

Ser Asp Gly Ser Phe Phe Leu Tyr Ser Arg Leu Thr Val Asp Lys Ser
420 425 430

Arg Trp Gln Glu Gly Asn Val Phe Ser Cys Ser Val Met His Glu Ala
435 440 445

Leu His Asn His Tyr Thr Gln Lys Ser Leu Ser Leu Ser Leu Gly Lys
450 455 460

<210> 14
<211> 360
<212> PRT
<213> Artificial Sequence

<220>
<223> Amino acid sequence of human PD-L1 V like domain(19-133) fused hyFc

<400> 14
Phe Thr Val Thr Val Pro Lys Asp Leu Tyr Val Val Glu Tyr Gly Ser
1 5 10 15

Asn Met Thr Ile Glu Cys Lys Phe Pro Val Glu Lys Gln Leu Asp Leu
20 25 30

Ala Ala Leu Ile Val Tyr Trp Glu Met Glu Asp Lys Asn Ile Ile Gln
35 40 45

Phe Val His Gly Glu Glu Asp Leu Lys Val Gln His Ser Ser Tyr Arg
50 55 60

Gln Arg Ala Arg Leu Leu Lys Asp Gln Leu Ser Leu Gly Asn Ala Ala
65 70 75 80

Leu Gln Ile Thr Asp Val Lys Leu Gln Asp Ala Gly Val Tyr Arg Cys
85 90 95

Met Ile Ser Tyr Gly Gly Ala Asp Tyr Lys Arg Ile Thr Val Lys Val
100 105 110

Asn Ala Pro Arg Asn Thr Gly Arg Gly Gly Glu Glu Lys Lys Lys Glu
115 120 125

Lys Glu Lys Glu Glu Gln Glu Glu Arg Glu Thr Lys Thr Pro Glu Cys
130 135 140

Pro Ser His Thr Gln Pro Leu Gly Val Phe Leu Phe Pro Pro Lys Pro
145 150 155 160

Lys Asp Thr Leu Met Ile Ser Arg Thr Pro Glu Val Thr Cys Val Val
165 170 175

Val Asp Val Ser Gln Glu Asp Pro Glu Val Gln Phe Asn Trp Tyr Val
180 185 190

Asp Gly Val Glu Val His Asn Ala Lys Thr Lys Pro Arg Glu Glu Gln

195 200 205

Phe Asn Ser Thr Tyr Arg Val Val Ser Val Leu Thr Val Leu His Gln
210 215 220

Asp Trp Leu Asn Gly Lys Glu Tyr Lys Cys Lys Val Ser Asn Lys Gly
225 230 235 240

Leu Pro Ser Ser Ile Glu Lys Thr Ile Ser Lys Ala Lys Gly Gln Pro
245 250 255

Arg Glu Pro Gln Val Tyr Thr Leu Pro Pro Ser Gln Glu Glu Met Thr
260 265 270

Lys Asn Gln Val Ser Leu Thr Cys Leu Val Lys Gly Phe Tyr Pro Ser
275 280 285

Asp Ile Ala Val Glu Trp Glu Ser Asn Gly Gln Pro Glu Asn Asn Tyr
290 295 300

Lys Thr Thr Pro Pro Val Leu Asp Ser Asp Gly Ser Phe Phe Leu Tyr
305 310 315 320

Ser Arg Leu Thr Val Asp Lys Ser Arg Trp Gln Glu Gly Asn Val Phe
325 330 335

Ser Cys Ser Val Met His Glu Ala Leu His Asn His Tyr Thr Gln Lys
340 345 350

Ser Leu Ser Leu Ser Leu Gly Lys
355 360

<210> 15
<211> 354
<212> PRT
<213> Artificial Sequence

<220>
<223> Amino acid sequence of human PD-L1 V like domain(19-127) fused hyFc

<400> 15
Phe Thr Val Thr Val Pro Lys Asp Leu Tyr Val Val Glu Tyr Gly Ser
1 5 10 15

Asn Met Thr Ile Glu Cys Lys Phe Pro Val Glu Lys Gln Leu Asp Leu
20 25 30

Ala Ala Leu Ile Val Tyr Trp Glu Met Glu Asp Lys Asn Ile Ile Gln
35 40 45

Phe Val His Gly Glu Glu Asp Leu Lys Val Gln His Ser Ser Tyr Arg
50 55 60

Gln Arg Ala Arg Leu Leu Lys Asp Gln Leu Ser Leu Gly Asn Ala Ala
65 70 75 80

Leu Gln Ile Thr Asp Val Lys Leu Gln Asp Ala Gly Val Tyr Arg Cys
85 90 95

Met Ile Ser Tyr Gly Gly Ala Asp Tyr Lys Arg Ile Thr Arg Asn Thr
100 105 110

Gly Arg Gly Gly Glu Glu Lys Lys Lys Glu Lys Glu Lys Glu Glu Gln
115 120 125

Glu Glu Arg Glu Thr Lys Thr Pro Glu Cys Pro Ser His Thr Gln Pro
130 135 140

Leu Gly Val Phe Leu Phe Pro Pro Lys Pro Lys Asp Thr Leu Met Ile
145 150 155 160

Ser Arg Thr Pro Glu Val Thr Cys Val Val Val Asp Val Ser Gln Glu
165 170 175

Asp Pro Glu Val Gln Phe Asn Trp Tyr Val Asp Gly Val Glu Val His
180 185 190

Asn Ala Lys Thr Lys Pro Arg Glu Glu Gln Phe Asn Ser Thr Tyr Arg
195 200 205

Val Val Ser Val Leu Thr Val Leu His Gln Asp Trp Leu Asn Gly Lys
210 215 220

Glu Tyr Lys Cys Lys Val Ser Asn Lys Gly Leu Pro Ser Ser Ile Glu
225 230 235 240

Lys Thr Ile Ser Lys Ala Lys Gly Gln Pro Arg Glu Pro Gln Val Tyr
245 250 255

Thr Leu Pro Pro Ser Gln Glu Glu Met Thr Lys Asn Gln Val Ser Leu
260 265 270

Thr Cys Leu Val Lys Gly Phe Tyr Pro Ser Asp Ile Ala Val Glu Trp
275 280 285

Glu Ser Asn Gly Gln Pro Glu Asn Asn Tyr Lys Thr Thr Pro Pro Val
290 295 300

Leu Asp Ser Asp Gly Ser Phe Phe Leu Tyr Ser Arg Leu Thr Val Asp
305 310 315 320

Lys Ser Arg Trp Gln Glu Gly Asn Val Phe Ser Cys Ser Val Met His
325 330 335

Glu Ala Leu His Asn His Tyr Thr Gln Lys Ser Leu Ser Leu Ser Leu
340 345 350

Gly Lys

<210> 16
<211> 347
<212> PRT
<213> Artificial Sequence

<220>
<223> Amino acid sequence of human PD-L1 V like domain(19-120) fused hyFc

<400> 16
Phe Thr Val Thr Val Pro Lys Asp Leu Tyr Val Val Glu Tyr Gly Ser
1 5 10 15

Asn Met Thr Ile Glu Cys Lys Phe Pro Val Glu Lys Gln Leu Asp Leu
20 25 30

Ala Ala Leu Ile Val Tyr Trp Glu Met Glu Asp Lys Asn Ile Ile Gln
35 40 45

Phe Val His Gly Glu Glu Asp Leu Lys Val Gln His Ser Ser Tyr Arg
50 55 60

Gln Arg Ala Arg Leu Leu Lys Asp Gln Leu Ser Leu Gly Asn Ala Ala
65 70 75 80

Leu Gln Ile Thr Asp Val Lys Leu Gln Asp Ala Gly Val Tyr Arg Cys
85 90 95

Met Ile Ser Tyr Gly Gly Arg Asn Thr Gly Arg Gly Gly Glu Glu Lys
100 105 110

Lys Lys Glu Lys Glu Lys Glu Glu Gln Glu Glu Arg Glu Thr Lys Thr
115 120 125

Pro Glu Cys Pro Ser His Thr Gln Pro Leu Gly Val Phe Leu Phe Pro
130 135 140

Pro Lys Pro Lys Asp Thr Leu Met Ile Ser Arg Thr Pro Glu Val Thr
145 150 155 160

Cys Val Val Val Asp Val Ser Gln Glu Asp Pro Glu Val Gln Phe Asn
165 170 175

Trp Tyr Val Asp Gly Val Glu Val His Asn Ala Lys Thr Lys Pro Arg
180 185 190

Glu Glu Gln Phe Asn Ser Thr Tyr Arg Val Val Ser Val Leu Thr Val
195 200 205

Leu His Gln Asp Trp Leu Asn Gly Lys Glu Tyr Lys Cys Lys Val Ser
210 215 220

Asn Lys Gly Leu Pro Ser Ser Ile Glu Lys Thr Ile Ser Lys Ala Lys
225 230 235 240

Gly Gln Pro Arg Glu Pro Gln Val Tyr Thr Leu Pro Pro Ser Gln Glu
245 250 255

Glu Met Thr Lys Asn Gln Val Ser Leu Thr Cys Leu Val Lys Gly Phe
260 265 270

Tyr Pro Ser Asp Ile Ala Val Glu Trp Glu Ser Asn Gly Gln Pro Glu
275 280 285

Asn Asn Tyr Lys Thr Thr Pro Pro Val Leu Asp Ser Asp Gly Ser Phe
290 295 300

Phe Leu Tyr Ser Arg Leu Thr Val Asp Lys Ser Arg Trp Gln Glu Gly
305 310 315 320

Asn Val Phe Ser Cys Ser Val Met His Glu Ala Leu His Asn His Tyr
325 330 335

Thr Gln Lys Ser Leu Ser Leu Ser Leu Gly Lys
340 345

<210> 17
<211> 341
<212> PRT
<213> Artificial Sequence

<220>
<223> Amino acid sequence of human PD-L1 V like domain(19-114) fused hyFc

<400> 17
Phe Thr Val Thr Val Pro Lys Asp Leu Tyr Val Val Glu Tyr Gly Ser

```
1               5                   10                  15
Asn Met Thr Ile Glu Cys Lys Phe Pro Val Glu Lys Gln Leu Asp Leu
            20                  25                  30
Ala Ala Leu Ile Val Tyr Trp Glu Met Glu Asp Lys Asn Ile Ile Gln
        35                  40                  45
Phe Val His Gly Glu Glu Asp Leu Lys Val Gln His Ser Ser Tyr Arg
    50                  55                  60
Gln Arg Ala Arg Leu Leu Lys Asp Gln Leu Ser Leu Gly Asn Ala Ala
65                  70                  75                  80
Leu Gln Ile Thr Asp Val Lys Leu Gln Asp Ala Gly Val Tyr Arg Cys
                85                  90                  95
Arg Asn Thr Gly Arg Gly Gly Glu Glu Lys Lys Lys Glu Lys Glu Lys
            100                 105                 110
Glu Glu Gln Glu Glu Arg Glu Thr Lys Thr Pro Glu Cys Pro Ser His
        115                 120                 125
Thr Gln Pro Leu Gly Val Phe Leu Phe Pro Pro Lys Pro Lys Asp Thr
    130                 135                 140
Leu Met Ile Ser Arg Thr Pro Glu Val Thr Cys Val Val Val Asp Val
145                 150                 155                 160
Ser Gln Glu Asp Pro Glu Val Gln Phe Asn Trp Tyr Val Asp Gly Val
                165                 170                 175
Glu Val His Asn Ala Lys Thr Lys Pro Arg Glu Glu Gln Phe Asn Ser
            180                 185                 190
Thr Tyr Arg Val Val Ser Val Leu Thr Val Leu His Gln Asp Trp Leu
        195                 200                 205
Asn Gly Lys Glu Tyr Lys Cys Lys Val Ser Asn Lys Gly Leu Pro Ser
    210                 215                 220
Ser Ile Glu Lys Thr Ile Ser Lys Ala Lys Gly Gln Pro Arg Glu Pro
225                 230                 235                 240
Gln Val Tyr Thr Leu Pro Pro Ser Gln Glu Glu Met Thr Lys Asn Gln
                245                 250                 255
Val Ser Leu Thr Cys Leu Val Lys Gly Phe Tyr Pro Ser Asp Ile Ala
            260                 265                 270
Val Glu Trp Glu Ser Asn Gly Gln Pro Glu Asn Asn Tyr Lys Thr Thr
        275                 280                 285
Pro Pro Val Leu Asp Ser Asp Gly Ser Phe Phe Leu Tyr Ser Arg Leu
    290                 295                 300
Thr Val Asp Lys Ser Arg Trp Gln Glu Gly Asn Val Phe Ser Cys Ser
305                 310                 315                 320
Val Met His Glu Ala Leu His Asn His Tyr Thr Gln Lys Ser Leu Ser
                325                 330                 335
Leu Ser Leu Gly Lys
            340
```

<210> 18
<211> 466

<212> PRT
<213> Artificial Sequence

<220>
<223> Amino acid sequence of human PD-L1 VC like domain(19-239) fused hyFcM1

<400> 18

Phe Thr Val Thr Val Pro Lys Asp Leu Tyr Val Val Glu Tyr Gly Ser
1               5                   10                  15

Asn Met Thr Ile Glu Cys Lys Phe Pro Val Glu Lys Gln Leu Asp Leu
            20                  25                  30

Ala Ala Leu Ile Val Tyr Trp Glu Met Glu Asp Lys Asn Ile Ile Gln
        35                  40                  45

Phe Val His Gly Glu Glu Asp Leu Lys Val Gln His Ser Ser Tyr Arg
    50                  55                  60

Gln Arg Ala Arg Leu Leu Lys Asp Gln Leu Ser Leu Gly Asn Ala Ala
65                  70                  75                  80

Leu Gln Ile Thr Asp Val Lys Leu Gln Asp Ala Gly Val Tyr Arg Cys
                85                  90                  95

Met Ile Ser Tyr Gly Gly Ala Asp Tyr Lys Arg Ile Thr Val Lys Val
            100                 105                 110

Asn Ala Pro Tyr Asn Lys Ile Asn Gln Arg Ile Leu Val Val Asp Pro
        115                 120                 125

Val Thr Ser Glu His Glu Leu Thr Cys Gln Ala Glu Gly Tyr Pro Lys
    130                 135                 140

Ala Glu Val Ile Trp Thr Ser Ser Asp His Gln Val Leu Ser Gly Lys
145                 150                 155                 160

Thr Thr Thr Thr Asn Ser Lys Arg Glu Glu Lys Leu Phe Asn Val Thr
                165                 170                 175

Ser Thr Leu Arg Ile Asn Thr Thr Thr Asn Glu Ile Phe Tyr Cys Thr
            180                 185                 190

Phe Arg Arg Leu Asp Pro Glu Glu Asn His Thr Ala Glu Leu Val Ile
        195                 200                 205

Pro Glu Leu Pro Leu Ala His Pro Pro Asn Glu Arg Thr Arg Asn Thr
    210                 215                 220

Gly Arg Gly Gly Glu Glu Lys Lys Gly Gly Lys Glu Lys Glu Glu Gln
225                 230                 235                 240

Glu Glu Arg Glu Thr Lys Thr Pro Glu Cys Pro Ser His Thr Gln Pro
                245                 250                 255

Leu Gly Val Phe Leu Phe Pro Pro Lys Pro Lys Asp Thr Leu Met Ile
            260                 265                 270

Ser Arg Thr Pro Glu Val Thr Cys Val Val Val Asp Val Ser Gln Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Tyr Arg
305                 310                 315                 320

Val Val Ser Val Leu Thr Val Leu His Gln Asp Trp Leu Asn Gly Lys
325 330 335

Glu Tyr Lys Cys Lys Val Ser Asn Lys Gly Leu Pro Ser Ser Ile Glu
340 345 350

Lys Thr Ile Ser Lys Ala Lys Gly Gln Pro Arg Glu Pro Gln Val Tyr
355 360 365

Thr Leu Pro Pro Ser Gln Glu Glu Met Thr Lys Asn Gln Val Ser Leu
370 375 380

Thr Cys Leu Val Lys Gly Phe Tyr Pro Ser Asp Ile Ala Val Glu Trp
385 390 395 400

Glu Ser Asn Gly Gln Pro Glu Asn Asn Tyr Lys Thr Thr Pro Pro Val
405 410 415

Leu Asp Ser Asp Gly Ser Phe Phe Leu Tyr Ser Arg Leu Thr Val Asp
420 425 430

Lys Ser Arg Trp Gln Glu Gly Asn Val Phe Ser Cys Ser Val Met His
435 440 445

Glu Ala Leu His Asn His Tyr Thr Gln Lys Ser Leu Ser Leu Ser Leu
450 455 460

Gly Lys
465

<210> 19
<211> 464
<212> PRT
<213> Artificial Sequence

<220>
<223> Amino acid sequence of human PD-L1 VC like domain (21-239) fused hyFcM1

<400> 19
Val Thr Val Pro Lys Asp Leu Tyr Val Val Glu Tyr Gly Ser Asn Met
1 5 10 15

Thr Ile Glu Cys Lys Phe Pro Val Glu Lys Gln Leu Asp Leu Ala Ala
20 25 30

Leu Ile Val Tyr Trp Glu Met Glu Asp Lys Asn Ile Ile Gln Phe Val
35 40 45

His Gly Glu Glu Asp Leu Lys Val Gln His Ser Ser Tyr Arg Gln Arg
50 55 60

Ala Arg Leu Leu Lys Asp Gln Leu Ser Leu Gly Asn Ala Ala Leu Gln
65 70 75 80

Ile Thr Asp Val Lys Leu Gln Asp Ala Gly Val Tyr Arg Cys Met Ile
85 90 95

Ser Tyr Gly Gly Ala Asp Tyr Lys Arg Ile Thr Val Lys Val Asn Ala
100 105 110

Pro Tyr Asn Lys Ile Asn Gln Arg Ile Leu Val Val Asp Pro Val Thr
115 120 125

Ser Glu His Glu Leu Thr Cys Gln Ala Glu Gly Tyr Pro Lys Ala Glu

130 135 140

Val Ile Trp Thr Ser Ser Asp His Gln Val Leu Ser Gly Lys Thr Thr
145 150 155 160

Thr Thr Asn Ser Lys Arg Glu Glu Lys Leu Phe Asn Val Thr Ser Thr
165 170 175

Leu Arg Ile Asn Thr Thr Thr Asn Glu Ile Phe Tyr Cys Thr Phe Arg
180 185 190

Arg Leu Asp Pro Glu Glu Asn His Thr Ala Glu Leu Val Ile Pro Glu
195 200 205

Leu Pro Leu Ala His Pro Pro Asn Glu Arg Thr Arg Asn Thr Gly Arg
210 215 220

Gly Gly Glu Glu Lys Lys Gly Gly Lys Glu Lys Glu Glu Gln Glu Glu
225 230 235 240

Arg Glu Thr Lys Thr Pro Glu Cys Pro Ser His Thr Gln Pro Leu Gly
245 250 255

Val Phe Leu Phe Pro Pro Lys Pro Lys Asp Thr Leu Met Ile Ser Arg
260 265 270

Thr Pro Glu Val Thr Cys Val Val Val Asp Val Ser Gln Glu Asp Pro
275 280 285

Glu Val Gln Phe Asn Trp Tyr Val Asp Gly Val Glu Val His Asn Ala
290 295 300

Lys Thr Lys Pro Arg Glu Glu Gln Phe Asn Ser Thr Tyr Arg Val Val
305 310 315 320

Ser Val Leu Thr Val Leu His Gln Asp Trp Leu Asn Gly Lys Glu Tyr
325 330 335

Lys Cys Lys Val Ser Asn Lys Gly Leu Pro Ser Ser Ile Glu Lys Thr
340 345 350

Ile Ser Lys Ala Lys Gly Gln Pro Arg Glu Pro Gln Val Tyr Thr Leu
355 360 365

Pro Pro Ser Gln Glu Glu Met Thr Lys Asn Gln Val Ser Leu Thr Cys
370 375 380

Leu Val Lys Gly Phe Tyr Pro Ser Asp Ile Ala Val Glu Trp Glu Ser
385 390 395 400

Asn Gly Gln Pro Glu Asn Asn Tyr Lys Thr Thr Pro Pro Val Leu Asp
405 410 415

Ser Asp Gly Ser Phe Phe Leu Tyr Ser Arg Leu Thr Val Asp Lys Ser
420 425 430

Arg Trp Gln Glu Gly Asn Val Phe Ser Cys Ser Val Met His Glu Ala
435 440 445

Leu His Asn His Tyr Thr Gln Lys Ser Leu Ser Leu Ser Leu Gly Lys
450 455 460

<210> 20
<211> 360
<212> PRT

<213> Artificial Sequence

<220>
<223> Amino acid sequence of human PD-L1 V like domain(19-133) fused hyFcM1

<400> 20

```
Phe Thr Val Thr Val Pro Lys Asp Leu Tyr Val Val Glu Tyr Gly Ser
1               5                   10                  15

Asn Met Thr Ile Glu Cys Lys Phe Pro Val Glu Lys Gln Leu Asp Leu
            20                  25                  30

Ala Ala Leu Ile Val Tyr Trp Glu Met Glu Asp Lys Asn Ile Ile Gln
        35                  40                  45

Phe Val His Gly Glu Glu Asp Leu Lys Val Gln His Ser Ser Tyr Arg
    50                  55                  60

Gln Arg Ala Arg Leu Leu Lys Asp Gln Leu Ser Leu Gly Asn Ala Ala
65                  70                  75                  80

Leu Gln Ile Thr Asp Val Lys Leu Gln Asp Ala Gly Val Tyr Arg Cys
                85                  90                  95

Met Ile Ser Tyr Gly Gly Ala Asp Tyr Lys Arg Ile Thr Val Lys Val
            100                 105                 110

Asn Ala Pro Arg Asn Thr Gly Arg Gly Gly Glu Glu Lys Lys Gly Gly
        115                 120                 125

Lys Glu Lys Glu Glu Gln Glu Glu Arg Glu Thr Lys Thr Pro Glu Cys
    130                 135                 140

Pro Ser His Thr Gln Pro Leu Gly Val Phe Leu Phe Pro Pro Lys Pro
145                 150                 155                 160

Lys Asp Thr Leu Met Ile Ser Arg Thr Pro Glu Val Thr Cys Val Val
                165                 170                 175

Val Asp Val Ser Gln Glu Asp Pro Glu Val Gln Phe Asn Trp Tyr Val
            180                 185                 190

Asp Gly Val Glu Val His Asn Ala Lys Thr Lys Pro Arg Glu Glu Gln
        195                 200                 205

Phe Asn Ser Thr Tyr Arg Val Val Ser Val Leu Thr Val Leu His Gln
    210                 215                 220

Asp Trp Leu Asn Gly Lys Glu Tyr Lys Cys Lys Val Ser Asn Lys Gly
225                 230                 235                 240

Leu Pro Ser Ser Ile Glu Lys Thr Ile Ser Lys Ala Lys Gly Gln Pro
                245                 250                 255

Arg Glu Pro Gln Val Tyr Thr Leu Pro Pro Ser Gln Glu Glu Met Thr
            260                 265                 270

Lys Asn Gln Val Ser Leu Thr Cys Leu Val Lys Gly Phe Tyr Pro Ser
        275                 280                 285

Asp Ile Ala Val Glu Trp Glu Ser Asn Gly Gln Pro Glu Asn Asn Tyr
    290                 295                 300

Lys Thr Thr Pro Pro Val Leu Asp Ser Asp Gly Ser Phe Phe Leu Tyr
305                 310                 315                 320
```

Ser Arg Leu Thr Val Asp Lys Ser Arg Trp Gln Glu Gly Asn Val Phe
325 330 335

Ser Cys Ser Val Met His Glu Ala Leu His Asn His Tyr Thr Gln Lys
340 345 350

Ser Leu Ser Leu Ser Leu Gly Lys
355 360

<210> 21
<211> 354
<212> PRT
<213> Artificial Sequence

<220>
<223> Amino acid sequence of human PD-L1 V like domain(19-127) fused hyFcM1

<400> 21
Phe Thr Val Thr Val Pro Lys Asp Leu Tyr Val Val Glu Tyr Gly Ser
1 5 10 15

Asn Met Thr Ile Glu Cys Lys Phe Pro Val Glu Lys Gln Leu Asp Leu
20 25 30

Ala Ala Leu Ile Val Tyr Trp Glu Met Glu Asp Lys Asn Ile Ile Gln
35 40 45

Phe Val His Gly Glu Glu Asp Leu Lys Val Gln His Ser Ser Tyr Arg
50 55 60

Gln Arg Ala Arg Leu Leu Lys Asp Gln Leu Ser Leu Gly Asn Ala Ala
65 70 75 80

Leu Gln Ile Thr Asp Val Lys Leu Gln Asp Ala Gly Val Tyr Arg Cys
85 90 95

Met Ile Ser Tyr Gly Gly Ala Asp Tyr Lys Arg Ile Thr Arg Asn Thr
100 105 110

Gly Arg Gly Gly Glu Glu Lys Lys Gly Gly Lys Glu Lys Glu Glu Gln
115 120 125

Glu Glu Arg Glu Thr Lys Thr Pro Glu Cys Pro Ser His Thr Gln Pro
130 135 140

Leu Gly Val Phe Leu Phe Pro Pro Lys Pro Lys Asp Thr Leu Met Ile
145 150 155 160

Ser Arg Thr Pro Glu Val Thr Cys Val Val Val Asp Val Ser Gln Glu
165 170 175

Asp Pro Glu Val Gln Phe Asn Trp Tyr Val Asp Gly Val Glu Val His
180 185 190

Asn Ala Lys Thr Lys Pro Arg Glu Glu Gln Phe Asn Ser Thr Tyr Arg
195 200 205

Val Val Ser Val Leu Thr Val Leu His Gln Asp Trp Leu Asn Gly Lys
210 215 220

Glu Tyr Lys Cys Lys Val Ser Asn Lys Gly Leu Pro Ser Ser Ile Glu
225 230 235 240

Lys Thr Ile Ser Lys Ala Lys Gly Gln Pro Arg Glu Pro Gln Val Tyr
245 250 255

Thr Leu Pro Pro Ser Gln Glu Glu Met Thr Lys Asn Gln Val Ser Leu
260 265 270

Thr Cys Leu Val Lys Gly Phe Tyr Pro Ser Asp Ile Ala Val Glu Trp
275 280 285

Glu Ser Asn Gly Gln Pro Glu Asn Asn Tyr Lys Thr Thr Pro Pro Val
290 295 300

Leu Asp Ser Asp Gly Ser Phe Phe Leu Tyr Ser Arg Leu Thr Val Asp
305 310 315 320

Lys Ser Arg Trp Gln Glu Gly Asn Val Phe Ser Cys Ser Val Met His
325 330 335

Glu Ala Leu His Asn His Tyr Thr Gln Lys Ser Leu Ser Leu Ser Leu
340 345 350

Gly Lys

<210> 22
<211> 347
<212> PRT
<213> Artificial Sequence

<220>
<223> Amino acid sequence of human PD-L1 V like domain(19-120) fused hyFcM1

<400> 22
Phe Thr Val Thr Val Pro Lys Asp Leu Tyr Val Val Glu Tyr Gly Ser
1 5 10 15

Asn Met Thr Ile Glu Cys Lys Phe Pro Val Glu Lys Gln Leu Asp Leu
20 25 30

Ala Ala Leu Ile Val Tyr Trp Glu Met Glu Asp Lys Asn Ile Ile Gln
35 40 45

Phe Val His Gly Glu Glu Asp Leu Lys Val Gln His Ser Ser Tyr Arg
50 55 60

Gln Arg Ala Arg Leu Leu Lys Asp Gln Leu Ser Leu Gly Asn Ala Ala
65 70 75 80

Leu Gln Ile Thr Asp Val Lys Leu Gln Asp Ala Gly Val Tyr Arg Cys
85 90 95

Met Ile Ser Tyr Gly Gly Arg Asn Thr Gly Arg Gly Gly Glu Glu Lys
100 105 110

Lys Gly Gly Lys Glu Lys Glu Glu Gln Glu Glu Arg Glu Thr Lys Thr
115 120 125

Pro Glu Cys Pro Ser His Thr Gln Pro Leu Gly Val Phe Leu Phe Pro
130 135 140

Pro Lys Pro Lys Asp Thr Leu Met Ile Ser Arg Thr Pro Glu Val Thr
145 150 155 160

Cys Val Val Val Asp Val Ser Gln Glu Asp Pro Glu Val Gln Phe Asn
165 170 175

Trp Tyr Val Asp Gly Val Glu Val His Asn Ala Lys Thr Lys Pro Arg

180 185 190

Glu Glu Gln Phe Asn Ser Thr Tyr Arg Val Val Ser Val Leu Thr Val
195 200 205

Leu His Gln Asp Trp Leu Asn Gly Lys Glu Tyr Lys Cys Lys Val Ser
210 215 220

Asn Lys Gly Leu Pro Ser Ser Ile Glu Lys Thr Ile Ser Lys Ala Lys
225 230 235 240

Gly Gln Pro Arg Glu Pro Gln Val Tyr Thr Leu Pro Pro Ser Gln Glu
245 250 255

Glu Met Thr Lys Asn Gln Val Ser Leu Thr Cys Leu Val Lys Gly Phe
260 265 270

Tyr Pro Ser Asp Ile Ala Val Glu Trp Glu Ser Asn Gly Gln Pro Glu
275 280 285

Asn Asn Tyr Lys Thr Thr Pro Pro Val Leu Asp Ser Asp Gly Ser Phe
290 295 300

Phe Leu Tyr Ser Arg Leu Thr Val Asp Lys Ser Arg Trp Gln Glu Gly
305 310 315 320

Asn Val Phe Ser Cys Ser Val Met His Glu Ala Leu His Asn His Tyr
325 330 335

Thr Gln Lys Ser Leu Ser Leu Ser Leu Gly Lys
340 345

<210> 23
<211> 341
<212> PRT
<213> Artificial Sequence

<220>
<223> Amino acid sequence of human PD-L1 V like domain(19~114) fused hyFcM1

<400> 23
Phe Thr Val Thr Val Pro Lys Asp Leu Tyr Val Val Glu Tyr Gly Ser
1 5 10 15

Asn Met Thr Ile Glu Cys Lys Phe Pro Val Glu Lys Gln Leu Asp Leu
20 25 30

Ala Ala Leu Ile Val Tyr Trp Glu Met Glu Asp Lys Asn Ile Ile Gln
35 40 45

Phe Val His Gly Glu Glu Asp Leu Lys Val Gln His Ser Ser Tyr Arg
50 55 60

Gln Arg Ala Arg Leu Leu Lys Asp Gln Leu Ser Leu Gly Asn Ala Ala
65 70 75 80

Leu Gln Ile Thr Asp Val Lys Leu Gln Asp Ala Gly Val Tyr Arg Cys
85 90 95

Arg Asn Thr Gly Arg Gly Gly Glu Glu Lys Lys Gly Gly Lys Glu Lys
100 105 110

Glu Glu Gln Glu Glu Arg Glu Thr Lys Thr Pro Glu Cys Pro Ser His
115 120 125

Thr Gln Pro Leu Gly Val Phe Leu Phe Pro Pro Lys Pro Lys Asp Thr
130 135 140

Leu Met Ile Ser Arg Thr Pro Glu Val Thr Cys Val Val Val Asp Val
145 150 155 160

Ser Gln Glu Asp Pro Glu Val Gln Phe Asn Trp Tyr Val Asp Gly Val
165 170 175

Glu Val His Asn Ala Lys Thr Lys Pro Arg Glu Glu Gln Phe Asn Ser
180 185 190

Thr Tyr Arg Val Val Ser Val Leu Thr Val Leu His Gln Asp Trp Leu
195 200 205

Asn Gly Lys Glu Tyr Lys Cys Lys Val Ser Asn Lys Gly Leu Pro Ser
210 215 220

Ser Ile Glu Lys Thr Ile Ser Lys Ala Lys Gly Gln Pro Arg Glu Pro
225 230 235 240

Gln Val Tyr Thr Leu Pro Pro Ser Gln Glu Glu Met Thr Lys Asn Gln
245 250 255

Val Ser Leu Thr Cys Leu Val Lys Gly Phe Tyr Pro Ser Asp Ile Ala
260 265 270

Val Glu Trp Glu Ser Asn Gly Gln Pro Glu Asn Asn Tyr Lys Thr Thr
275 280 285

Pro Pro Val Leu Asp Ser Asp Gly Ser Phe Phe Leu Tyr Ser Arg Leu
290 295 300

Thr Val Asp Lys Ser Arg Trp Gln Glu Gly Asn Val Phe Ser Cys Ser
305 310 315 320

Val Met His Glu Ala Leu His Asn His Tyr Thr Gln Lys Ser Leu Ser
325 330 335

Leu Ser Leu Gly Lys
340

<210> 24
<211> 1392
<212> DNA
<213> Artificial Sequence

<220>
<223> nucleotide sequence of mouse PD-L1 VC like domain fused mouse IgG2a Fc variant

<400> 24
ttcactatta ccgctcctaa ggatctgtac gtggtggaat atggaagtaa cgtgactatg 60

gagtgccggt ttccagtgga gcgcgaactg gacctgctgg ccctggtggt gtactgggag 120

aaggaagatg agcaggtcat ccagttcgtg gccggggagg aagacctgaa acctcagcat 180

agtaacttta ggggaagagc cagcctgcca aaggaccagc tgctgaaagg gaatgccgct 240

ctgcagatca cagacgtgaa gctgcaggat gccggcgtgt actgctgtat cattagctac 300

ggcggagccg attataagag gattacactg aaagtgaacg ctccctatag gaaaatcaat 360

cagagaattt ccgtggaccc tgctacttct gaacacgagc tgatctgcca ggccgaggga 420

tacccagaag ctgaagtgat ctggaccaac tccgatcatc agcccgtgtc tgggaagcgg 480

agtgtgacca caagccgcac cgagggcatg ctgctgaatg tgacaagctc cctgcgggtg 540

aacgccactg ctaatgacgt gttctattgt accttttggc gctctcagcc aggacagaac 600

cacaccgccg aactgatcat tccagagctg cccgctacac atccccctca gaatagaact 660

gctagcgctg agcccagagg ccctaccatc aagccctgcc ccccctgcaa gtgccctgcc 720

cctaacctgg agggcggccc ttccgtgttc atcttccctc ccaagatcaa ggacgtgctg 780

atgatctccc tgtcccctat cgtgacctgc gtggtggtgg acgtgtccga ggacgaccca 840

gacgtgcaga tcagctggtt cgtgaacaac gtggaggtgc acaccgccca gacccagacc 900

cacagagagg actacaactc caccctgagg gtggtgagcg ccctgcctat ccagcaccag 960

gactggatga gcggcaaggc cttcgcctgt gccgtgaaca acaaggacct gcctgccccc 1020

atcgagagaa ccatcagcaa gcccaagggc agcgtgaggg ccccacaggt gtacgtgctg 1080

cctccccccg aggaggagat gaccaagaag caggtgaccc tgacctgcat ggtgaccgac 1140

ttcatgcccg aggacatcta cgtggagtgg accaacaacg gcaagaccga gctgaactac 1200

aagaacaccg agcctgtgct ggacagcgac ggctcctact tcatgtactc caagctgaga 1260

gtggagaaga agaactgggt ggagagaaac tcctacagct gcagcgtggt gcacgagggc 1320

ctgcacaacc accacaccac caagagcttc tccagaaccc ccggcaaggg cggcggcaac 1380

tccggatcct aa 1392

<210> 25
<211> 1077
<212> DNA
<213> Artificial Sequence

<220>
<223> nucleotide sequence of mouse PD-L1 V like domain fused mouse IgG2a Fc variant

<400> 25
ttcactatta ccgctcctaa ggatctgtac gtggtggaat atggaagtaa cgtgactatg 60

gagtgccggt ttccagtgga gcgcgaactg gacctgctgg ccctggtggt gtactgggag 120

aaggaagatg agcaggtcat ccagttcgtg gccggggagg aagacctgaa acctcagcat 180

agtaacttta ggggaagagc cagcctgcca aaggaccagc tgctgaaagg gaatgccgct 240

ctgcagatca cagacgtgaa gctgcaggat gccggcgtgt actgctgtat cattagctac 300

ggcggagccg attataagag gattacactg aaagtgaacg ctcccgctag cgctgagccc 360

agaggcccta ccatcaagcc ctgccccccc tgcaagtgcc ctgcccctaa cctggagggc 420

ggcccttccg tgttcatctt ccctcccaag atcaaggacg tgctgatgat ctccctgtcc 480

cctatcgtga cctgcgtggt ggtggacgtg tccgaggacg acccagacgt gcagatcagc 540

tggttcgtga acaacgtgga ggtgcacacc gcccagaccc agacccacag agaggactac 600

aactccaccc tgagggtggt gagcgccctg cctatccagc accaggactg gatgagcggc 660

aaggccttcg cctgtgccgt gaacaacaag gacctgcctg cccccatcga gagaaccatc 720

agcaagccca agggcagcgt gagggcccca caggtgtacg tgctgcctcc ccccgaggag 780

gagatgacca agaagcaggt gaccctgacc tgcatggtga ccgacttcat gcccgaggac 840

atctacgtgg agtggaccaa caacggcaag accgagctga actacaagaa caccgagcct 900

gtgctggaca gcgacggctc ctacttcatg tactccaagc tgagagtgga gaagaagaac 960

tgggtggaga gaaactccta cagctgcagc gtggtgcacg agggcctgca caaccaccac 1020

accaccaaga gcttctccag aacccccggc aagggcggcg gcaactccgg atcctaa 1077

<210> 26
<211> 1398
<212> DNA
<213> Artificial Sequence

<220>
<223> nucleotide sequence of human PD-L1 VC like domain(19-239) fused hyFc

<400> 26
ttcaccgtga ccgtgcccaa ggacctgtac gtggtggagt acggcagcaa catgaccatc 60

gagtgcaagt tccccgtgga gaagcagctg gacctggccg ccctgatcgt gtactgggag 120

atggaggaca agaacatcat ccagttcgtg cacggcgagg aggacctgaa ggtgcagcac 180

agcagctaca gacagagagc cagactgctg aaggaccagc tgagcctggg caacgccgcc 240

ctgcagatca ccgacgtgaa gctgcaggac gccggcgtgt acagatgcat gatcagctac 300

ggcggcgccg actacaagag aatcaccgtg aaggtgaacg cccccctacaa caagatcaac 360

cagagaatcc tggtggtgga ccccgtgacc agcgagcacg agctgacctg ccaggccgag 420

ggctacccca aggccgaggt gatctggacc agcagcgacc accaggtgct gagcggcaag 480

accaccacca ccaacagcaa gagagaggag aagctgttca acgtgaccag caccctgaga 540

atcaacacca caaccaacga gatcttctac tgcaccttca gaagactgga ccccgaggag 600

aaccacaccg ccgagctggt gatccccgag ctgcccctgg cccacccccc caacgagaga 660

acccgcaaca ccggccgcgg cggcgaggag aagaagaagg agaaggagaa ggaggagcag 720

gaggagcgcg agaccaagac ccccgagtgc cccagccaca cccagcccct gggcgtgttc 780

ctgttccccc ccaagcccaa ggacaccctg atgatcagcc gcacccccga ggtgacctgc 840

gtggtcgtgg atgtgagcca ggaagatccc gaagtgcagt tcaactggta cgtggatggc 900

gtggaagtgc acaacgccaa gaccaagccc agagaagagc agttcaactc cacctacaga 960

gtggtgagcg tgctgaccgt gctgcaccag gactggctga acggcaagga gtacaagtgc 1020

aaggtgtcca acaaaggcct gcccagctcc atcgagaaga ccatcagcaa agccaaaggc 1080

cagcccagag aaccccaggt gtacaccctg cctcccagcc aggaagagat gaccaagaac 1140

caggtgtccc tgacctgcct ggtgaaaggc ttctacccca gcgacatcgc cgtggagtgg 1200

gaaagcaacg gccagcccga gaacaattac aagacaaccc ctcccgtgct ggatagcgat 1260

ggcagcttct ttctgtacag cagactgacc gtggacaaga gcagatggca ggaaggcaac 1320

gtgttcagct gcagcgtgat gcacgaagcc ctgcacaacc actacaccca gaagagcctg 1380

tccctgagcc tgggcaag 1398

<210> 27
<211> 1392
<212> DNA
<213> Artificial Sequence

<220>
<223> nucleotide sequence of human PD-L1 VC like domain(21-239) fused hyFc

<400> 27
gtgactgtcc caaaagatct gtacgtggtc gagtatggct ccaacatgac catcgagtgc 60

aaattccctg tggaaaagca gctggacctg gccgctctga tcgtgtactg ggagatggaa 120

gataagaaca tcatccagtt cgtgcacgga gaggaagacc tgaaggtcca gcattccagc 180

tatagacagc gcgctcgact gctgaaagac cagctgagcc tgggcaacgc agccctgcag 240

atcactgacg tgaagctgca ggatgcagga gtctaccggt gcatgatctc ttacggcgga 300

gcagattata aaagaattac cgtgaaggtc aatgcccccct ataacaagat caatcagagg 360

attctggtgg tcgaccctgt gacaagtgag cacgaactga cttgtcaggc tgaggggtac 420

ccaaaagcag aagtgatctg gacatctagt gatcatcagg tcctgtcagg caagaccaca 480

actaccaact ccaaacgcga ggaaaagctg ttcaatgtga caagcactct gcgaatcaac 540

acaactacca atgagatttt ctattgcacc tttaggcggc tggaccccga ggaaaaccac 600

acagccgagc tggtcatccc agaactgccc ctggctcatc cccctaacga gcgtactcgc 660

aacaccggcc gcggcggcga ggagaagaag aaggagaagg agaaggagga gcaggaggag 720

cgcgagacca agacccccga gtgccccagc cacacccagc ccctgggcgt gttcctgttc 780

ccccccaagc ccaaggacac cctgatgatc agccgcaccc ccgaggtgac ctgcgtggtc 840

gtggatgtga gccaggaaga tcccgaagtg cagttcaact ggtacgtgga tggcgtggaa 900

gtgcacaacg ccaagaccaa gcccagagaa gagcagttca actccaccta cagagtggtg 960

agcgtgctga ccgtgctgca ccaggactgg ctgaacggca aggagtacaa gtgcaaggtg 1020

tccaacaaag gcctgcccag ctccatcgag aagaccatca gcaaagccaa aggccagccc 1080

agagaacccc aggtgtacac cctgcctccc agccaggaag agatgaccaa gaaccaggtg 1140

tccctgacct gcctggtgaa aggcttctac cccagcgaca tcgccgtgga gtgggaaagc 1200

aacggccagc ccgagaacaa ttacaagaca acccctcccg tgctggatag cgatggcagc 1260

ttctttctgt acagcagact gaccgtggac aagagcagat ggcaggaagg caacgtgttc 1320

agctgcagcg tgatgcacga agccctgcac aaccactaca cccagaagag cctgtccctg 1380

agcctgggca ag 1392

<210> 28

<211> 1080
<212> DNA
<213> Artificial Sequence

<220>
<223> nucleotide sequence of human PD-L1 V like domain(19-133) fused hyFc

<400> 28
ttcaccgtga ccgtgcccaa ggacctgtac gtggtggagt acggcagcaa catgaccatc 60

gagtgcaagt tccccgtgga gaagcagctg gacctggccg ccctgatcgt gtactgggag 120

atggaggaca agaacatcat ccagttcgtg cacggcgagg aggacctgaa ggtgcagcac 180

agcagctaca gacagagagc cagactgctg aaggaccagc tgagcctggg caacgccgcc 240

ctgcagatca ccgacgtgaa gctgcaggac gccggcgtgt acagatgcat gatcagctac 300

ggcggcgccg actacaagag aatcaccgtg aaggtgaacg ccccccgcaa caccggccgc 360

ggcggcgagg agaagaagaa ggagaaggag aaggaggagc aggaggagcg cgagaccaag 420

acccccgagt gccccagcca cacccagccc ctgggcgtgt tcctgttccc ccccaagccc 480

aaggacaccc tgatgatcag ccgcaccccc gaggtgacct gcgtggtcgt ggatgtgagc 540

caggaagatc ccgaagtgca gttcaactgg tacgtggatg gcgtggaagt gcacaacgcc 600

aagaccaagc ccagagaaga gcagttcaac tccacctaca gagtggtgag cgtgctgacc 660

gtgctgcacc aggactggct gaacggcaag gagtacaagt gcaaggtgtc caacaaaggc 720

ctgcccagct ccatcgagaa gaccatcagc aaagccaaag gccagcccag agaaccccag 780

gtgtacaccc tgcctcccag ccaggaagag atgaccaaga accaggtgtc cctgacctgc 840

ctggtgaaag gcttctaccc cagcgacatc gccgtggagt gggaaagcaa cggccagccc 900

gagaacaatt acaagacaac ccctcccgtg ctggatagcg atggcagctt ctttctgtac 960

agcagactga ccgtggacaa gagcagatgg caggaaggca acgtgttcag ctgcagcgtg 1020

atgcacgaag ccctgcacaa ccactacacc cagaagagcc tgtccctgag cctgggcaag 1080

1080

<210> 29
<211> 1062
<212> DNA
<213> Artificial Sequence

<220>
<223> nucleotide sequence of human PD-L1 V like domain(19-127) fused hyFc

<400> 29
ttcaccgtga ccgtgcccaa ggacctgtac gtggtggagt acggcagcaa catgaccatc 60

gagtgcaagt tccccgtgga gaagcagctg gacctggccg ccctgatcgt gtactgggag 120

atggaggaca agaacatcat ccagttcgtg cacggcgagg aggacctgaa ggtgcagcac 180

agcagctaca gacagagagc cagactgctg aaggaccagc tgagcctggg caacgccgcc 240

ctgcagatca ccgacgtgaa gctgcaggac gccggcgtgt acagatgcat gatcagctac 300

ggcggcgccg actacaagag aatcacccgc aacaccggcc gcggcggcga ggagaagaag 360

aaggagaagg agaaggagga gcaggaggag cgcgagacca agacccccga gtgccccagc 420

cacacccagc ccctgggcgt gttcctgttc ccccccaagc ccaaggacac cctgatgatc 480

agccgcaccc ccgaggtgac ctgcgtggtc gtggatgtga gccaggaaga tcccgaagtg 540

cagttcaact ggtacgtgga tggcgtggaa gtgcacaacg ccaagaccaa gcccagagaa 600

gagcagttca actccaccta cagagtggtg agcgtgctga ccgtgctgca ccaggactgg 660

ctgaacggca aggagtacaa gtgcaaggtg tccaacaaag gcctgcccag ctccatcgag 720

aagaccatca gcaaagccaa aggccagccc agagaacccc aggtgtacac cctgcctccc 780

agccaggaag agatgaccaa gaaccaggtg tccctgacct gcctggtgaa aggcttctac 840

cccagcgaca tcgccgtgga gtgggaaagc aacggccagc ccgagaacaa ttacaagaca 900

acccctcccg tgctggatag cgatggcagc ttctttctgt acagcagact gaccgtggac 960

aagagcagat ggcaggaagg caacgtgttc agctgcagcg tgatgcacga agccctgcac 1020

aaccactaca cccagaagag cctgtccctg agcctgggca ag 1062

<210> 30
<211> 1041
<212> DNA
<213> Artificial Sequence

<220>
<223> nucleotide sequence of human PD-L1 V like domain(19-120) fused hyFc

<400> 30
ttcaccgtga ccgtgcccaa ggacctgtac gtggtggagt acggcagcaa catgaccatc 60

gagtgcaagt tccccgtgga gaagcagctg gacctggccg ccctgatcgt gtactgggag 120

atggaggaca agaacatcat ccagttcgtg cacggcgagg aggacctgaa ggtgcagcac 180

agcagctaca gacagagagc cagactgctg aaggaccagc tgagcctggg caacgccgcc 240

ctgcagatca ccgacgtgaa gctgcaggac gccggcgtgt acagatgcat gatcagctac 300

ggcggccgca acaccggccg cggcggcgag gagaagaaga aggagaagga gaaggaggag 360

caggaggagc gcgagaccaa gacccccgag tgccccagcc acacccagcc cctgggcgtg 420

ttcctgttcc cccccaagcc caaggacacc ctgatgatca gccgcacccc cgaggtgacc 480

tgcgtggtcg tggatgtgag ccaggaagat cccgaagtgc agttcaactg gtacgtggat 540

ggcgtggaag tgcacaacgc caagaccaag cccagagaag agcagttcaa ctccacctac 600

agagtggtga gcgtgctgac cgtgctgcac caggactggc tgaacggcaa ggagtacaag 660

tgcaaggtgt ccaacaaagg cctgcccagc tccatcgaga agaccatcag caaagccaaa 720

ggccagccca gagaacccca ggtgtacacc ctgcctccca gccaggaaga gatgaccaag 780

aaccaggtgt ccctgacctg cctggtgaaa ggcttctacc ccagcgacat cgccgtggag 840

tgggaaagca acggccagcc cgagaacaat tacaagacaa cccctcccgt gctggatagc 900

gatggcagct tctttctgta cagcagactg accgtggaca agagcagatg gcaggaaggc 960

aacgtgttca gctgcagcgt gatgcacgaa gccctgcaca accactacac ccagaagagc 1020

ctgtccctga gcctgggcaa g 1041

<210> 31
<211> 1023
<212> DNA
<213> Artificial Sequence

<220>
<223> nucleotide sequence of human PD-L1 V like domain (19-114) fused hyFc

<400> 31
ttcaccgtga ccgtgcccaa ggacctgtac gtggtggagt acggcagcaa catgaccatc 60

gagtgcaagt tccccgtgga gaagcagctg gacctggccg ccctgatcgt gtactgggag 120

atggaggaca agaacatcat ccagttcgtg cacggcgagg aggacctgaa ggtgcagcac 180

agcagctaca gacagagagc cagactgctg aaggaccagc tgagcctggg caacgccgcc 240

ctgcagatca ccgacgtgaa gctgcaggac gccggcgtgt acagatgccg caacaccggc 300

cgcggcggcg aggagaagaa gaaggagaag gagaaggagg agcaggagga gcgcgagacc 360

aagacccccg agtgccccag ccacacccag cccctgggcg tgttcctgtt cccccccaag 420

cccaaggaca ccctgatgat cagccgcacc cccgaggtga cctgcgtggt cgtggatgtg 480

agccaggaag atcccgaagt gcagttcaac tggtacgtgg atggcgtgga agtgcacaac 540

gccaagacca agcccagaga agagcagttc aactccacct acagagtggt gagcgtgctg 600

accgtgctgc accaggactg gctgaacggc aaggagtaca agtgcaaggt gtccaacaaa 660

ggcctgccca gctccatcga gaagaccatc agcaaagcca aaggccagcc cagagaaccc 720

caggtgtaca ccctgcctcc cagccaggaa gagatgacca agaaccaggt gtccctgacc 780

tgcctggtga aaggcttcta ccccagcgac atcgccgtgg agtgggaaag caacggccag 840

cccgagaaca attacaagac aacccctccc gtgctggata gcgatggcag cttctttctg 900

tacagcagac tgaccgtgga caagagcaga tggcaggaag gcaacgtgtt cagctgcagc 960

gtgatgcacg aagccctgca caaccactac acccagaaga gcctgtccct gagcctgggc 1020

aag 1023

<210> 32
<211> 1401
<212> DNA
<213> Artificial Sequence

<220>
<223> nucleotide sequence of human PD-L1 VC like domain(19-239) fused hyFcM1

<400> 32

ttcaccgtga ccgtgcccaa ggacctgtac gtggtggagt acggcagcaa catgaccatc 60
gagtgcaagt tccccgtgga gaagcagctg gacctggccg ccctgatcgt gtactgggag 120
atggaggaca agaacatcat ccagttcgtg cacggcgagg aggacctgaa ggtgcagcac 180
agcagctaca gacagagagc cagactgctg aaggaccagc tgagcctggg caacgccgcc 240
ctgcagatca ccgacgtgaa gctgcaggac gccggcgtgt acagatgcat gatcagctac 300
ggcggcgccg actacaagag aatcaccgtg aaggtgaacg ccccctacaa caagatcaac 360
cagagaatcc tggtggtgga ccccgtgacc agcgagcacg agctgacctg ccaggccgag 420
ggctacccca aggccgaggt gatctggacc agcagcgacc accaggtgct gagcggcaag 480
accaccacca ccaacagcaa gagagaggag aagctgttca acgtgaccag caccctgaga 540
atcaacacca caaccaacga gatcttctac tgcaccttca gaagactgga ccccgaggag 600
aaccacaccg ccgagctggt gatccccgag ctgcccctgg cccaccccc caacgagaga 660
accagaaaca ccggcagagg cggcgaggag aaaaagggcg gcaaggagaa ggaggagcag 720
gaggaaagag agaccaagac ccccgagtgc cccagccaca cccagcccct gggcgtgttc 780
ctgttccctc ccaagcccaa ggacaccctg atgatcagca gaacccccga ggtgacctgc 840
gtggtcgtgg atgtgagcca ggaagatccc gaagtgcagt tcaactggta cgtggatggc 900
gtggaagtgc acaacgccaa gaccaagccc agagaagagc agttcaactc cacctacaga 960
gtggtgagcg tgctgaccgt gctgcaccag gactggctga acggcaagga gtacaagtgc 1020
aaggtgtcca acaaaggcct gcccagctcc atcgagaaga ccatcagcaa agccaaaggc 1080
cagcccagag aaccccaggt gtacaccctg cctcccagcc aggaagagat gaccaagaac 1140
caggtgtccc tgacctgcct ggtgaaaggc ttctacccca gcgacatcgc cgtggagtgg 1200
gaaagcaacg gccagcccga gaacaattac aagacaaccc ctcccgtgct ggatagcgat 1260
ggcagcttct ttctgtacag cagactgacc gtggacaaga gcagatggca ggaaggcaac 1320
gtgttcagct gcagcgtgat gcacgaagcc ctgcacaacc actacaccca gaagagcctg 1380
tccctgagcc tgggcaagtg a 1401

<210> 33
<211> 1395
<212> DNA
<213> Artificial Sequence

<220>
<223> nucleotide sequence of human PD-L1 VC like domain(21-239) fused hyFcM1

<400> 33
gtgactgtcc caaaagatct gtacgtggtc gagtatggct ccaacatgac catcgagtgc 60
aaattccctg tggaaaagca gctggacctg gccgctctga tcgtgtactg ggagatggaa 120
gataagaaca tcatccagtt cgtgcacgga gaggaagacc tgaaggtcca gcattccagc 180
tatagacagc gcgctcgact gctgaaagac cagctgagcc tgggcaacgc agccctgcag 240

atcactgacg tgaagctgca ggatgcagga gtctaccggt gcatgatctc ttacggcgga 300

gcagattata aaagaattac cgtgaaggtc aatgcccccct ataacaagat caatcagagg 360

attctggtgg tcgaccctgt gacaagtgag cacgaactga cttgtcaggc tgaggggtac 420

ccaaaagcag aagtgatctg gacatctagt gatcatcagg tcctgtcagg caagaccaca 480

actaccaact ccaaacgcga ggaaaagctg ttcaatgtga caagcactct gcgaatcaac 540

acaactacca atgagatttt ctattgcacc tttaggcggc tggaccccga ggaaaaccac 600

acagccgagc tggtcatccc agaactgccc ctggctcatc cccctaacga gcgtactaga 660

aacaccggca gaggcggcga ggagaaaaag ggcggcaagg agaaggagga gcaggaggaa 720

agagagacca agacccccga gtgccccagc cacacccagc ccctgggcgt gttcctgttc 780

cctcccaagc ccaaggacac cctgatgatc agcagaaccc ccgaggtgac ctgcgtggtc 840

gtggatgtga gccaggaaga tcccgaagtg cagttcaact ggtacgtgga tggcgtggaa 900

gtgcacaacg ccaagaccaa gcccagagaa gagcagttca actccaccta cagagtggtg 960

agcgtgctga ccgtgctgca ccaggactgg ctgaacggca aggagtacaa gtgcaaggtg 1020

tccaacaaag gcctgcccag ctccatcgag aagaccatca gcaaagccaa aggccagccc 1080

agagaacccc aggtgtacac cctgcctccc agccaggaag agatgaccaa gaaccaggtg 1140

tccctgacct gcctggtgaa aggcttctac cccagcgaca tcgccgtgga gtgggaaagc 1200

aacggccagc ccgagaacaa ttacaagaca acccctcccg tgctggatag cgatggcagc 1260

ttctttctgt acagcagact gaccgtggac aagagcagat ggcaggaagg caacgtgttc 1320

agctgcagcg tgatgcacga agccctgcac aaccactaca cccagaagag cctgtccctg 1380

agcctgggca agtga 1395

<210> 34
<211> 1083
<212> DNA
<213> Artificial Sequence

<220>
<223> nucleotide sequence of human PD-L1 V like domain fused hyFcM1 (19-133)

<400> 34
ttcaccgtga ccgtgcccaa ggacctgtac gtggtggagt acggcagcaa catgaccatc 60

gagtgcaagt tccccgtgga gaagcagctg gacctggccg ccctgatcgt gtactgggag 120

atggaggaca agaacatcat ccagttcgtg cacggcgagg aggacctgaa ggtgcagcac 180

agcagctaca gacagagagc cagactgctg aaggaccagc tgagcctggg caacgccgcc 240

ctgcagatca ccgacgtgaa gctgcaggac gccggcgtgt acagatgcat gatcagctac 300

ggcggcgccg actacaagag aatcaccgtg aaggtgaacg cccccagaaa caccggcaga 360

ggcggcgagg agaaaaaggg cggcaaggag aaggaggagc aggaggaaag agagaccaag 420

acccccgagt gccccagcca cacccagccc ctgggcgtgt tcctgttccc tcccaagccc 480

aaggacaccc tgatgatcag cagaaccccc gaggtgacct gcgtggtcgt ggatgtgagc 540

caggaagatc ccgaagtgca gttcaactgg tacgtggatg gcgtggaagt gcacaacgcc 600

aagaccaagc ccagagaaga gcagttcaac tccacctaca gagtggtgag cgtgctgacc 660

gtgctgcacc aggactggct gaacggcaag gagtacaagt gcaaggtgtc caacaaaggc 720

ctgcccagct ccatcgagaa gaccatcagc aaagccaaag gccagcccag agaaccccag 780

gtgtacaccc tgcctcccag ccaggaagag atgaccaaga accaggtgtc cctgacctgc 840

ctggtgaaag gcttctaccc cagcgacatc gccgtggagt gggaaagcaa cggccagccc 900

gagaacaatt acaagacaac ccctcccgtg ctggatagcg atggcagctt ctttctgtac 960

agcagactga ccgtggacaa gagcagatgg caggaaggca acgtgttcag ctgcagcgtg 1020

atgcacgaag ccctgcacaa ccactacacc cagaagagcc tgtccctgag cctgggcaag 1080

tga 1083

<210> 35
<211> 1065
<212> DNA
<213> Artificial Sequence

<220>
<223> nucleotide sequence of human PD-L1 V like domain(19-127) fused hyFcM1

<400> 35
ttcaccgtga ccgtgcccaa ggacctgtac gtggtggagt acggcagcaa catgaccatc 60

gagtgcaagt tccccgtgga gaagcagctg gacctggccg ccctgatcgt gtactgggag 120

atggaggaca agaacatcat ccagttcgtg cacggcgagg aggacctgaa ggtgcagcac 180

agcagctaca gacagagagc cagactgctg aaggaccagc tgagcctggg caacgccgcc 240

ctgcagatca ccgacgtgaa gctgcaggac gccggcgtgt acagatgcat gatcagctac 300

ggcggcgccg actacaagag aatcaccaga aacaccggca gaggcggcga ggagaaaaag 360

ggcggcaagg agaaggagga gcaggaggaa agagagacca agacccccga gtgccccagc 420

cacacccagc ccctgggcgt gttcctgttc cctcccaagc ccaaggacac cctgatgatc 480

agcagaaccc ccgaggtgac ctgcgtggtc gtggatgtga gccaggaaga tcccgaagtg 540

cagttcaact ggtacgtgga tggcgtggaa gtgcacaacg ccaagaccaa gcccagagaa 600

gagcagttca actccaccta cagagtggtg agcgtgctga ccgtgctgca ccaggactgg 660

ctgaacggca aggagtacaa gtgcaaggtg tccaacaaag gcctgcccag ctccatcgag 720

aagaccatca gcaaagccaa aggccagccc agagaacccc aggtgtacac cctgcctccc 780

agccaggaag agatgaccaa gaaccaggtg tccctgacct gcctggtgaa aggcttctac 840

cccagcgaca tcgccgtgga gtgggaaagc aacggccagc ccgagaacaa ttacaagaca 900

acccctcccg tgctggatag cgatggcagc ttctttctgt acagcagact gaccgtggac 960

aagagcagat ggcaggaagg caacgtgttc agctgcagcg tgatgcacga agccctgcac 1020

aaccactaca cccagaagag cctgtccctg agcctgggca agtga 1065

<210> 36
<211> 1044
<212> DNA
<213> Artificial Sequence

<220>
<223> nucleotide sequence of human PD-L1 V like domain(19-120) fused hyFcM1

<400> 36
ttcaccgtga ccgtgcccaa ggacctgtac gtggtggagt acggcagcaa catgaccatc 60

gagtgcaagt tccccgtgga gaagcagctg gacctggccg ccctgatcgt gtactgggag 120

atggaggaca agaacatcat ccagttcgtg cacggcgagg aggacctgaa ggtgcagcac 180

agcagctaca gacagagagc cagactgctg aaggaccagc tgagcctggg caacgccgcc 240

ctgcagatca ccgacgtgaa gctgcaggac gccggcgtgt acagatgcat gatcagctac 300

ggcggcagaa acaccggcag aggcggcgag gagaaaaagg gcggcaagga gaaggaggag 360

caggaggaaa gagagaccaa gacccccgag tgccccagcc acacccagcc cctgggcgtg 420

ttcctgttcc ctcccaagcc caaggacacc ctgatgatca gcagaacccc cgaggtgacc 480

tgcgtggtcg tggatgtgag ccaggaagat cccgaagtgc agttcaactg gtacgtggat 540

ggcgtggaag tgcacaacgc caagaccaag cccagagaag agcagttcaa ctccacctac 600

agagtggtga gcgtgctgac cgtgctgcac caggactggc tgaacggcaa ggagtacaag 660

tgcaaggtgt ccaacaaagg cctgcccagc tccatcgaga agaccatcag caaagccaaa 720

ggccagccca gagaacccca ggtgtacacc ctgcctccca gccaggaaga gatgaccaag 780

aaccaggtgt ccctgacctg cctggtgaaa ggcttctacc ccagcgacat cgccgtggag 840

tgggaaagca acggccagcc cgagaacaat tacaagacaa cccctcccgt gctggatagc 900

gatggcagct tctttctgta cagcagactg accgtggaca agagcagatg gcaggaaggc 960

aacgtgttca gctgcagcgt gatgcacgaa gccctgcaca accactacac ccagaagagc 1020

ctgtccctga gcctgggcaa gtga 1044

<210> 37
<211> 1026
<212> DNA
<213> Artificial Sequence

<220>
<223> nucleotide sequence of human PD-L1 V like domain(19-114) fused hyFcM1

<400> 37
ttcaccgtga ccgtgcccaa ggacctgtac gtggtggagt acggcagcaa catgaccatc 60

gagtgcaagt tccccgtgga gaagcagctg gacctggccg ccctgatcgt gtactgggag 120

atggaggaca agaacatcat ccagttcgtg cacggcgagg aggacctgaa ggtgcagcac 180

agcagctaca gacagagagc cagactgctg aaggaccagc tgagcctggg caacgccgcc 240

ctgcagatca ccgacgtgaa gctgcaggac gccggcgtgt acagatgcag aaacaccggc 300

agaggcggcg aggagaaaaa gggcggcaag gagaaggagg agcaggagga aagagagacc 360

aagacccccg agtgccccag ccacacccag cccctgggcg tgttcctgtt ccctcccaag 420

cccaaggaca ccctgatgat cagcagaacc cccgaggtga cctgcgtggt cgtggatgtg 480

agccaggaag atcccgaagt gcagttcaac tggtacgtgg atggcgtgga agtgcacaac 540

gccaagacca agcccagaga agagcagttc aactccacct acagagtggt gagcgtgctg 600

accgtgctgc accaggactg gctgaacggc aaggagtaca agtgcaaggt gtccaacaaa 660

ggcctgccca gctccatcga gaagaccatc agcaaagcca aaggccagcc cagagaaccc 720

caggtgtaca ccctgcctcc cagccaggaa gagatgacca agaaccaggt gtccctgacc 780

tgcctggtga aaggcttcta ccccagcgac atcgccgtgg agtgggaaag caacggccag 840

cccgagaaca attacaagac aacccctccc gtgctggata gcgatggcag cttctttctg 900

tacagcagac tgaccgtgga caagagcaga tggcaggaag gcaacgtgtt cagctgcagc 960

gtgatgcacg aagccctgca caaccactac acccagaaga gcctgtccct gagcctgggc 1020

aagtga 1026

<210> 38
<211> 75
<212> DNA
<213> Artificial Sequence

<220>
<223> Codon-optimized tissue plasminogen activator secretory signal sequence nucleotide sequence

<400> 38
atggacgcca tgctgagagg cctgtgctgc gtgctgctgc tgtgcggcgc cgtgttcgtg 60

agccccagcc acgcc 75

<210> 39
<211> 109
<212> PRT
<213> Artificial Sequence

<220>
<223> amino acid sequence of human PD-L1 V like domain(19-127)

<400> 39
Phe Thr Val Thr Val Pro Lys Asp Leu Tyr Val Val Glu Tyr Gly Ser
1 5 10 15

Asn Met Thr Ile Glu Cys Lys Phe Pro Val Glu Lys Gln Leu Asp Leu
20 25 30

Ala Ala Leu Ile Val Tyr Trp Glu Met Glu Asp Lys Asn Ile Ile Gln
35 40 45

Phe Val His Gly Glu Glu Asp Leu Lys Val Gln His Ser Ser Tyr Arg
50 55 60

Gln Arg Ala Arg Leu Leu Lys Asp Gln Leu Ser Leu Gly Asn Ala Ala
65 70 75 80

Leu Gln Ile Thr Asp Val Lys Leu Gln Asp Ala Gly Val Tyr Arg Cys
85 90 95

Met Ile Ser Tyr Gly Gly Ala Asp Tyr Lys Arg Ile Thr
100 105

<210> 40
<211> 115
<212> PRT
<213> Artificial Sequence

<220>
<223> amino acid sequence of human PD-L1 V like domain(19-133)

<400> 40
Phe Thr Val Thr Val Pro Lys Asp Leu Tyr Val Val Glu Tyr Gly Ser
1 5 10 15

Asn Met Thr Ile Glu Cys Lys Phe Pro Val Glu Lys Gln Leu Asp Leu
20 25 30

Ala Ala Leu Ile Val Tyr Trp Glu Met Glu Asp Lys Asn Ile Ile Gln
35 40 45

Phe Val His Gly Glu Glu Asp Leu Lys Val Gln His Ser Ser Tyr Arg
50 55 60

Gln Arg Ala Arg Leu Leu Lys Asp Gln Leu Ser Leu Gly Asn Ala Ala
65 70 75 80

Leu Gln Ile Thr Asp Val Lys Leu Gln Asp Ala Gly Val Tyr Arg Cys
85 90 95

Met Ile Ser Tyr Gly Gly Ala Asp Tyr Lys Arg Ile Thr Val Lys Val
100 105 110

Asn Ala Pro
115

<210> 41
<211> 221
<212> PRT
<213> Artificial Sequence

<220>
<223> amino acid sequence of human PD-L1 VC like domain (19-239)

<400> 41
Phe Thr Val Thr Val Pro Lys Asp Leu Tyr Val Val Glu Tyr Gly Ser
1 5 10 15

Asn Met Thr Ile Glu Cys Lys Phe Pro Val Glu Lys Gln Leu Asp Leu
20 25 30

Ala Ala Leu Ile Val Tyr Trp Glu Met Glu Asp Lys Asn Ile Ile Gln
35 40 45

Phe Val His Gly Glu Glu Asp Leu Lys Val Gln His Ser Ser Tyr Arg
50 55 60

Gln Arg Ala Arg Leu Leu Lys Asp Gln Leu Ser Leu Gly Asn Ala Ala
65 70 75 80

Leu Gln Ile Thr Asp Val Lys Leu Gln Asp Ala Gly Val Tyr Arg Cys
85 90 95

Met Ile Ser Tyr Gly Gly Ala Asp Tyr Lys Arg Ile Thr Val Lys Val
100 105 110

Asn Ala Pro Tyr Asn Lys Ile Asn Gln Arg Ile Leu Val Val Asp Pro
115 120 125

Val Thr Ser Glu His Glu Leu Thr Cys Gln Ala Glu Gly Tyr Pro Lys
130 135 140

Ala Glu Val Ile Trp Thr Ser Ser Asp His Gln Val Leu Ser Gly Lys
145 150 155 160

Thr Thr Thr Thr Asn Ser Lys Arg Glu Glu Lys Leu Phe Asn Val Thr
165 170 175

Ser Thr Leu Arg Ile Asn Thr Thr Thr Asn Glu Ile Phe Tyr Cys Thr
180 185 190

Phe Arg Arg Leu Asp Pro Glu Glu Asn His Thr Ala Glu Leu Val Ile
195 200 205

Pro Glu Leu Pro Leu Ala His Pro Pro Asn Glu Arg Thr
210 215 220

<210> 42
<211> 245
<212> PRT
<213> Artificial Sequence

<220>
<223> amino acid sequence of hyFcM2

<400> 42
Arg Asn Thr Gly Arg Gly Gly Glu Glu Lys Lys Gly Ser Lys Glu Lys
1 5 10 15

Glu Glu Gln Glu Glu Arg Glu Thr Lys Thr Pro Glu Cys Pro Ser His
20 25 30

Thr Gln Pro Leu Gly Val Phe Leu Phe Pro Pro Lys Pro Lys Asp Thr
35 40 45

Leu Met Ile Ser Arg Thr Pro Glu Val Thr Cys Val Val Val Asp Val
50 55 60

Ser Gln Glu Asp Pro Glu Val Gln Phe Asn Trp Tyr Val Asp Gly Val
65 70 75 80

Glu Val His Asn Ala Lys Thr Lys Pro Arg Glu Glu Gln Phe Asn Ser
85 90 95

Thr Tyr Arg Val Val Ser Val Leu Thr Val Leu His Gln Asp Trp Leu
100 105 110

Asn Gly Lys Glu Tyr Lys Cys Lys Val Ser Asn Lys Gly Leu Pro Ser
115 120 125

Ser Ile Glu Lys Thr Ile Ser Lys Ala Lys Gly Gln Pro Arg Glu Pro
130 135 140

Gln Val Tyr Thr Leu Pro Pro Ser Gln Glu Glu Met Thr Lys Asn Gln
145 150 155 160

Val Ser Leu Thr Cys Leu Val Lys Gly Phe Tyr Pro Ser Asp Ile Ala
165 170 175

Val Glu Trp Glu Ser Asn Gly Gln Pro Glu Asn Asn Tyr Lys Thr Thr
180 185 190

Pro Pro Val Leu Asp Ser Asp Gly Ser Phe Phe Leu Tyr Ser Arg Leu
195 200 205

Thr Val Asp Lys Ser Arg Trp Gln Glu Gly Asn Val Phe Ser Cys Ser
210 215 220

Val Met His Glu Ala Leu His Asn His Tyr Thr Gln Lys Ser Leu Ser
225 230 235 240

Leu Ser Leu Gly Lys
245

<210> 43
<211> 245
<212> PRT
<213> Artificial Sequence

<220>
<223> amino acid sequence of hyFcM3

<400> 43
Arg Asn Thr Gly Arg Gly Gly Glu Glu Lys Lys Ser Gly Lys Glu Lys
1 5 10 15

Glu Glu Gln Glu Glu Arg Glu Thr Lys Thr Pro Glu Cys Pro Ser His
20 25 30

Thr Gln Pro Leu Gly Val Phe Leu Phe Pro Pro Lys Pro Lys Asp Thr
35 40 45

Leu Met Ile Ser Arg Thr Pro Glu Val Thr Cys Val Val Val Asp Val
50 55 60

Ser Gln Glu Asp Pro Glu Val Gln Phe Asn Trp Tyr Val Asp Gly Val
65 70 75 80

Glu Val His Asn Ala Lys Thr Lys Pro Arg Glu Glu Gln Phe Asn Ser
85 90 95

Thr Tyr Arg Val Val Ser Val Leu Thr Val Leu His Gln Asp Trp Leu
100 105 110

Asn Gly Lys Glu Tyr Lys Cys Lys Val Ser Asn Lys Gly Leu Pro Ser
115 120 125

Ser Ile Glu Lys Thr Ile Ser Lys Ala Lys Gly Gln Pro Arg Glu Pro
130 135 140

Gln Val Tyr Thr Leu Pro Pro Ser Gln Glu Glu Met Thr Lys Asn Gln
145 150 155 160

Val Ser Leu Thr Cys Leu Val Lys Gly Phe Tyr Pro Ser Asp Ile Ala
165 170 175

Val Glu Trp Glu Ser Asn Gly Gln Pro Glu Asn Asn Tyr Lys Thr Thr
180 185 190

Pro Pro Val Leu Asp Ser Asp Gly Ser Phe Phe Leu Tyr Ser Arg Leu
195 200 205

Thr Val Asp Lys Ser Arg Trp Gln Glu Gly Asn Val Phe Ser Cys Ser
210 215 220

Val Met His Glu Ala Leu His Asn His Tyr Thr Gln Lys Ser Leu Ser
225 230 235 240

Leu Ser Leu Gly Lys
245

<210> 44
<211> 245
<212> PRT
<213> Artificial Sequence

<220>
<223> amino acid sequence of hyFcM4

<400> 44
Arg Asn Thr Gly Arg Gly Gly Glu Glu Lys Lys Ser Ser Lys Glu Lys
1 5 10 15

Glu Glu Gln Glu Glu Arg Glu Thr Lys Thr Pro Glu Cys Pro Ser His
20 25 30

Thr Gln Pro Leu Gly Val Phe Leu Phe Pro Pro Lys Pro Lys Asp Thr
35 40 45

Leu Met Ile Ser Arg Thr Pro Glu Val Thr Cys Val Val Val Asp Val
50 55 60

Ser Gln Glu Asp Pro Glu Val Gln Phe Asn Trp Tyr Val Asp Gly Val
65 70 75 80

Glu Val His Asn Ala Lys Thr Lys Pro Arg Glu Glu Gln Phe Asn Ser
85 90 95

Thr Tyr Arg Val Val Ser Val Leu Thr Val Leu His Gln Asp Trp Leu
100 105 110

Asn Gly Lys Glu Tyr Lys Cys Lys Val Ser Asn Lys Gly Leu Pro Ser
115 120 125

Ser Ile Glu Lys Thr Ile Ser Lys Ala Lys Gly Gln Pro Arg Glu Pro
130 135 140

Gln Val Tyr Thr Leu Pro Pro Ser Gln Glu Glu Met Thr Lys Asn Gln
145 150 155 160

Val Ser Leu Thr Cys Leu Val Lys Gly Phe Tyr Pro Ser Asp Ile Ala
165 170 175

Val Glu Trp Glu Ser Asn Gly Gln Pro Glu Asn Asn Tyr Lys Thr Thr
180 185 190

Pro Pro Val Leu Asp Ser Asp Gly Ser Phe Phe Leu Tyr Ser Arg Leu
195 200 205

Thr Val Asp Lys Ser Arg Trp Gln Glu Gly Asn Val Phe Ser Cys Ser
210 215 220

Val Met His Glu Ala Leu His Asn His Tyr Thr Gln Lys Ser Leu Ser
225 230 235 240

Leu Ser Leu Gly Lys
245

<210> 45
<211> 5
<212> PRT
<213> Artificial Sequence

<220>
<223> PD-L1 linker

<400> 45
Lys Val Asn Ala Pro
1 5

<210> 46
<211> 6
<212> PRT
<213> Artificial Sequence

<220>
<223> PD-L1 linker

<400> 46
Val Lys Val Asn Ala Pro
1 5

<210> 47
<211> 106
<212> PRT
<213> Artificial Sequence

<220>
<223> amino acid sequence containng human PD-L1 C like domain (134-239)

<400> 47
Tyr Asn Lys Ile Asn Gln Arg Ile Leu Val Val Asp Pro Val Thr Ser
1 5 10 15

Glu His Glu Leu Thr Cys Gln Ala Glu Gly Tyr Pro Lys Ala Glu Val
20 25 30

Ile Trp Thr Ser Ser Asp His Gln Val Leu Ser Gly Lys Thr Thr Thr
35 40 45

Thr Asn Ser Lys Arg Glu Glu Lys Leu Phe Asn Val Thr Ser Thr Leu
50 55 60

Arg Ile Asn Thr Thr Thr Asn Glu Ile Phe Tyr Cys Thr Phe Arg Arg
65 70 75 80

Leu Asp Pro Glu Glu Asn His Thr Ala Glu Leu Val Ile Pro Glu Leu
85 90 95

Pro Leu Ala His Pro Pro Asn Glu Arg Thr
100 105

<210> 48
<211> 107
<212> PRT
<213> Artificial Sequence

<220>
<223> amino acid sequence of human PD-L1 V like domain (21-127)

<400> 48
Val Thr Val Pro Lys Asp Leu Tyr Val Val Glu Tyr Gly Ser Asn Met
1 5 10 15

Thr Ile Glu Cys Lys Phe Pro Val Glu Lys Gln Leu Asp Leu Ala Ala
20 25 30

Leu Ile Val Tyr Trp Glu Met Glu Asp Lys Asn Ile Ile Gln Phe Val
35 40 45

His Gly Glu Glu Asp Leu Lys Val Gln His Ser Ser Tyr Arg Gln Arg
50 55 60

Ala Arg Leu Leu Lys Asp Gln Leu Ser Leu Gly Asn Ala Ala Leu Gln
65 70 75 80

Ile Thr Asp Val Lys Leu Gln Asp Ala Gly Val Tyr Arg Cys Met Ile
85 90 95

Ser Tyr Gly Gly Ala Asp Tyr Lys Arg Ile Thr
100 105

<210> 49
<211> 105
<212> PRT
<213> Artificial Sequence

<220>
<223> amino acid sequence including human PD-L1 C like domain (134-238)

<400> 49
Tyr Asn Lys Ile Asn Gln Arg Ile Leu Val Val Asp Pro Val Thr Ser
1 5 10 15

Glu His Glu Leu Thr Cys Gln Ala Glu Gly Tyr Pro Lys Ala Glu Val
20 25 30

Ile Trp Thr Ser Ser Asp His Gln Val Leu Ser Gly Lys Thr Thr Thr
35 40 45

Thr Asn Ser Lys Arg Glu Glu Lys Leu Phe Asn Val Thr Ser Thr Leu
50 55 60

Arg Ile Asn Thr Thr Thr Asn Glu Ile Phe Tyr Cys Thr Phe Arg Arg
65 70 75 80

Leu Asp Pro Glu Glu Asn His Thr Ala Glu Leu Val Ile Pro Glu Leu
85 90 95

Pro Leu Ala His Pro Pro Asn Glu Arg
100 105

<210> 50
<211> 107
<212> PRT
<213> Artificial Sequence

<220>
<223> amino acid sequence of mouse PD-L1 V like domain(21-127)

<400> 50

```
Ile Thr Ala Pro Lys Asp Leu Tyr Val Val Glu Tyr Gly Ser Asn Val
1               5                   10                  15

Thr Met Glu Cys Arg Phe Pro Val Glu Arg Glu Leu Asp Leu Leu Ala
            20                  25                  30

Leu Val Val Tyr Trp Glu Lys Glu Asp Glu Gln Val Ile Gln Phe Val
        35                  40                  45

Ala Gly Glu Glu Asp Leu Lys Pro Gln His Ser Asn Phe Arg Gly Arg
    50                  55                  60

Ala Ser Leu Pro Lys Asp Gln Leu Leu Lys Gly Asn Ala Ala Leu Gln
65                  70                  75                  80

Ile Thr Asp Val Lys Leu Gln Asp Ala Gly Val Tyr Cys Cys Ile Ile
                85                  90                  95

Ser Tyr Gly Gly Ala Asp Tyr Lys Arg Ile Thr
            100                 105
```